# EUROPEAN PATENT SPECIFICATION

(11) **EP 3 411 715 B1**
(45) Date of publication and mention of the grant of the patent: **20.11.2019**
(21) Application number: 17710825.5
(22) Date of filing: 06.02.2017
(51) Int. Cl.: C07K 16/18

(54) **METHOD FOR DIAGNOSING AND TREATING CANCER USING ANTIBODIES THAT BIND TO C1 INACTIVATOR, C REACTIVE PROTEIN AND/OR COMPLEMENT COMPONENT C4**
VERFAHREN ZUR DIAGNOSE UND BEHANDLUNG VON KREBS MIT AN EINEN C1-INAKTIVATOR, EIN C-REAKTIVES PROTEIN UND/ODER EINE KOMPLEMENTKOMPONENTE C4 BINDENDEN ANTIKÖRPERN
MÉTHODE DE DIAGNOSTIC ET DE TRAITEMENT DU CANCER AU MOYEN D'ANTICORPS SE LIANT À UN INACTIVATEUR C1, À UNE PROTÉINE RÉACTIVE C ET/OU À UN COMPOSANT C4 DU COMPLÉMENT

(30) Priority: 05.02.2016 US 201662388720 P; 07.09.2016 US 201662495203 P; 01.12.2016 US 201662497760 P
(43) Date of publication of application: 12.12.2018
(73) Proprietor: Dana Genetic A/S, 2820 Gentofte (DK)
(72) Inventor: OSTHER, Kurt, Scottsdale Arizona 85259 (US)
(74) Representative: Plougmann Vingtoft a/s
(86) International application number: PCT/DK2017/050027
(87) International publication number: WO 2017/133746

(56) References cited:
- US-A- 4 132 769
- JOAKIM GÖRANSSON ET AL: "Pre-operative plasma levels of C-reactive protein, albumin and various plasma protease inhibitors for the pre-operative assessment of operability and recurrence in cancer surgery", EUROPEAN JOURNAL OF SURGICAL ONCOLOGY (EJSO), vol. 22, no. 6, 1 December 1996 (1996-12-01), pages 607-617, XP055087668, ISSN: 0748-7983, DOI: 10.1016/S0748-7983(96)92398-7
- JUDIT KOCSIS ET AL: "High levels of acute phase proteins and soluble 70 kDa heat shock proteins are independent and additive risk factors for mortality in colorectal cancer", CELL STRESS AND CHAPERONES, vol. 16, no. 1, 22 August 2010 (2010-08-22), pages 49-55, XP055360678, EDINBURGH, GB ISSN: 1355-8145, DOI: 10.1007/s12192-010-0220-z
- NIELS BACH-MORTENSEN ET AL: "C1-esterase inactivators and C4 in malignant diseases", THE LANCET, vol. 306, no. 7933, 1 January 1975 (1975-01-01), pages 499-500, XP055246001,
- WANG C-S ET AL: "C-reactive protein and malignancy: Clinico-pathological association and therapeutic implication", CHANGGENG YIXUE ZAZHI - CHNAG GUNG MEDICAL JOURNAL, TAIPEI, CN, vol. 32, no. 5, 1 September 2009 (2009-09-01), pages 471-482, XP008131294, ISSN: 0255-8270

### SEQUENCE LISTING

<110> Osther, Kurt
<120> Method for diagnosing and treating cancer using antibodies that bind to C1 inactivator, C-Reactive Protein and/or Complement Component C4
<130> 59732PC01
<150> US 62/388,720
   <151> 2016-05-02
<150> US 62/497,760
   <151> 2016-12-01
<150> US 62/495,203
   <151> 2016-09-07
<160> 3
<170> PatentIn version 3.5
<210> 1
   <211> 500
   <212> PRT
   <213> Homo sapiens
<400> 1
<210> 2
   <211> 224
   <212> PRT
   <213> Homo sapiens
<400> 2
   Met Glu Lys Leu Leu Cys Phe Leu Val Leu Thr Ser Leu Ser His Ala
   1               5                   10                  15
   Phe Gly Gln Thr Asp Met Ser Arg Lys Ala Phe Val Phe Pro Lys Glu
               20                  25                  30
   Ser Asp Thr Ser Tyr Val Ser Leu Lys Ala Pro Leu Thr Lys Pro Leu
           35                  40                  45
   Lys Ala Phe Thr Val Cys Leu His Phe Tyr Thr Glu Leu Ser Ser Thr
       50                  55                  60
   Arg Gly Tyr Ser Ile Phe Ser Tyr Ala Thr Lys Arg Gln Asp Asn Glu
   65                  70                  75                  80
   Ile Leu Ile Phe Trp Ser Lys Asp Ile Gly Tyr Ser Phe Thr Val Gly
                   85                  90                  95
   Gly Ser Glu Ile Leu Phe Glu Val Pro Glu Val Thr Val Ala Pro Val
               100                 105                 110
   His Ile Cys Thr Ser Trp Glu Ser Ala Ser Gly Ile Val Glu Phe Trp
           115                 120                 125
   Val Asp Gly Lys Pro Arg Val Arg Lys Ser Leu Lys Lys Gly Tyr Thr
       130                 135                 140
   Val Gly Ala Glu Ala Ser Ile Ile Leu Gly Gln Glu Gln Asp Ser Phe
   145                 150                 155                 160
   Gly Gly Asn Phe Glu Gly Ser Gln Ser Leu Val Gly Asp Ile Gly Asn
                   165                 170                 175
   Val Asn Met Trp Asp Phe Val Leu Ser Pro Asp Glu Ile Asn Thr Ile
               180                 185                 190
   Tyr Leu Gly Gly Pro Phe Ser Pro Asn Val Leu Asn Trp Arg Ala Leu
           195                 200                 205
   Lys Tyr Glu Val Gln Gly Glu Val Phe Thr Lys Pro Gln Leu Trp Pro
       210                 215                 220
<210> 3
   <211> 1744
   <212> PRT
   <213> Homo sapeins
<400> 3
   Met Arg Leu Leu Trp Gly Leu Ile Trp Ala Ser Ser Phe Phe Thr Leu
   1               5                   10                  15
   Ser Leu Gln Lys Pro Arg Leu Leu Leu Phe Ser Pro Ser Val Val His
               20                  25                  30
   Leu Gly Val Pro Leu Ser Val Gly Val Gln Leu Gln Asp Val Pro Arg
           35                  40                  45
   Gly Gln Val Val Lys Gly Ser Val Phe Leu Arg Asn Pro Ser Arg Asn
       50                  55                  60
   Asn Val Pro Cys Ser Pro Lys Val Asp Phe Thr Leu Ser Ser Glu Arg
   65                  70                  75                  80
   Asp Phe Ala Leu Leu Ser Leu Gln Val Pro Leu Lys Asp Ala Lys Ser
                   85                  90                  95
   Cys Gly Leu His Gln Leu Leu Arg Gly Pro Glu Val Gln Leu Val Ala
               100                 105                 110
   His Ser Pro Trp Leu Lys Asp Ser Leu Ser Arg Thr Thr Asn Ile Gln
           115                 120                 125
   Gly Ile Asn Leu Leu Phe Ser Ser Arg Arg Gly His Leu Phe Leu Gln
       130                 135                 140
   Thr Asp Gln Pro Ile Tyr Asn Pro Gly Gln Arg Val Arg Tyr Arg Val
   145                 150                 155                 160
   Phe Ala Leu Asp Gln Lys Met Arg Pro Ser Thr Asp Thr Ile Thr Val
                   165                 170                 175
   Met Val Glu Asn Ser His Gly Leu Arg Val Arg Lys Lys Glu Val Tyr
               180                 185                 190
   Met Pro Ser Ser Ile Phe Gln Asp Asp Phe Val Ile Pro Asp Ile Ser
           195                 200                 205
   Glu Pro Gly Thr Trp Lys Ile Ser Ala Arg Phe Ser Asp Gly Leu Glu
       210                 215                 220
   Ser Asn Ser Ser Thr Gln Phe Glu Val Lys Lys Tyr Val Leu Pro Asn
   225                 230                 235                 240
   Phe Glu Val Lys Ile Thr Pro Gly Lys Pro Tyr Ile Leu Thr Val Pro
                   245                 250                 255
   Gly His Leu Asp Glu Met Gln Leu Asp Ile Gln Ala Arg Tyr Ile Tyr
               260                 265                 270
   Gly Lys Pro Val Gln Gly Val Ala Tyr Val Arg Phe Gly Leu Leu Asp
           275                 280                 285
   Glu Asp Gly Lys Lys Thr Phe Phe Arg Gly Leu Glu Ser Gln Thr Lys
       290                 295                 300
   Leu Val Asn Gly Gln Ser His Ile Ser Leu Ser Lys Ala Glu Phe Gln
   305                 310                 315                 320
   Asp Ala Leu Glu Lys Leu Asn Met Gly Ile Thr Asp Leu Gln Gly Leu
                   325                 330                 335
   Arg Leu Tyr Val Ala Ala Ala Ile Ile Glu Ser Pro Gly Gly Glu Met
               340                 345                 350
   Glu Glu Ala Glu Leu Thr Ser Trp Tyr Phe Val Ser Ser Pro Phe Ser
   355 360 365
   Leu Asp Leu Ser Lys Thr Lys Arg His Leu Val Pro Gly Ala Pro Phe
   370 375 380
   Leu Leu Gln Ala Leu Val Arg Glu Met Ser Gly Ser Pro Ala Ser Gly
   385 390 395 400
   Ile Pro Val Lys Val Ser Ala Thr Val Ser Ser Pro Gly Ser Val Pro
   405 410 415
   Glu Val Gln Asp Ile Gln Gln Asn Thr Asp Gly Ser Gly Gln Val Ser
   420 425 430
   Ile Pro Ile Ile Ile Pro Gln Thr Ile Ser Glu Leu Gln Leu Ser Val
   435 440 445
   Ser Ala Gly Ser Pro His Pro Ala Ile Ala Arg Leu Thr Val Ala Ala
   450 455 460
   Pro Pro Ser Gly Gly Pro Gly Phe Leu Ser Ile Glu Arg Pro Asp Ser
   465 470 475 480
   Arg Pro Pro Arg Val Gly Asp Thr Leu Asn Leu Asn Leu Arg Ala Val
   485 490 495
   Gly Ser Gly Ala Thr Phe Ser His Tyr Tyr Tyr Met Ile Leu Ser Arg
   500 505 510
   Gly Gln Ile Val Phe Met Asn Arg Glu Pro Lys Arg Thr Leu Thr Ser
   515 520 525
   Val Ser Val Phe Val Asp His His Leu Ala Pro Ser Phe Tyr Phe Val
   530 535 540
   Ala Phe Tyr Tyr His Gly Asp His Pro Val Ala Asn Ser Leu Arg Val
   545 550 555 560
   Asp Val Gln Ala Gly Ala Cys Glu Gly Lys Leu Glu Leu Ser Val Asp
   565 570 575
   Gly Ala Lys Gln Tyr Arg Asn Gly Glu Ser Val Lys Leu His Leu Glu
   580 585 590
   Thr Asp Ser Leu Ala Leu Val Ala Leu Gly Ala Leu Asp Thr Ala Leu
   595 600 605
   Tyr Ala Ala Gly Ser Lys Ser His Lys Pro Leu Asn Met Gly Lys Val
       610                 615                 620
   Phe Glu Ala Met Asn Ser Tyr Asp Leu Gly Cys Gly Pro Gly Gly Gly
   625                 630                 635                 640
   Asp Ser Ala Leu Gln Val Phe Gln Ala Ala Gly Leu Ala Phe Ser Asp
                   645                 650                 655
   Gly Asp Gln Trp Thr Leu Ser Arg Lys Arg Leu Ser Cys Pro Lys Glu
               660                 665                 670
   Lys Thr Thr Arg Lys Lys Arg Asn Val Asn Phe Gln Lys Ala Ile Asn
           675                 680                 685
   Glu Lys Leu Gly Gln Tyr Ala Ser Pro Thr Ala Lys Arg Cys Cys Gln
       690                 695                 700
   Asp Gly Val Thr Arg Leu Pro Met Met Arg Ser Cys Glu Gln Arg Ala
   705                 710                 715                 720
   Ala Arg Val Gln Gln Pro Asp Cys Arg Glu Pro Phe Leu Ser Cys Cys
                   725                 730                 735
   Gln Phe Ala Glu Ser Leu Arg Lys Lys Ser Arg Asp Lys Gly Gln Ala
               740                 745                 750
   Gly Leu Gln Arg Ala Leu Glu Ile Leu Gln Glu Glu Asp Leu Ile Asp
           755                 760                 765
   Glu Asp Asp Ile Pro Val Arg Ser Phe Phe Pro Glu Asn Trp Leu Trp
       770                 775                 780
   Arg Val Glu Thr Val Asp Arg Phe Gln Ile Leu Thr Leu Trp Leu Pro
   785                 790                 795                 800
   Asp Ser Leu Thr Thr Trp Glu Ile His Gly Leu Ser Leu Ser Lys Thr
                   805                 810                 815
   Lys Gly Leu Cys Val Ala Thr Pro Val Gln Leu Arg Val Phe Arg Glu
               820                 825                 830
   Phe His Leu His Leu Arg Leu Pro Met Ser Val Arg Arg Phe Glu Gln
           835                 840                 845
   Leu Glu Leu Arg Pro Val Leu Tyr Asn Tyr Leu Asp Lys Asn Leu Thr
   850 855 860
   Val Ser Val His Val Ser Pro Val Glu Gly Leu Cys Leu Ala Gly Gly
   865 870 875 880
   Gly Gly Leu Ala Gln Gln Val Leu Val Pro Ala Gly Ser Ala Arg Pro
   885 890 895
   Val Ala Phe Ser Val Val Pro Thr Ala Ala Thr Ala Val Ser Leu Lys
   900 905 910
   Val Val Ala Arg Gly Ser Phe Glu Phe Pro Val Gly Asp Ala Val Ser
   915 920 925
   Lys Val Leu Gln Ile Glu Lys Glu Gly Ala Ile His Arg Glu Glu Leu
   930 935 940
   Val Tyr Glu Leu Asn Pro Leu Asp His Arg Gly Arg Thr Leu Glu Ile
   945 950 955 960
   Pro Gly Asn Ser Asp Pro Asn Met Ile Pro Asp Gly Asp Phe Asn Ser
   965 970 975
   Tyr Val Arg Val Thr Ala Ser Asp Pro Leu Asp Thr Leu Gly Ser Glu
   980 985 990
   Gly Ala Leu Ser Pro Gly Gly Val Ala Ser Leu Leu Arg Leu Pro Arg
   995 1000 1005
   Gly Cys Gly Glu Gln Thr Met Ile Tyr Leu Ala Pro Thr Leu Ala
   1010 1015 1020
   Ala Ser Arg Tyr Leu Asp Lys Thr Glu Gln Trp Ser Thr Leu Pro
   1025 1030 1035
   Pro Glu Thr Lys Asp His Ala Val Asp Leu Ile Gln Lys Gly Tyr
   1040 1045 1050
   Met Arg Ile Gln Gln Phe Arg Lys Ala Asp Gly Ser Tyr Ala Ala
   1055 1060 1065
   Trp Leu Ser Arg Gly Ser Ser Thr Trp Leu Thr Ala Phe Val Leu
   1070 1075 1080
   Lys Val Leu Ser Leu Ala Gln Glu Gln Val Gly Gly Ser Pro Glu
   1085 1090 1095
   Lys Leu Gln Glu Thr Ser Asn Trp Leu Leu Ser Gln Gln Gln Ala
   1100 1105 1110
   Asp Gly Ser Phe Gln Asp Leu Ser Pro Val Ile His Arg Ser Met
   1115 1120 1125
   Gln Gly Gly Leu Val Gly Asn Asp Glu Thr Val Ala Leu Thr Ala
   1130 1135 1140
   Phe Val Thr Ile Ala Leu His His Gly Leu Ala Val Phe Gln Asp
   1145 1150 1155
   Glu Gly Ala Glu Pro Leu Lys Gln Arg Val Glu Ala Ser Ile Ser
   1160 1165 1170
   Lys Ala Ser Ser Phe Leu Gly Glu Lys Ala Ser Ala Gly Leu Leu
   1175 1180 1185
   Gly Ala His Ala Ala Ala Ile Thr Ala Tyr Ala Leu Thr Leu Thr
   1190 1195 1200
   Lys Ala Pro Ala Asp Leu Arg Gly Val Ala His Asn Asn Leu Met
   1205 1210 1215
   Ala Met Ala Gln Glu Thr Gly Asp Asn Leu Tyr Trp Gly Ser Val
   1220 1225 1230
   Thr Gly Ser Gln Ser Asn Ala Val Ser Pro Thr Pro Ala Pro Arg
   1235 1240 1245
   Asn Pro Ser Asp Pro Met Pro Gln Ala Pro Ala Leu Trp Ile Glu
   1250 1255 1260
   Thr Thr Ala Tyr Ala Leu Leu His Leu Leu Leu His Glu Gly Lys
   1265 1270 1275
   Ala Glu Met Ala Asp Gln Ala Ala Ala Trp Leu Thr Arg Gln Gly
   1280 1285 1290
   Ser Phe Gln Gly Gly Phe Arg Ser Thr Gln Asp Thr Val Ile Ala
   1295 1300 1305
   Leu Asp Ala Leu Ser Ala Tyr Trp Ile Ala Ser His Thr Thr Glu
   1310 1315 1320
   Glu Arg Gly Leu Asn Val Thr Leu Ser Ser Thr Gly Arg Asn Gly
   1325 1330 1335
   Phe Lys Ser His Ala Leu Gln Leu Asn Asn Arg Gln Ile Arg Gly
   1340 1345 1350
   Leu Glu Glu Glu Leu Gln Phe Ser Leu Gly Ser Lys Ile Asn Val
   1355 1360 1365
   Lys Val Gly Gly Asn Ser Lys Gly Thr Leu Lys Val Leu Arg Thr
   1370 1375 1380
   Tyr Asn Val Leu Asp Met Lys Asn Thr Thr Cys Gln Asp Leu Gln
   1385 1390 1395
   Ile Glu Val Thr Val Lys Gly His Val Glu Tyr Thr Met Glu Ala
   1400 1405 1410
   Asn Glu Asp Tyr Glu Asp Tyr Glu Tyr Asp Glu Leu Pro Ala Lys
   1415 1420 1425
   Asp Asp Pro Asp Ala Pro Leu Gln Pro Val Thr Pro Leu Gln Leu
   1430 1435 1440
   Phe Glu Gly Arg Arg Asn Arg Arg Arg Arg Glu Ala Pro Lys Val
   1445 1450 1455
   Val Glu Glu Gln Glu Ser Arg Val His Tyr Thr Val Cys Ile Trp
   1460 1465 1470
   Arg Asn Gly Lys Val Gly Leu Ser Gly Met Ala Ile Ala Asp Val
   1475 1480 1485
   Thr Leu Leu Ser Gly Phe His Ala Leu Arg Ala Asp Leu Glu Lys
   1490 1495 1500
   Leu Thr Ser Leu Ser Asp Arg Tyr Val Ser His Phe Glu Thr Glu
   1505 1510 1515
   Gly Pro His Val Leu Leu Tyr Phe Asp Ser Val Pro Thr Ser Arg
   1520 1525 1530
   Glu Cys Val Gly Phe Glu Ala Val Gln Glu Val Pro Val Gly Leu
   1535 1540 1545
   Val Gln Pro Ala Ser Ala Thr Leu Tyr Asp Tyr Tyr Asn Pro Glu
   1550 1555 1560
   Arg Arg Cys Ser Val Phe Tyr Gly Ala Pro Ser Lys Ser Arg Leu
   1565 1570 1575
   Leu Ala  Thr Leu Cys Ser Ala  Glu Val Cys Gln Cys  Ala Glu Gly
       1580                 1585                 1590
   Lys Cys  Pro Arg Gln Arg Arg  Ala Leu Glu Arg Gly  Leu Gln Asp
       1595                 1600                 1605
   Glu Asp  Gly Tyr Arg Met Lys  Phe Ala Cys Tyr Tyr  Pro Arg Val
       1610                 1615                 1620
   Glu Tyr  Gly Phe Gln Val Lys  Val Leu Arg Glu Asp  Ser Arg Ala
       1625                 1630                 1635
   Ala Phe  Arg Leu Phe Glu Thr  Lys Ile Thr Gln Val  Leu His Phe
       1640                 1645                 1650
   Thr Lys  Asp Val Lys Ala Ala  Ala Asn Gln Met Arg  Asn Phe Leu
       1655                 1660                 1665
   Val Arg  Ala Ser Cys Arg Leu  Arg Leu Glu Pro Gly  Lys Glu Tyr
       1670                 1675                 1680
   Leu Ile  Met Gly Leu Asp Gly  Ala Thr Tyr Asp Leu  Glu Gly His
       1685                 1690                 1695
   Pro Gln  Tyr Leu Leu Asp Ser  Asn Ser Trp Ile Glu  Glu Met Pro
       1700                 1705                 1710
   Ser Glu  Arg Leu Cys Arg Ser  Thr Arg Gln Arg Ala  Ala Cys Ala
       1715                 1720                 1725
   Gln Leu  Asn Asp Phe Leu Gln  Glu Tyr Gly Thr Gln  Gly Cys Gln
       1730                 1735                 1740
   Val

## Description

### Technical field of the invention

The present invention relates among other things to a pharmaceutical composition comprising as the active ingredient, one or more antibodies that bind to human C-reactive protein (anti-CRP) for use in the treatment of cancer selected from the group consisting of glioblastomas, oligodendroglioma and astrocytomas, inhibiting growth of cancer selected from the group consisting of glioblastomas, oligodendroglioma and astrocytomas and/or inhibiting proliferation of cancer selected from the group consisting of glioblastomas, oligodendroglioma and astrocytomas in an individual.

### Background of the invention

Despite a rapid increase in the number of available immune treatments of various cancer types there still exist a need not only to develop methods of diagnosing cancer applying simple and well known techniques - there also exist a need to develop pharmaceutical compositions for use in the treating these cancer types.

At present, there exists no treatment of e.g. glioblastomas. Hence, it would be advantageous to develop a method of diagnosing this type of cancer and even more advantageous to develop a pharmaceutical composition for use in the treatment of e.g. this specific cancer type. EP 2 368 564 A1 discloses the medical use of anti-CRP antibodies as pharmaceutical active in inflammatory diseases.

### Summary

Thus, an object relates to e.g. a novel method of diagnosing cancer in an individual by applying antibodies that bind to human C1 inactivator (anti-C1 IA), antibodies that bind to human C Reactive Protein (anti-CRP) and/or antibodies that bind to Complement Component C4 (anti-C4).

Thus, one aspect relates to a method for detecting and/or screening and/or monitoring cancer in an individual, said method comprising determining:
a) a first parameter represented by the level of human C1 esterase inactivator (C1 IA) in at least one sample from the individual and
b) a second parameter represented by the level of human C reactive protein (CRP) in at least one sample from the individual
wherein the presence of the first parameter above a first-reference level and the presence of the second parameter above a second-reference level is an indication that the individual is likely to have cancer.

Yet another aspect is to provide a method of monitoring cancer in an individual, said method comprising the steps of
a) successively withdrawing samples from an individual with cancer over a period of time,
b) incubating a sample obtained in step a) with one or more antibodies that bind to human C1 IA (anti-C1 IA) and determining the human C1 IA level in said sample,
c) incubating a sample obtained in step a) with one or more antibodies that bind to human CRP (anti-CRP) and determining the human CRP level in said sample,
d) comparing the determined C1 IA of each of the samples in b)
e) comparing the determined CRP of each of the samples in c) and thereby determining:
   a. whether the level of C1 IA and the level of CRP is higher than the level of human C1 IA and the level of CRP in the immediately preceding mixture, which is indicative of progress of cancer, or
   b. whether the level of C1 IA and the level of CRP is essentially equal to the level of C1 IA and the level of CRP in the immediately preceding mixture, which is indicative of steady stage of cancer, or
   c. whether the level of C1 IA and the level of CRP is lower than the level of C1 IA and the level of CRP in the immediately preceding mixture, which is indicative of recovery of cancer.

Still another aspect is to provide a kit comprising (i) one or more antibodies that bind to C1 IA (anti-C1 IA), one or more antibodies that bind to human CRP (anti-CRP) and/or one or more antibodies that bind to human C4 (anti-C4) and (ii) instructions for use of said kit.

A further aspect relates to a composition comprising, as the active ingredient, one or more antibodies that bind to human C1 IA (anti-C1 IA), one or more antibodies that bind to human CRP (anti-CRP) and/or one or more antibodies that bind to human C4 (anti-C4)

Yet another aspect relates to a pharmaceutical composition comprising, as the active ingredient, one or more antibodies that bind to human C1 IA (anti-C1 IA), one or more antibodies that bind to human CRP (anti-CRP) and/or one or more antibodies that bind to human C4 (anti-C4) and a pharmaceutically acceptable carrier, diluent and/or excipient.

A still further aspect relates to a diagnostic method or kit based upon the same variants of anti-C1 IA, anti-CRP and anti-component C4 as are used in cancer therapy, thereby allowing identification of individuals who are receptive for the cancer therapy. This aspect may alternatively be defined as a method for treatment of cancer, wherein the same variants of anti-C1 IA, anti-CRP and anti-component C4 that are used in a diagnostic method or kit as described herein are used for treatment of individuals identified in a detection and/or screening and/or monitoring method of the invention.

### Brief description of the figures

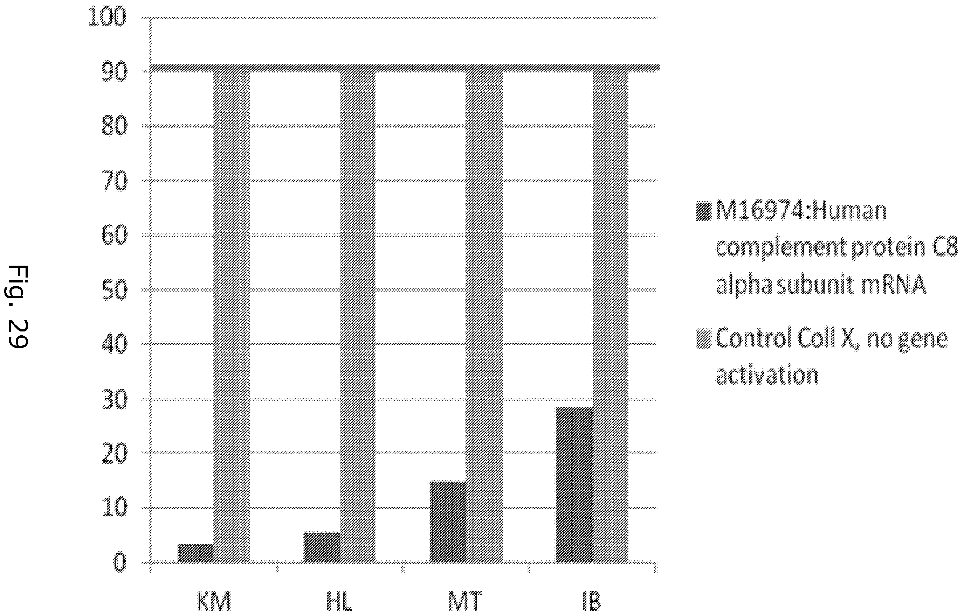

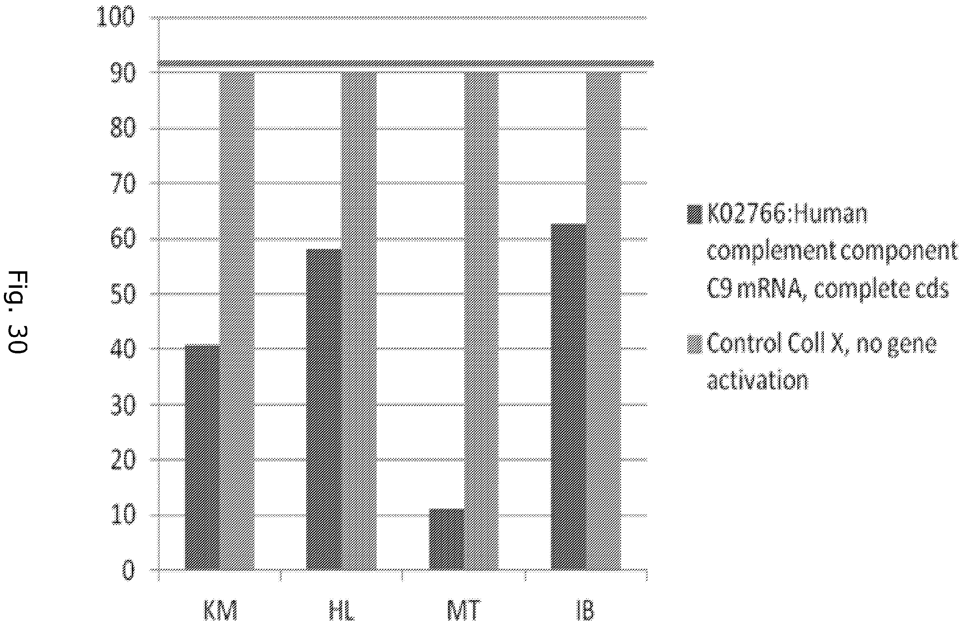

Figure 1 shows Laurell Rocket immunoelectrophoresis, Agarose gel containing 2% rabbit anti-C1 IA (BehringWerke,Germany). The C1 IA immunogen is applied in the left wells in serial dilution, a control with low dose C1 IA immunogen in 6 middle wells, the C1 IA of pool Standard C1 IA from donors is applied in serial dilution in the right wells.

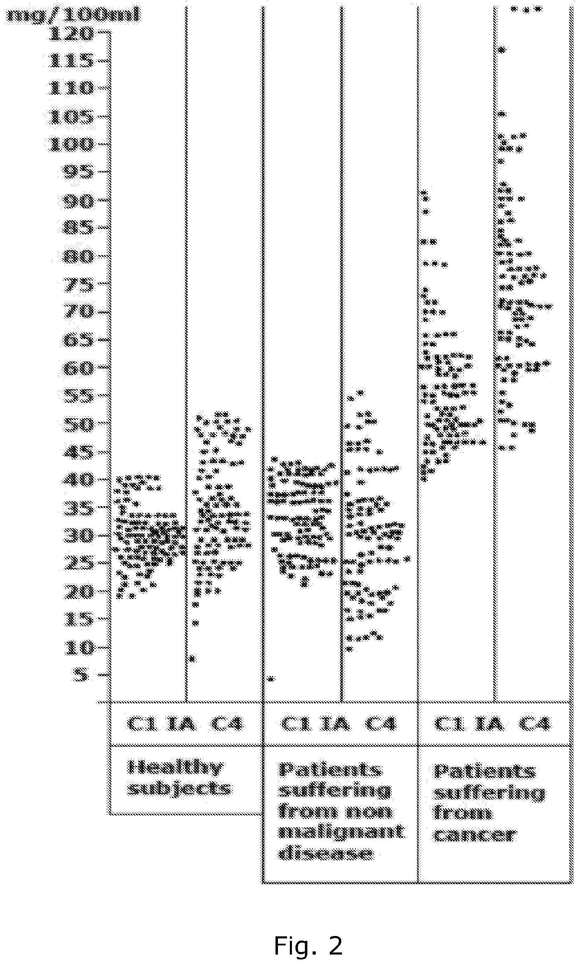

Figure 2 shows measurement of C1 IA and complement component C4 in serum samples using rabbit anti-C1 IA - and anti-C4 antibodies using Laurell rocket immune electrophoresis. The first group consists of measurements of serum from healthy subjects (C1 IA ranges from 15 to 40 mg%, and C4 ranges from 15 to 50 mg% in healthy subjects). The second group of serum samples was from patients with non-malignant diseases, essentially showing the same levels of C1 IA and C4 as the healthy subjects. The third group consists of serum samples from patients with different types of malignant cancers (carcinomas), showing overall higher levels of C1 IA and C4, with some overlapping between the non-malignant patient group.

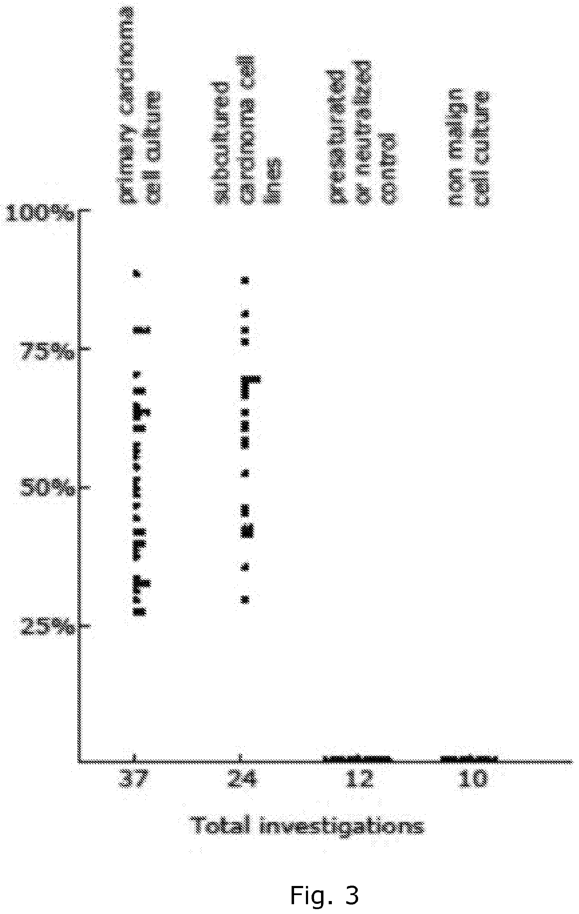

Figure 3 shows Leitz MPV2 Cytophotometer measurement of C1 IA, colum1 shows carcinoma cell cultures from various carcinoma biopsies in explants, results given in number of cells in percentage for each carcinoma cell culture tested (37) carcinoma cell cultures from explants from biopsies from patients with various types of carcinomas, using rabbit anti-C1 IA Fluorescein isothiocyanate (FITC). Column 2 shows 24 carcinoma cell sub-cultured for several days, and re-tested for the presence of C1 IA using anti-C1 IA FITC. Column 3 shows 12 cultures (controls) pre-incubated with unlabelled rabbit anti-C1 IA, washed and then incubated with rabbit anti-C1 IA FITC. In column 4, ten (10) cell cultures from non-malignant explants were incubated with rabbit anti-C1 IA FITC and showed no binding of FITC labeled anti-C1 IA.
Figure 4a shows an overview of the innate immune complex complement system's areas of "blocking" by C1 inhibitor affecting C1rs activation, which is blocked from acting with component C4 (in-circled three areas in upper left corner), in-circled C4 indicates no activation of C4 to C4b and no activation of C2 to C9. Blocking of CRP is inhibiting C5 (in-circled). "X" indicates the C5 blocked part of the complement system. The following crossed circle indicates no perforation due to inhibition of C5b - C9 lysis effect.

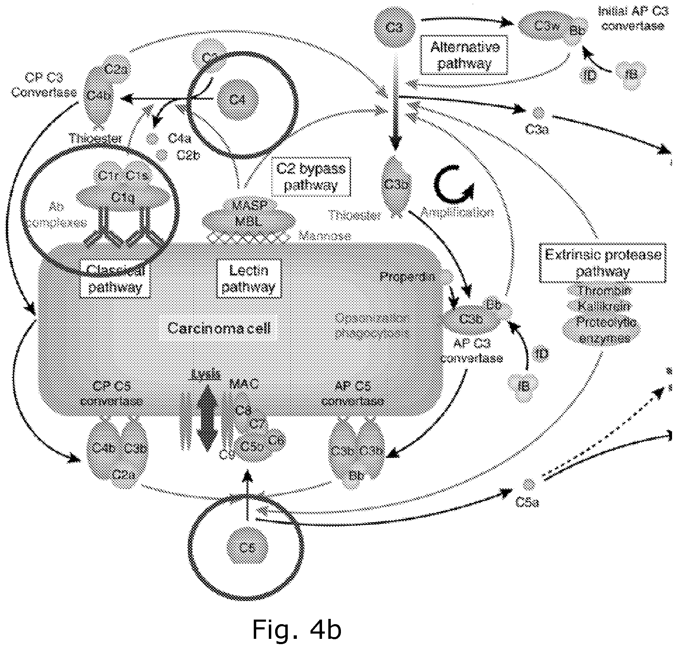

Figure 4b shows an overview of the innate immune complex complement system's areas de-blocked by recombinant human anti-C1 IA and recombinant human anti-CPR infused/injected into the carcinoma patient. A) The recombinant anti-C1-IA activates C1rs, and de-blocked C4 now splitting to C4b allowing activation of complement component C2 all the way to C9. B) Recombinant human anti-CRP activates/de-blocks the complement component C5 (in-circled) indicating the deblocking of complement component C5 that converts to C5b and allows activation of C6-C9 components of the complement system. The following arrow indicates lysis effect of the "built - up" C9 which perforate the carcinoma cell resulting in cell lysis and Cell death caused by the C9 perforation.

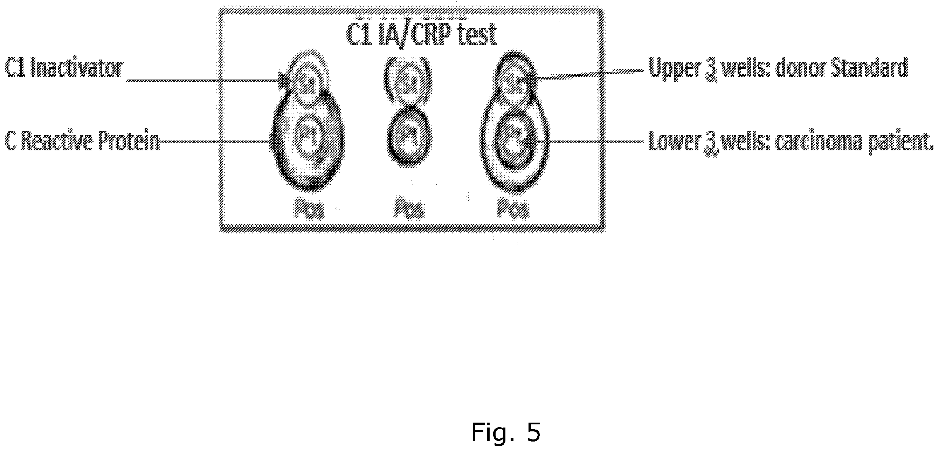

Figure 5 shows a modified Double Radial immunodiffusion (Double-RID) using the Mancini method. Mancini double immunodiffusion is a diffusion technique where the agar gel contains pig anti-C1 IA and pig anti-CRP (recently detected) consisting of purified immunogen from human carcinoma cells. The upper 3 wells contains 25 ul standard donor serum showing a C1 IA reaction and the lower wells contains serum from 3 cancer patients with various concentration of CRP and C1 IA. The diffusion is allowed overnight in refrigerator and examined the next day. It can be seen that the lower large radial rings crosses the upper C1 IA ring from the standard donor. These were later on in other tests identified to be CPR using anti-CRP originated from the carcinoma immunogen, directly harvested together with the C1 IA and purified using methods designed to purify C1 IA.
Figure 6a shows TRITS fluorescence of rat RG-2 glioma cells viewed in fluorescence microscope
Figure 6b shows TRITS fluorescence of rat RG-2 glioma cells viewed in fluorescence microscope
Figure 7a shows TRITS fluorescence of rat NS-1 (CNS-1) rat glioma cells viewed in fluorescence microscope
Figure 7b shows TRITS fluorescence of rat NS-1 (CNS-1) rat glioma cells viewed in fluorescence microscope.
Figure 8 shows green fluorescence derived from the incorporated GFP in the cells with the green fluorescence especially visible in the nucleus and in the cytoplasma of the glioma cells. The red TRITS labeled anti-CRP appears coating the periphery of the RG-2 glioma cells giving evidence of an plasma membrane located CRP reacting with rabbit anti-human (known to cross-react with rat ant-CRP).
Figure 9 shows an C1-IA coat of rat RG-2 glioma cells. Dapi stain is used for showing nuclei, and staining with Dapi together with anti-C1 IA (plus sandwich antibody (TRITS) provides evidence that the nuclei of these rat glioma cells is not stained with anti C1-IA and that C1 IA is merely present in the cell coat.
Figure 10a shows TRITS fluorescence of rat RG-2 glioma cells viewed in fluorescence microscope.
Figure 10b shows TRITS fluorescence of rat RG-2 glioma cells viewed in fluorescence microscope.
Figure 11a shows TRITS fluorescence of rat NS-1 (CNS-1) rat glioma cells expressing CRP, viewed in fluorescence microscope
Figure 11b shows TRITS fluorescence of rat NS-1 (CNS-1) rat glioma cells showing CRP expression, viewed in fluorescence microscope
Figure 12 shows Dark field microscopic exam of GFP incorporated rat RG-2 glioma cells giving evidence of the live RG-2 cells in the culture.
Figure 13 shows the green fluorescence derived from the incorporated GFP in the cells with the green fluorescence especially visible in the nucleus and in the cytoplasma of the glioma cells. The red TRITS labeled antibody appears coating the plasma membrane of the RG-2 glioma cells giving evidence of an plasma membrane -located coated protein, C-Reactive Protein (CRP) reacting with rabbit anti human (known to cross-react with rat anti C-Reactive Protein) and by using the sandwich method, a TRITS labeled secondary antibody is showing the location of the CRP on the cells.
Figure 14 shows C1 inactivator coat of rat RG-2 glioma cells. Upper left picture, merged GFP green stain is used for showing nuclei and locating binding of C1 inactivator to rat RG-2 glioma cells, upper left picture rat RG-2 glioma cells showing C1 IA as indicated by anti C1 IA antibody, plus secondary sandwich antibody labelled with TRITS. Lower right picture reveals GFP gene staining in the RG-2 cells.

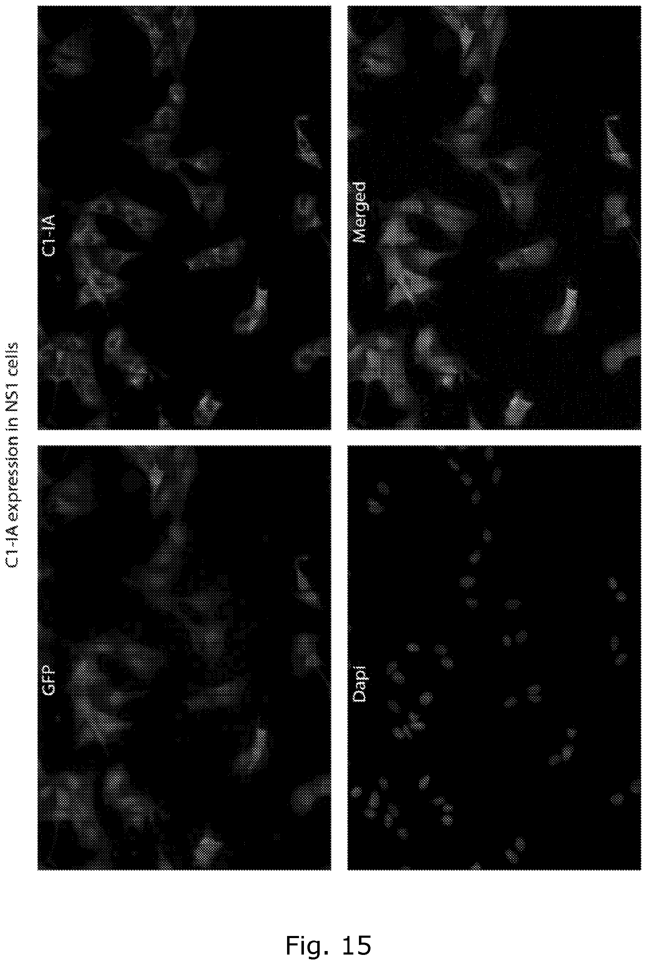

Figure 15 shows four pictures showing NS-1 rat glioma cells identified as live cells with GFP gene in upper right picture, and Dapi blue nucleus staining in lower right picture.The glioma cells were made impermeable for immunostaining with anti C1 IA TRITS in upper left picture. In the lower left picture the immune staining with primay anti C1 IA antibody and TRITS labelled secondary antibody staining (sandwich technique was merged with Dapi blue staining indicating the nucleus of the cells in contrast to the outer coat showing expression of C1 IA.

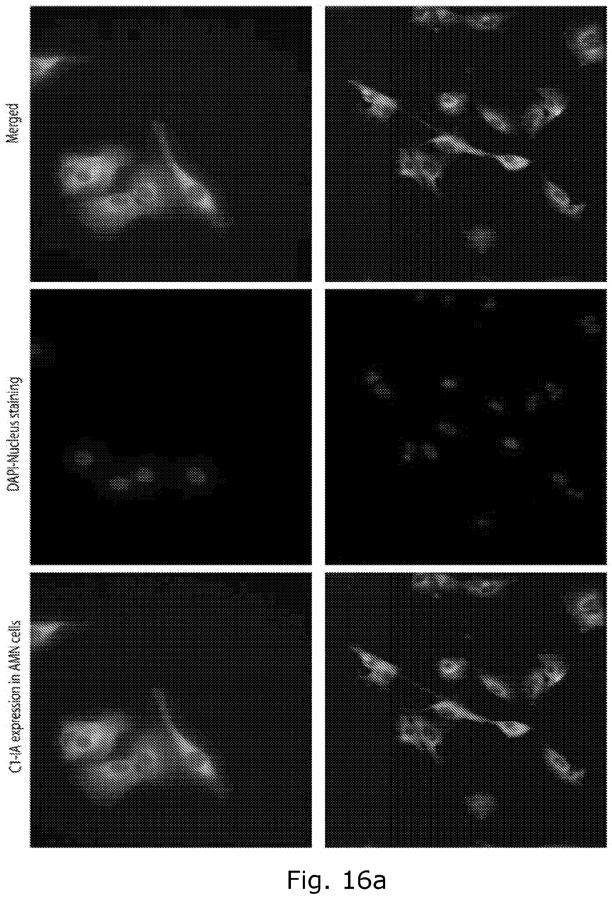

Figure 16a shows C1 inactivator coat expressed on human AMN glioma (glioblastoma) cells shown by incubation of the cells with anti C1 inactivator and using secondary FITC labelled antibody (sandwich method), done on using impermeabilization of the cells cells as shown in left upper and lower picture at different magnifications. The Dapi blue staining was performed on the same cells and shown in the on upper and lower mid section. The upper and lower pictures on the right side shows a merged versions of the C1 IA expression and Dapi nucleu staining, giving evidence that the plasma membrane of the cells is coated with C1 IA.

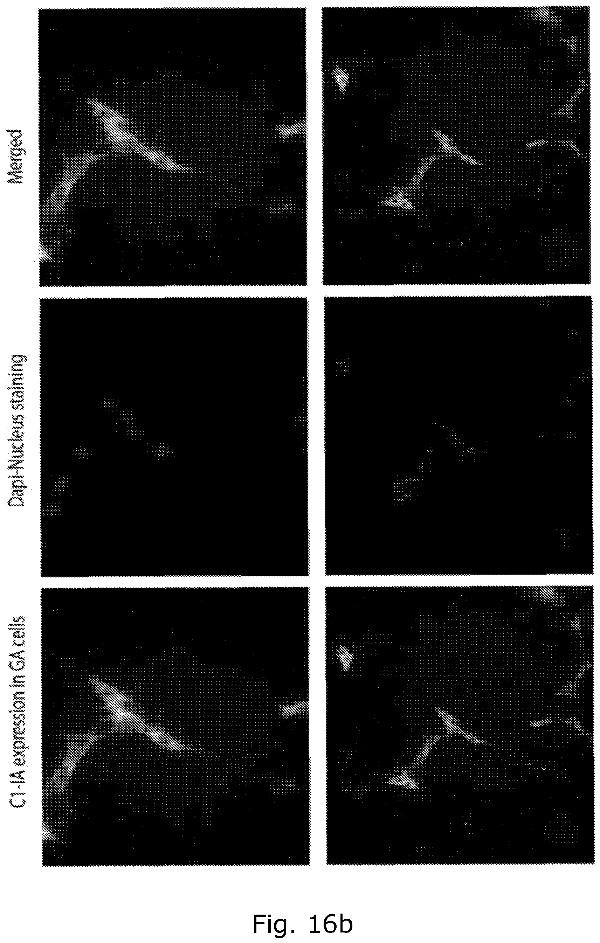

Figure 16b shows C1 inactivator coat expressed on human GA glioma (glioblastoma) cells shown by incubation of the cells with anti C1 inactivator and using secondary FITC labelled antibody (sandwich method), done on using impermeabilization of the cells cells as shown in left upper and lower picture at different magnifications. The Dapi blue staining was performed on the same cells and shown in the on upper and lower mid section. The upper and lower pictures on the right side shows a merged versions of the C1 IA expression and Dapi nucleu staining, giving evidence that the plasma membrane of the cells is coated with C1 IA.

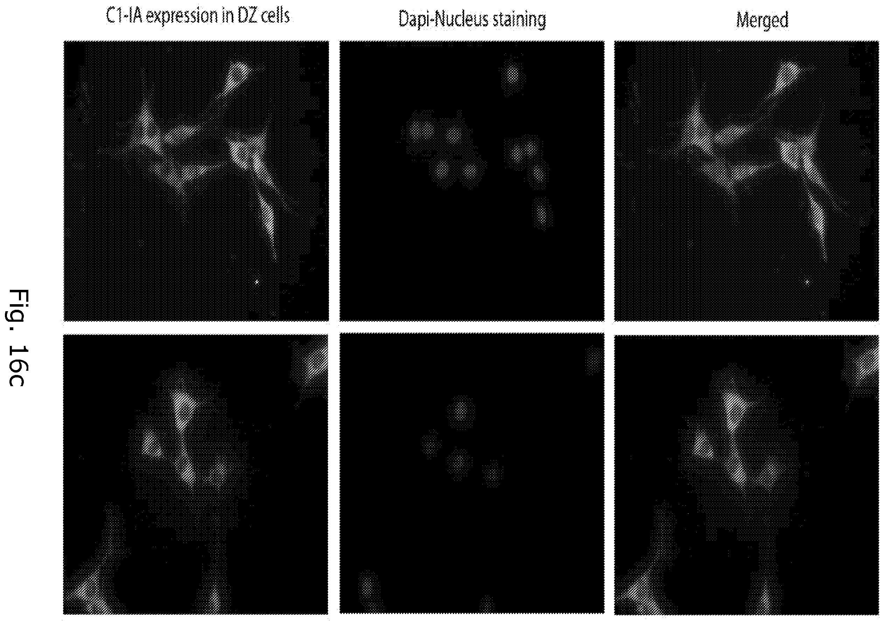

Figure 16c shows C1 inactivator coat expressed on human DZ glioma (glioblastoma) cells shown by incubation of the cells with anti C1 inactivator and using secondary FITC labelled antibody (sandwich method), done on using impermeabilization of the cells as shown in left upper and lower picture at different magnifications. TheDapi blue staining was performed on the same cells and shown in the on upper and lower mid section. The upper and lower pictures on the right side show merged versions of the C1 IA expression and Dapi nucleu staining, giving evidence that the plasma membrane of the cells is coated with C1 IA.
Figure 17 shows a weak C1 IA expression in the nucleus of the human skin fibroblasts, as opposed to the cancer cells (e.g., rat glioma and human glioma/glioblastoma), where the C1 IA gave a strong signal in the cytoplasm and in the plasma membranes of the cancer cells.
Figure 18 shows in the three images labelled A and one picture labelled B, CRP in fibroblast (benign skin fibroblasts), incubation with anti CRP and with secondary FITC labelled antibody without treatment for impermeabilization. There is no significant CRP expression observed. The pictures labelled CRP in GA cells (mid lower picture; human GA glioma/glioblastoma cells) and CRP in AMN cells (lower right; human AMN glioma/glioblastoma cells) show CRP expression. There is in these two last pictures a significant binding of the CRP.
Figure 19 and 20 show the affymetrix Gene Chip Array.
Figure 21 shows gene read out of C1r NCBI X03084 Human mRNA for C1r mRNA of the complement system from each of the four (4) patients: KM, HL, MT, and IB.
   Dark purple = Read out level of C1 inhibitor (C1 IA): ≥ 1 log over border line = C1 IA Gene Acitivity present in all four (4) different mesenchymal cell cultures Light purple = Read out level of Control X, with not gene activation.
   Red border line: ≤90 read out = no gene activity of collagen X
Figure 22 shows gene read out of C1q NCBI X03084 Human mRNA for C1q B-chain of the complement system from each of the four (4) patients: KM, HL, MT, and IB.
   Dark purple = Read out level of C1q-B chain ≤90 = no C1q gene activity
   Light purple = Read out level of Control X, ≤90 = with not gene activation.
   Red border line: ≤90 read out = no gene activity of C1q-B or collagen type X
Figure 23 shows gene read out of C1r NCBI M14048, Human mRNA for C1r mRNA of the complement system from each of the four (4) patients: KM, HL, MT, and IB.
   Dark purple = Read out level of C1r: significantly ≥ 1 log over border line = Gene Acitivity. Light purple = Read out level of Control X, with not gene activation. Border line: ≤90 read out = no gene activity of collagen X.
Figure 24 shows gene read out of C4b NCBI U24578. Human mRNA for C4b of the complement system from each of the four (4) patients: KM, HL, MT, and IB.
   Dark purple = Read out level of C4b chain ≤90 = no C4b gene activity Light purple = Read out level of complement Control X, ≤90 = with not gene activation. Border line: ≤90 read out = no gene activity of C4b or collagen type X
Figure 25 shows Gene Read out of C5 NCBI M57729. C5b is a cleaved part of the Human mRNA for C5 of the complement system from each of the four (4) patients, KM, HL, MT, and IB.
   Dark purple = Read out level of C5 chain ≤90 = no C5 gene activity
   Light purple = Read out level of complement Control X, ≤90 = with not gene activation. Border line: ≤90 read out = no gene activity of C5 or collagen type X.
Figure 26 shows gene read out of complement component C6 NCBI X72177. C6 of the complement system from each of the four (4) patients: KM, HL, MT, and IB.
   Dark purple = Read out level of C6 chain ≤90 = no C6 gene activity
   Light purple = Read out level of complement Control X, ≤90 = with not gene activation.
   Border line: ≤90 read out = no gene activity of C6 or collagen type X.
Figure 27 shows gene read out of complement component C7 NCBI J03507.
   C7 of the complement system from each of the four (4) patients: KM, HL, MT, and IB.
   Dark purple = Read out level of C7 chain ≤90 = no C7 gene activity
   Light purple = Read out level of complement Control X, ≤90 = with not gene activation.
   Border line: ≤90 read out = no gene activity of C7 or collagen type X.

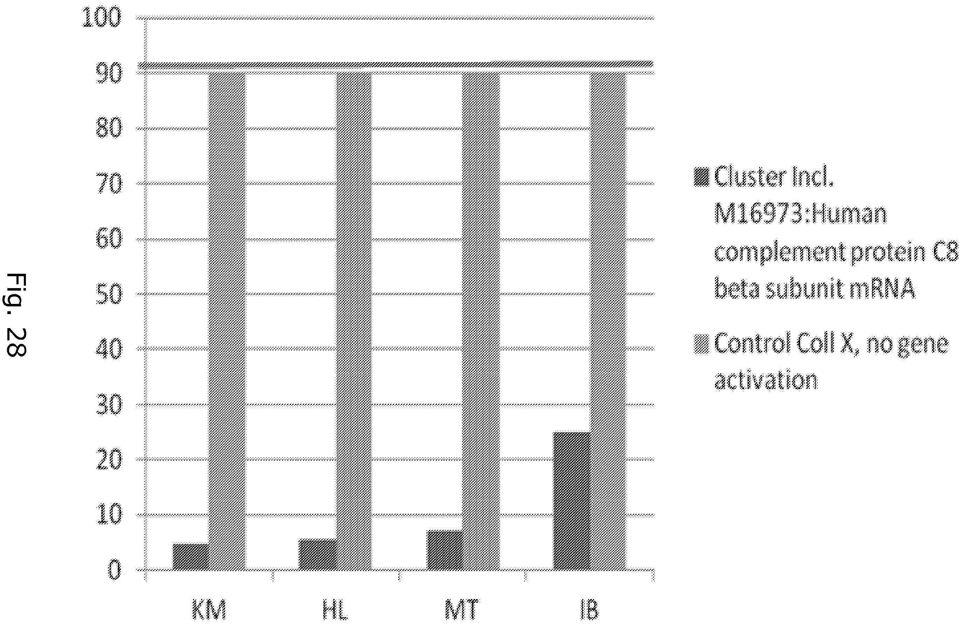

Figure 28 shows Gene Read out of complement component C8 (beta) NCBI M16973. C8 of the complement system from each of the four (4) patients: KM, HL, MT, and IB.
   Dark purple = Read out level of C8 chain ≤90 = no C8 (beta) gene activity
   Light purple = Read out level of complement Control X, ≤90 = with not gene activation.
   Border line: ≤90 read out = no gene activity of C8 or collagen type X
Figure 29 shows gene read out of complement component C8 (alpha) NCBI M16974.
   C8 of the complement system from each of the four (4) patients: KM, HL, MT, and IB.
   Dark purple = Read out level of C8 chain ≤90 = no C8 (alpha) gene activity
   Light purple = Read out level of complement Control X, ≤90 = with no gene activation.
   Border line: ≤90 read out = no gene activity of C8 (alpha) or collagen type X'
Figure 30 shows gene read out of complement component C9 NCBI K02766.
   C9 of the complement system from each of the four (4) patients: KM, HL, MT, and IB.
   Dark purple = Read out level of C9 chain ≤90 = no C9 gene activity
   Light purple = Read out level of complement Control X, ≤90 = with not gene activation.
   Border line: ≤90 read out = no gene activity of C9 or collagen type X.

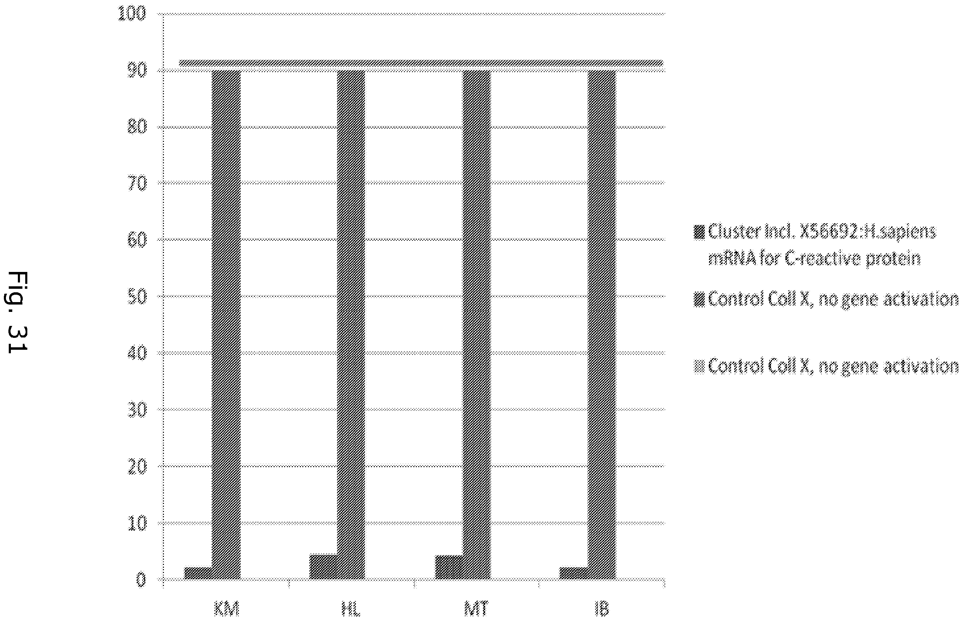

Figure 31 shows gene read out of C-Reactive PI X56692. C-Reactive protein (CRP) on benign mesenchymal cells (chondroblasts) from each of the four (4) patients: KM, HL, MT, and IB from which the cartilage was harvested and the cells cultured using the explants method, show no gene activation of CRP
   Dark purple = Read out level of CRP ≤90 = no CRP (alpha) gene activity
   Light purple = Read out level of complement Control X, ≤90 = with not gene activation.
   Border line: ≤90 read out = no gene activity of C-Reactive Protein or collagen type X.

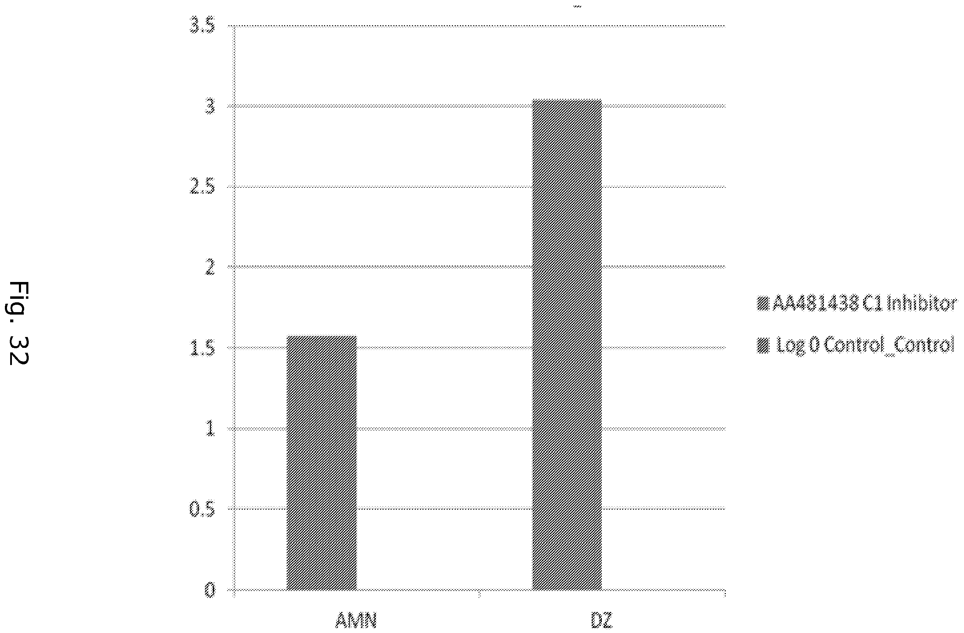

Figure 32 shows NCBI 144838 mRNA C1 inhibitor in glioblastoma from patients AMN and DZ. Values in glioblastoma exceeding Log 1 over normal brain tissue in both AMN and DZ.

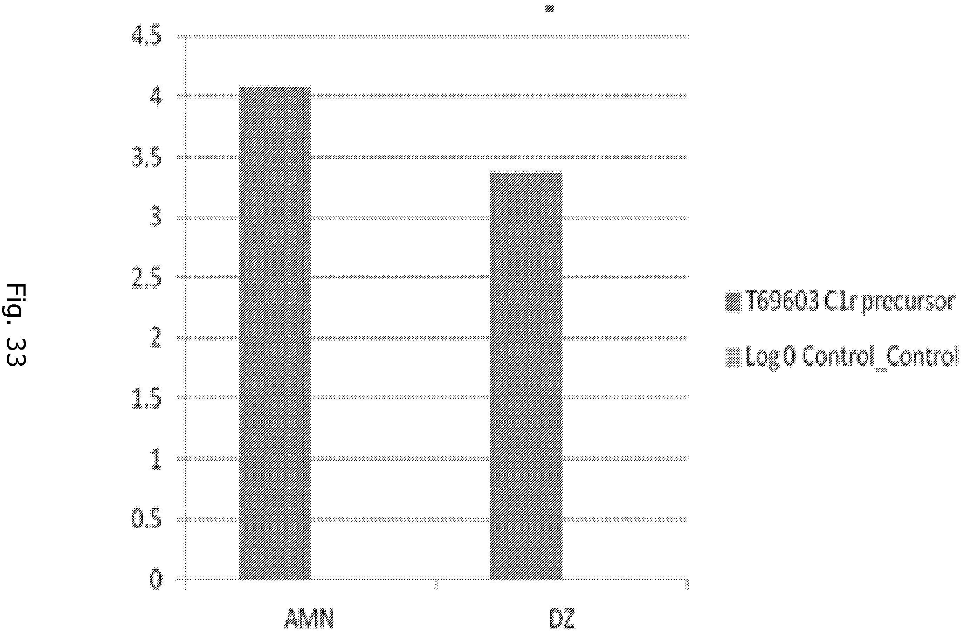

Figure 33 shows NCBI T69603 mRNA C1r precursor in glioblastoma from patients AMN and DZ. Values in glioblastoma exceeding Log 1 over normal brain tissue in both AMN and DZ.

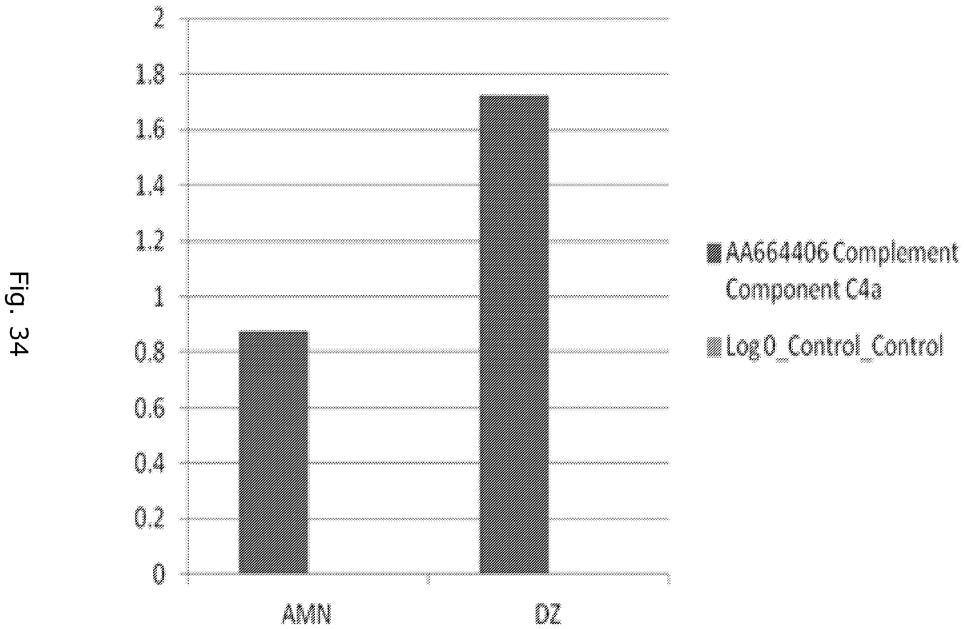

Figure 34 shows NBCI AA664406 mRNA Complement Component C4a in glioblastoma from patients AMN and DZ. Values in glioblastoma exceeding Log 1 over normal brain tissue in both AMN and DZ.

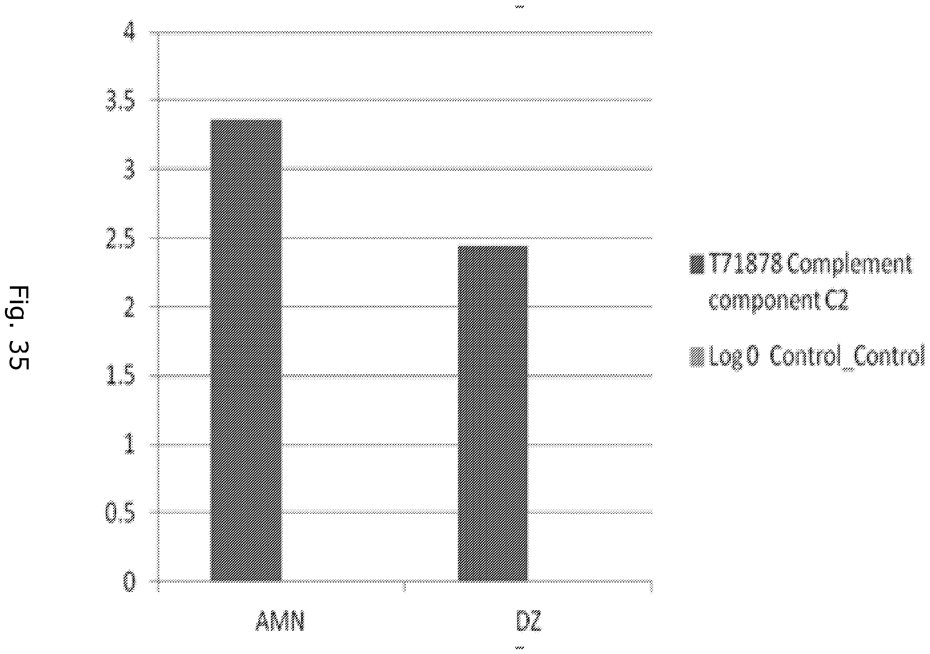

Figure 35 shows NCBI T71878 mRNA NBCI AA664406 Complement Component C2 in glioblastoma from patients AMN and DZ Values in glioblastoma exceeding Log 1 over normal brain tissue in both AMN and DZ.

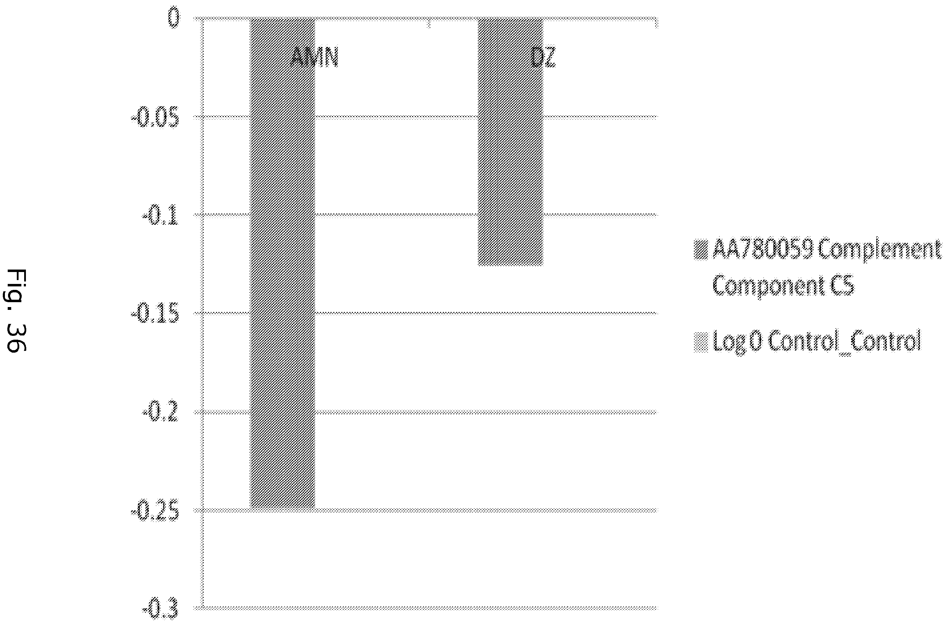

Figure 36 shows NCBI AA780059 mRNA NBCI Complement Component C5 in glioblastoma from AMN and from DZ Values in glioblastoma negative Log under normal brain tissue in both AMN and DZ.

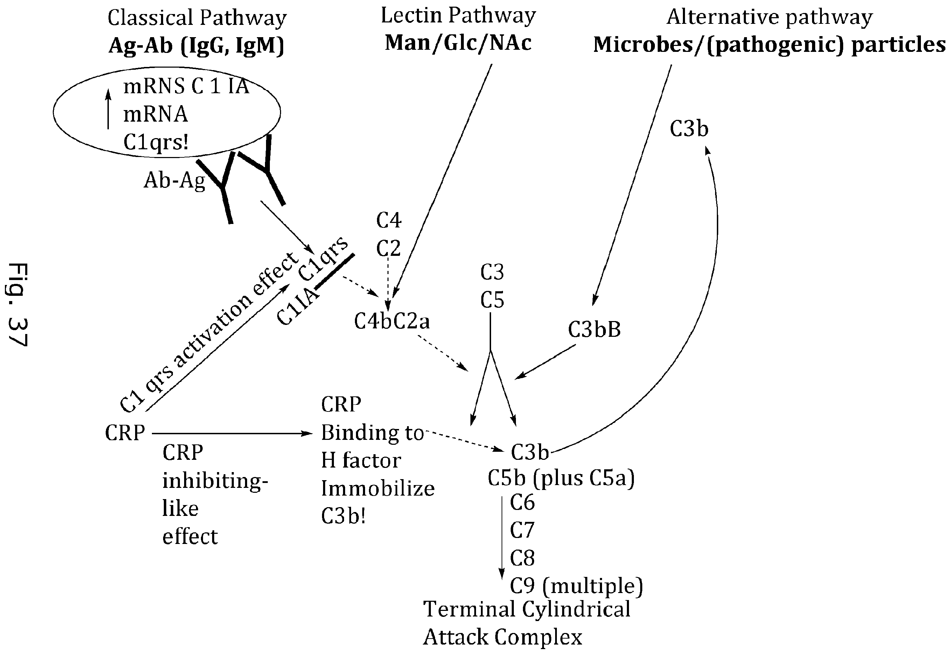

Figure 37 shows an overview of the complement system and how C1 IA and/or CRP inhibit the complement system.

The present invention will now be described in more detail in the following.

### Detailed description of the invention

### Definitions

Prior to discussing the present invention in further detail, the following terms and conventions will first be defined:
Antibody: In the present context, an antibody is a protein that specifically binds a corresponding antigen. Antibodies may particularly stem from the immune system of e.g. mammals, and may be directed towards antigens related to foreign bodies. An antibody is an intact immunoglobulin having two light and two heavy chains. Thus, a single isolated antibody or fragment may be originating from the non-limiting list of a polyclonal antibody, a monoclonal antibody, a synthetic antibody, a recombinant antibody, a chimeric antibody, a heterochimeric antibody, or a humanized antibody. The term antibody is used both to refer to a homogeneous molecular mixture or a mixture such as a serum product made up of a plurality of different molecular entities.

In the present context C1 inactivator (C1 IA) is a protease inhibitor belonging to the serpin superfamily. Its main function is the inhibition of the complement system to prevent spontaneous activation. C1-inhibitor is an acute-phase protein that circulates in blood. In the present context the terms C1 inactivator, C1-inhibitor, C1-inh or C1 esterase inhibitor are used herein interchangeably. C1 inactivator may be abbreviated: C1 IA or C1-IA. The protein is a human protein - thus, the term "human" may be inserted in from of C1 IA. C1 IA is an alpha 1 neuraminoglycotprotein amino acid protein with a molecular weight of 110-130 kDa. The molecular weight is variable due to potential differences in glycosylation of the protein. The C1 IA protein is a C1-inhibitor (C1-inh, C1 esterase inhibitor) which is a protease inhibitor belonging to the serpin superfamily. Its main function is the inhibition of the complement system to prevent spontaneous activation and it is an acute-phase protein that circulates in blood at levels of around 0.25 g/L. The levels rise ∼2-fold during inflammation. C1-inhibitor irreversibly binds to and inactivates C1r and C1s proteases in the C1 complex of classical pathway of complement. Aliases for the protein are SERPING1, C1IN, C1INH, C1NH, HAE1, HAE2, serpin family G member 1. In humans, the C1 IA protein is encoded by a nucleic acid sequence encoding the amino acid sequence shown in SEQ ID NO:1. Genebank accession number: X54486 and Gene ID: 710 in NCBI.

In the present context C-Reactive Protein (CRP) is an annular (ring-shaped), pentameric protein found in blood plasma, whose levels rise in response to inflammation. It is an acute-phase protein of hepatic origin that increases following interleukin-6 secretion by macrophages and T cells. Its physiological role is to bind to lysophosphatidylcholine expressed on the surface of dead or dying cells (and some types of bacteria) in order to activate the complement system via the C1Q complex. CRP is synthesized by the liver in response to factors released by macrophages and fat cells (adipocytes). C Reactive Protein may be abbreviated: CRP. The protein is a human protein - thus, the term "human" may be inserted in front of CRP. The CRP protein belongs to the pentaxin family. It is involved in several host defense related functions based on its ability to recognize foreign pathogens and damaged cells of the host and to initiate their elimination by interacting with humoral and cellular effector systems in the blood. Consequently, the level of this protein in plasma increases greatly during acute phase response to tissue injury, infection, or other inflammatory stimuli. In Figure 37 it can be seen that C-Reactive Protein enhances the C1qrs complex, and via its binding to H factor CRP immobilizes complement component factor C3b. In humans, the CRP protein is encoded by a nucleic acid sequence encoding the amino acid sequence shown in SEQ ID NO:2. Genbank accession; X56692 and GENE ID: Gene ID: 1401 in NCBI).

Complement Component C4 (C4) in humans, is a protein involved in the intricate complement system, originating from the human leukocyte antigen (HLA) system. It serves a number of critical functions in immunity, tolerance, and autoimmunity with the other numerous components. Furthermore, it is a crucial factor in connecting the recognition pathways of the overall system instigated by antibody-antigen (Ab-Ag) complexes to the other effector proteins of the innate immune response. Complement Component C4 may be abbreviated: C4. The protein is a human protein - thus, the term "human" may be inserted in from of C4. C4 is Also known as CH; C4F; CO4; C4B1; C4B2; C4B3; C4B5; C4BD; C4B12; C4B_2; CPAMD3. Complement factor 4, part of the classical activation pathway. The protein is expressed as a single chain precursor which is proteolytically cleaved into a trimer of alpha, beta, and gamma chains prior to secretion. The trimer provides a surface for interaction between the antigen-antibody complex and other complement components. The alpha chain may be cleaved to release C4 anaphylatoxin, a mediator of local inflammation. Deficiency of this protein is associated with systemic lupus erythematosus. This gene localizes to the major histocompatibility complex (MHC) class III region on chromosome 6. Varying haplotypes of this gene cluster exist, such that individuals may have 1, 2, or 3 copies of this gene. In addition, this gene exists as a long form and a short form due to the presence or absence of a 6.4 kb endogenous HERV-K retrovirus in intron 9.

In humans, the C4 protein is encoded by a nucleic acid sequence encoding the amino acid sequence shown in SEQ ID NO:3. Gene ID: 721 in NCBI and Genebank accession U24578.

The term "antibody which binds to human C1 inactivator (C1 IA)" (anti-C1 IA) as used herein, refers to any antibody binding an epitope on the extracellular part of C1 IA.

The term "antibody which binds to human C Reactive Protein (CRP)" (anti-CRP) as used herein, refers to any antibody binding an epitope on the extracellular part of CRP.

The term "antibody which binds to Complement Component C4 (anti-C4)" as used herein, refers to any antibody binding an epitope on the extracellular part of C4.

The term "epitope" means a protein determinant capable of specific binding to an antibody. Epitopes usually consist of surface groupings of molecules such as amino acids, sugar side chains or a combination thereof and usually have specific three dimensional structural characteristics, as well as specific charge characteristics. Conformational and non-conformational epitopes are distinguished in that the binding to the former but not the latter is lost in the presence of denaturing solvents. The epitope may comprise amino acid residues which are directly involved in the binding, and other amino acid residues, which are not directly involved in the binding, such as amino acid residues which are effectively blocked or covered by the specific antigen binding peptide (in other words, the amino acid residue is within the footprint of the specific antigen binding peptide).

The complement system is a part of the immune system that enhances (complements) the ability of antibodies and phagocytic cells to clear microbes and damaged cells from an organism, promotes inflammation, and attacks the pathogen's plasma membrane. It is part of the innate immune system which is not adaptable and does not change over the course of an individual's lifetime. It can be recruited and brought into action by the adaptive immune system.

In the present context the term "anti-C1 IA" is to be understood as an antibody molecule that binds to human C1 inactivator (C1 IA).

In the present context the term "anti-CRP" is to be understood as an antibody molecule that binds to human C Reactive Protein.

In the present context the term "anti-C4" is to be understood as an antibody molecule that binds to human Complement Component C4 (C4).

The terms "monoclonal antibody", "monoclonal Ab", "monoclonal antibody composition", "mAb", or the like, as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be produced by a hybridoma which includes a B cell obtained from a transgenic or transchromosomal non-human animal, such as a transgenic mouse, having a genome comprising a human heavy chain transgene and a light chain transgene, fused to an immortalized cell.

### Diagnosis/screening/monitoring

One aspect provides a method for method for detecting and/or screening and/or monitoring cancer in an individual suitable to facilitate the early diagnosis of a cancer.

It is another aspect to provide a method for monitoring the recurrence of a cancer, status of a cancer or the effect of cancer treatment in an individual.

In one example the disclosure relates to a method for detecting and/or screening and/or monitoring cancer in an individual, said method comprising determining:
a) a first parameter represented by the level of C1 esterase inactivator (C1 IA) in at least one sample from the individual and
b) a second parameter represented by the level of C reactive protein (CRP) in at least one sample from the individual
wherein the presence of the first parameter above a first-reference level and the presence of the second parameter above a second-reference level is an indication that the individual is likely to have cancer.

Monitoring cancer also means recurrence of cancer.

The method may also comprise determining:
c) a third parameter represented by the level of human complement component C4 (C4) in at least one sample from the individual,
and wherein the presence of the first parameter above a first-reference level, the presence of the second parameter above a second-reference level and the presence of the third parameter above a third-reference level is an indication that the individual is likely to have cancer.

In a further aspect the disclosure relates to a method for detecting and/or screening and/or monitoring cancer in an individual, said method comprising determining:
a) a first parameter represented by the concentration of C1 esterase inactivator (C1 IA) in at least one excreta from the individual and
b) a second parameter represented by the concentration of human complement component C4 (C4) in at least one excreta from the individual
wherein the presence of the first parameter at or above a predetermined first discrimination value and the presence of the second parameter above a second predetermined discrimination value is an indication that the individual has a high likelihood of having cancer.

The terms excreta and sample are used herein interchangeably.

The level of human C1 IA, human CRP and/or human C4 may be the concentration of human C1 IA, human CRP and/or human C4.

It has surprisingly been found by the inventors that high levels of C1 esterase inactivator (C1 IA) in combination with high levels of C reactive protein in human excreta may be indicative of cancer.

It is therefore possible to provide an easy and inexpensive method for early detection and monitoring of a cancer in an individual or large populations using the method of the present invention.

In order not to obtain any false-positive results it is important to determine a reference level, which divides the tested individuals in a group having either a high or low likelihood of having cancer.

The first-reference level is established by measuring the concentration of C1 esterase inactivator (C1 IA) in both a healthy control population and a population with known cancer and thereby determining the discriminating value. The discriminating value identifies the cancer population with either a predetermined specificity or a predetermined sensitivity or both, and is based on an analysis of the relation between the concentration values and the known clinical data of the healthy control population and the cancer patient population.

The second-reference level is established by measuring the concentration of human C reactive protein (CRP) in both a healthy control population and a population with known cancer and thereby determining the discriminating value. The discriminating value identifies the cancer population with either a predetermined specificity or a predetermined sensitivity or both, and is based on an analysis of the relation between the concentration values and the known clinical data of the healthy control population and the cancer patient population.

The third-reference level is established by measuring the concentration of human complement component C4 (C4) in both a healthy control population and a population with known cancer and thereby determining the discriminating value. The discriminating value identifies the cancer population with either a predetermined specificity or a predetermined sensitivity or both, and is based on an analysis of the relation between the concentration values and the known clinical data of the healthy control population and the cancer patient population.

The discriminating value determined in this manner is valid for the same experimental set-up in future individual tests.

In yet an example the first-reference level is determined by determining the concentration of human C1 IA in at least one sample in both a healthy control population and a population with known cancer, thereby determining the first-reference level which identifies the cancer population with a predetermined specificity or a predetermined sensitivity. Likewise the second-reference level may be determined by determining the total concentration of human CRP in at least one sample in both a healthy control population and a population with known cancer, thereby determining the first-reference level which identifies the cancer population with a predetermined specificity or a predetermined sensitivity. Also, the third-reference level is determined by determining the total concentration of human C4 in at least one sample in both a healthy control population and a population with known cancer, thereby determining the first-reference level which identifies the cancer population with a predetermined specificity or a predetermined sensitivity.

In an example the first parameter may be the concentration of human C1 IA, the second parameter may be the concentration of human CRP and the third parameter may be the concentration of human C4.

In a further example the concentration of human C1 IA and/or the concentration of human CRP and/or the concentration of human C4 may be obtained any time before operation and/or medical treatment and/or irradiation.

In another example the concentration of human C1 IA and/or the concentration of human CRP and/or the concentration of native human C4 may be obtained any time after an operation and/or medical treatment and/or irradiation, such as 2 weeks, 1 month, 1.5 month, 2 months, 3 month, 4 months, 5 month, 6 months, 7 month, 8 months post operation and/or medical treatment and/or irradiation.

The levels of human C1 IA, human CRP and/or human C4 may be measured by conventional analytical methods, such as immunological methods known to the art.

Measurements of biological markers such as human C1 IA, human CRP and/or human C4 can be combined with measurements of other molecules at gene, RNA, or protein level in accordance with the teachings herein.

As stated above, determining the concentrations of biological markers such as human C1 IA, human CRP and/or human C4 may be made at the protein or nucleic acid levels. Ligands to human C1 IA, human CRP and/or C4 are particularly useful in detecting and/or quantitating these molecules.

Antibodies that bind to human C1 IA, human CRP and/or human C4 are particularly useful. Techniques for the assays contemplated herein are known in the art and include, for example, sandwich assays, xMAP multiplexing, Luminex, ELISA and ELISpot. Reference to antibodies includes parts of antibodies, "mammalianized" (e.g. humanized) antibodies, polyclonal, recombinant or synthetic antibodies and hybrid and single chain antibodies.

Thus, it may be preferred that the concentration of human C1 IA, human CRP and/or human C4 is measured by means of an immunoassay. The immunoassay may be ELISA and the ELISA result may be obtained using high sensitivity CRP testing (HSCRP) testing.

In an example the ELISA applies one or more antibodies that bind to human C1 IA, one or more antibodies that bind to human CPR and/or one or more antibodies that bind to human C4.

Both polyclonal and monoclonal antibodies may be obtained by immunization with human C1 IA, human CRP and/or human C4 or antigenic fragments thereof and either type may be utilizable for immunoassays. The methods of obtaining these types of sera are well known in the art.

Polyclonal sera may be less preferred but are relatively easily prepared by injection of a suitable laboratory animal with an effective amount of e.g. human C1 IA, human CRP and/or human C4 or antigenic part thereof, collecting serum or plasma from the animal and isolating specific sera by any of the known immuno-adsorbent techniques. Although antibodies produced by this method are utilizable in virtually any type of immunoassay, they are generally less favoured because of the potential heterogeneity of the product.

The use of monoclonal antibodies in an immunoassay may be particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Detection can also be obtained by either direct measure of the human C1 IA, human CRP or human C4 using specific antibody in a competitive fluorescent polarization immunoassay (CFIPA) or by detection of homodimerization of interferon-gamma by dimerization induced fluorescence polarization (DIFP).
In an embodiment the human C1 IA may comprise or consist of the amino acid sequence set forth in SEQ ID NO: 1.
In an embodiment the human CRP may comprise or consist of the amino acid sequence set forth in SEQ ID NO: 2.
In an embodiment the human C4 may comprise or consists of the amino acid sequence set forth in SEQ ID NO: 3.

The antibodies (i.e. the one or more antibodies that bind to human C1 IA, the one or more antibodies that bind to human CPR and/or the one or more antibodies that bind to human C4) may be selected from the group consisting of polyclonal antibody, a monoclonal antibody, a synthetic antibody, a recombinant antibody, a chimeric antibody, a heterochimeric antibody, or a humanized antibody and oligoclonal antibody.

In a preferred embodiment the antibody may be produced by recombinant methods or by a hybridoma method; such methods will be known to the person skilled in the art.

In an example the cancer is selected from the group consisting of malignant carcinoma, squamous carcinomas (oesophageal cancer, larynx cancer, bronchial carcinomas rectal carcinomas, pancreas carcinoma), adenocarcinomas, such as colon carcinomas of various types and sub types, pancreas carcinomas, ductal pancreas carcinoma, acinar pancreas carcinoma all glandular epithelial structures in which malignant adenocarcinomas such as breast carcinomas, bronchial carcinomas, ranging from lung alveolar carcinomas, breast carcinomas, e.g., ductal, lobular, etc., hepatocellular carcinomas and kidney carcinomas bladder carcinomas, malignant brain tumour including glioblastomas and oligodendroglioma and certain grades of astrocytomas (grade III).

In another aspect the present disclosure relates to a method of monitoring cancer in an individual, said method comprising the steps of
a) successively withdrawing samples from an individual with cancer over a period of time,
b) incubating a sample obtained in step a) with one or more antibodies that bind to human C1 IA (anti-C1 IA) and determining the human C1 IA level in said sample,
c) incubating a sample obtained in step a) with one or more antibodies that bind to human CRP (anti-CRP) and determining the human CRP level in said sample,
d) comparing the determined C1 IA of each of the samples in b)
e) comparing the determined CRP of each of the samples in c) and thereby determining:
   a. whether the level of C1 IA and the level of CRP is higher than the level of human C1 IA and the level of CRP in the immediately preceding mixture, which is indicative of progress of cancer, or
   b. whether the level of C1 IA and the level of CRP is essentially equal to the level of C1 IA and the level of CRP in the immediately preceding mixture, which is indicative of steady stage of cancer, or
   c. whether the level of C1 IA and the level of CRP s lower than the level of C1 IA and the level of CRP in the immediately preceding mixture, which is indicative of recovery of cancer.

In a further aspect the disclosure relates to a method of monitoring cancer in an individual, said method comprising the steps of
a) successively withdrawing samples from an individual with cancer over a period of time,
b) incubating a sample obtained in step a) with one or more antibodies that bind to human C1 IA (anti-C1 IA) and determining the human C1 IA level in said sample,
c) incubating a sample obtained in step a) with one or more antibodies that bind to human CRP (anti-CRP) and determining the human CRP level in said sample,
d) incubating a sample obtained in step a) with one or more antibodies that bind to human C4 (anti-C4) and determining the human C4 level in said sample,
e) comparing the determined C1 IA of each of the samples in b)
f) comparing the determined CRP of each of the samples in c)
g) comparing the determined C4 of each of the samples in d) and thereby determining:
   a. whether the level of C1 IA and the level of CRP is higher than the level of C1 IA and the level of CRP in the immediately preceding mixture and whether the levels of C4 is lower than the level of C4 in the immediately preceding mixture, which is indicative of progress of cancer, or
   b. whether the level of C1 IA and the level of CRP is essentially equal to the level of C1 IA and the level of CRP in the immediately preceding mixture and whether the levels of C4 is essentially equal to the level of C4 in the immediately preceding mixture, which is indicative of steady stage of cancer, or
   c. whether the levels of C1 IA and CRP is lower than the level of C1 IA and the level of CRP in the immediately preceding mixture and whether the levels of C4 is higher than the level of C4 in the immediately preceding mixture, which is indicative of recovery of cancer.

### Specificity and sensitivity

The sensitivity of any given diagnostic test define the proportion of individuals with a positive response who are correctly identified or diagnosed by the test, e.g. the sensitivity is 100%, if all individuals with a given condition have a positive test. The specificity of a given screening test reflects the proportion of individuals without the condition who are correctly identified or diagnosed by the test, e.g. 100 % specificity is, if all individuals without the condition have a negative test result.

Sensitivity is defined as the proportion of individuals with a given condition (e.g. cancer), who are correctly identified by the described methods of the invention (e.g. has a positive test-result).

Specificity herein is defined as the proportion of individuals without the condition (e.g. cancer), who are correctly identified by the described methods of the invention (e.g. has a negative test result)

### Reference levels

As will be generally understood by those of skill in the art, methods for screening/monitoring/determining cancer are processes of decision making by comparison. For any decision-making process, reference-values based on subjects having the disease and/or subjects not having the disease, infection, or condition of interest are needed.

The reference levels for each of the proteins (C1 IA, CRP and/or C4) can be based on several criteria including the number of subjects who would go on for further invasive diagnostic testing, the average risk of having and/or developing e.g. cancer to all the subjects who go on for further diagnostic testing, a decision that any subject whose patient specific risk is greater than a certain risk level should go on for further invasive diagnostic testing or other criteria known to those skilled in the art.

The reference levels can be adjusted based on several criteria such as but not restricted to certain group of individuals tested. E.g. the reference levels could be set lower in individuals with known cancer, reference levels may be higher in groups of otherwise healthy individuals with low risk of developing cancer.

Disclosed is a method for determining if a subject is likely to have cancer, which comprises:
(a) obtaining from the subject a sample, and
(b) quantitatively determining the concentration of human C1 IA and the concentration of human CRP present in the sample, the presence of the human C1 IA polypeptide present in the sample at a concentration higher than the selected reference level and the presence of the human CRP present in the sample at a concentration higher than the selected reference level, indicating the that the subject is likely to have cancer.

The first, second and third reference levels which has been determined by measuring the parameter or parameters in both a healthy control population and a population with known cancer thereby determining the reference levels which identifies the cancer population with either a predetermined specificity or a predetermined sensitivity based on an analysis of the relation between the parameter values and the known clinical data of the healthy control population and the cancer patient population, such as it is apparent from the detailed discussion in the examples herein. The reference levels determined in this manner is valid for the same experimental setup in future individual tests.

In the specific experimental setups described herein, the level threshold of human C1 IA useful as a first-reference level was found to be 35 mg/100 ml sample. Normal serum concentrations of human C1 IA are in the range from 15-35 mg/ml sample whereas normal serum concentrations of CRP are 00 mg/100ml sample.

In an example the first-reference level may be 35 mg human C1 IA/100 ml sample. In another example the second-reference level may be 00 mg human CRP/100 ml sample. In a further example third-reference level is 45 mg human C4/100 ml sample.

Clearly and as will be known to the skilled person the first, second and third-reference levels must be determined individually for specific populations.

Thus, based on the above, if a patient has a concentration of human C1 IA above the first-reference level and concentration of CRP above the second-reference level the patient is likely to have cancer.

The multivariate DISCRIMINANT analysis and other risk assessments can be performed on the commercially available computer program statistical package Statistical Analysis System (manufactured and sold by SAS Institute Inc.) or by other methods of multivariate statistical analysis or other statistical software packages or screening software known to those skilled in the art.

The method can equally well be used for monitoring the response to treatment and the progress of the cancer as rising human C1 IA concentrations in combination with rising human CRP C4 values may suggest that a patient could have a negative development of the cancer. In such cases the discriminating value is set specifically for each individual.

The method may be used both for an individual and for an entire population, but more appropriately to a population already identified as having an increased risk of developing cancer, e.g. individuals with a genetic disposition, individuals who have been exposed to carcinogenic substances, or individuals with cancer-predisposing non-malignant diseases.

When an individual has been identified as having high human C1 IA concentrations in combination with high CRP concentrations in his or her excreta, the individual should be referred for further examination.

### Receiver-operating characteristics

Accuracy of a diagnostic test is best described by its receiver-operating characteristics (ROC) (see especially Zweig et al. 1993. The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease, latent or recent infection versus no infection, or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results]. This has also been referred to as positivity in the presence of a disease or condition. It is calculated solely from the affected subgroup. On the x axis is the false-positive fraction, or 1 - specificity [defined as (number of false-positive results) / (number of true-negative + number of false-positive results)]. It is an index of specificity and is calculated entirely from the unaffected subgroup.

Because the true-and false-positive fractions are calculated entirely separately, by using the test results from two different subgroups, the ROC plot is independent of the prevalence of disease in the sample. Each point on the ROC plot represents a sensitivity/- specificity pair corresponding to a particular decision threshold. A test with perfect discrimination (no overlap in the two distributions of results) has an ROC plot that passes through the upper left corner, where the true- positive fraction is 1.0, or 100% (perfect sensitivity), and the false- positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Thus, it is an object to provide a method for diagnosing cancer in an individual, the method comprising:
a) determining the concentration of human C1 IA and the concentration of human CRP in a sample of said individual,
b) constructing a percentile plot of the human C1 IA and the concentration of human CPR concentrations obtained from a healthy population
c) constructing a ROC (receiver operating characteristics) curve based on the human C1 IA and human CRP concentrations determined in the healthy population and on the human C1 IA and human concentrations determined in a population with known cancer
d) selecting a desired specificity
e) determining from the ROC curve the sensitivity corresponding to the desired specificity
f) determining from the percentile plot the human C1 IA and human CRP concentrations corresponding to the determined sensitivity; and
g) predicting the individual to have cancer, if the concentration of human C1 IA in the sample is equal to or higher than said human C1 IA concentration corresponding to the determined specificity and if the concentration of human CRP in the sample is higher than said human CRP concentration corresponding to the determined specificity, and
h) predicting the individual as unlikely or not to have cancer if the concentration of human C1 IA in the sample is lower than said human C1 IA concentrationI corresponding to the determined specificity and if the concentration of human CRP in the sample is equal to or above than said human CRP concentration corresponding to the determined specificity.

The specificity of the method may be from 70% to 100%, more preferably 80% to 100%, more preferably 90% to 100%. Thus in one example the specificity is 80%, 81%, 82%, 83%, 84%, 85%, 86%, 87%, 88%, 89%, 90%, 91%, 92%, 93%, 94%, 95%, 96%, 97%, 98%, 99% or 100%.

The sensitivity of the method may be from 70% to 100%, more preferably 80% to 100%, more preferably 90% to 100%. Thus in one example the sensitivity is 80%, 81%, 82%, 83%, 84%, 85%, 86%, 87%, 88%, 89%, 90%, 91%, 92%, 93%, 94%, 95%, 96%, 97%, 98%, 99% or 100%.

The concentration of human C1 IA and human CRP may be compared to a set of reference data or a reference value such as the reference levels to determine whether the subject is at an increased risk or likelihood of e.g. cancer.

Alternatively reference levels can be determined as the mean, median or geometric mean of the negative control group ((e.g. healthy population, healthy individual, population with known cancer or individual with known cancer) +/- one or more standard deviations or a value derived from the standard deviation)

### Prognosis

In one example human C1 IA, human CRP and/or human C4 may be used for predicting the prognosis of subjects diagnosed with cancer. When used in patient prognosis the method may help to predict the course and probable outcome of the cancer, thus assisting the skilled artisan in selecting the appropriate treatment method and predict the effect of a certain treatment for the condition.

### Sample

It may be contemplated that the least one sample is selected from the group consisting of blood, serum, saliva, spinal fluid, cerebro spinal fluid, pleura fluid, ascites fluid, urine, tissue samples, tissue cells and combinations thereof.

In a preferred example the sample is derived from blood. Generally, blood is maintained in the presence of an anticoagulant (preferably heparin, alternatively e.g. citrate or EDTA). The anticoagulant is present in the blood collection tube when blood is added. The use of blood collection tubes is preferably but not necessarily compatible with standard automated laboratory systems and these are amenable to analysis in large-scale and random access sampling. Blood collection tubes also minimize handling costs and reduce laboratory exposure to whole blood and plasma and, hence, reduce the risk of laboratory personnel from contracting a pathogenic agent such as but not limited to human immunodeficiency virus.

Alliquots of whole blood may be in volumes ranging from 10µL-4000 µl, such as but not limited to 50µL, 100 µl, 200 µl, 300 µl, 400 µl, 500 µl, 600 µl, 700 µl, 800 µl, 900µl, 1000 µl, 1100 µl, 1200 µl, 1300 µl, 1400 µl, 1500 µl, 1600 µl, 1700 µl, 1800 µl, 1900µl, 2000µl, 2100 µl, 2200 µl, 2300 µl, 2400 µl, 2500 µl, 2600 µl, 2700 µl, 2800 µl, 2900µl or 3000µl.

### Antibodies

In one aspect, the present disclosure relates to one or more antibodies which binds to human C1-IA. In a further aspect, the present disclosure relates to one or more antibodies which binds to human CRP. In one aspect, the present disclosure relates to one or more antibodies which binds to human C4.

The one or more antibodies that bind to human C1 IA (anti-C1 IA), one or more antibodies that bind to human CPR (anti-CRP) and/or one or more antibodies that bind to human C4 (anti-C4) may clearly be applied in all aspects of the present invention - i.e. they may be applied in the method for diagnosis/screening and/or monitoring cancer in an individual, in the kit and the composition and pharmaceutical compositions of the present disclosure.

In an example the human C1 IA may comprise or consist of the amino acid sequence set forth in SEQ ID NO: 1.

In an example the human CRP may comprise or consist of the amino acid sequence set forth in SEQ ID NO: 2.

In an example the human C4 may comprises or consists of the amino acid sequence set forth in SEQ ID NO: 3.

In one example, C1 IA is human C1 IA.

In one example, CRP is human CRP.

In one example, C4 is human C4.

The antibodies (i.e. the one or more antibodies that bind to human C1 IA, the one or more antibodies that bind to human CPR and/or the one or more antibodies that bind to human C4) may be selected from the group consisting of of polyclonal antibody, a monoclonal antibody, a synthetic antibody, a recombinant antibody, a chimeric antibody, a heterochimeric antibody, or a humanized antibody and an oligoclonal antibody.

In a preferred example the antibody may be produced by recombinant methods or by a hybridoma method; such methods will be known to the person skilled in the art.

In a preferred example the one or more anti-C1 IA may be IgG anti-C1 IA and/or IgM anti-C1 IA. The one or more IgG anti-C1 IA may be selected from the group consisting of IgG1 anti-C1 IA, IgG2 anti-C1 IA, IgG3 anti-C1 IA, IgG4 anti-C1 IA and mixtures thereof.

In a preferred example the one or more anti-CRP may be IgG anti-CRP or IgM anti-CRP. The one or more IgG anti-CRP may be selected from the group consisting of IgG1 anti-CRP, IgG2 anti-CRP, IgG3 anti-CRP, IgG4 anti-CRP and mixtures thereof.

In a preferred example the one or more anti-C4 may be IgG anti-C4 and/or IgM anti-C4. The one or more IgG anti-C4 may selected from the group consisting of IgG1 anti-C4, IgG2 anti-C4, IgG3 anti-C4, IgG4 anti-C4 and mixtures thereof.

The antibodies for use in the present invention can be produced by several methods.

One method for producing such antibodies comprises transfecting a mammalian host cell with one or more vectors comprising relevant sequences of the antibody which should be produced, culturing the host cell and recovering and purifying the antibody molecule.

Another method for producing such antibodies comprises construction of hybridoma cells that produce the chimeric or humanized monoclonal antibodies.

Human recombinant antibodies may be produced by using human IgG produced in Human Embryonic Kidney cells or any placenta derived or amnion derived cells from humans. The antigenic determinants can in the case of C1 inhibitor be obtained from faxed B lymphocytes from a Quincke edema patient who has the type of Quincke (type 1), where the patient has developed anti C1 inhibitor as the reason for the patients C1 inhibitor deficiency. The antigenic determinants are isolated from the patient's B lymphocyte that are producing antigenic deterimants against C1 inhibitor (which may be obtained from a university hospital that has patients with Quincke's edema e.g., Lund University, Sweden or Washington University Medical School in St. Louis Missouri). Other types of antigenic determinants against CRP may be identified in collaboration with Washington University Medical School, St. Louis, Missouri.

In one embodiment, the antibody comprises a heavy chain of selected from the group consisting of IgG1, IgG2, IgG3, and IgG4.

In one embodiment, the antibody is a single-chain antibody.

In further aspect, the antibody may be a multispecific antibody comprising at least a first binding region of an antibody according to any aspect or embodiment herein described, and a second binding region which binds a different target or epitope than the first binding region. The term "multispecific antibody" as used herein, refers to antibodies wherein the binding regions two to at least two, such as at least three, different antigens or at least two, such as at least three, different epitopes on the same antigen.

In one embodiment, the antibody may be a bispecific antibody comprising a first binding region of an antibody according to any aspect or embodiments herein described, and a second binding region which binds a different target or epitope than the first binding region. The term "bispecific" as used herein, refers to binding molecules, such as antibodies wherein the binding regions of the binding molecule bind to two different antigens or two different epitopes on the same antigen. The term "bispecific antibody" refers to an antibody having specificities for at least two different, typically non-overlapping, epitopes. Such epitopes may be on the same or different targets. If the epitopes are on different targets, such targets may be on the same cell or different cells, cell types or structures, such as extracellular tissue. The term "different target" as used herein, refers to another protein, molecule or the like than C1 IA, CRP and/or C4 or an C1 IA, CRP and/or C4 fragment.

The antibodies (i.e. the anti-C1 IA, anti-CRP and/or anti-C4) may be conjugated to a therapeutic or diagnostic moiety, such as a cytotoxic agent, a chemotherapeutic drug, a cytokine, an immunosuppressant, antibiotic, or a radioisotope. Such conjugates are referred to herein as "immunoconjugates". Immunoconjugates which include one or more cytotoxins are referred to as "immunotoxins". Antibodies conjugated to a cytotoxic agent, drug or the like are also known as antibody-drug conjugates (ADC). In one embodiment, the therapeutic moiety is a cytotoxic agent. A cytotoxin or cytotoxic agent includes any agent that is detrimental to (e.g., kills) cells. Suitable cytotoxic agents for forming immunoconjugates of the present invention include taxol, tubulysins, duostatins, cytochalasin B, gramicidin D, ethidium bromide, emetine, mitomycin, etoposide, tenoposide, vincristine, vinblastine, colchicin, doxorubicin, daunorubicin, dihydroxy anthracin dione, maytansine or an analog or derivative thereof, mitoxantrone, mithramycin, actinomycin D, 1-dehydrotestosterone, glucocorticoids, procaine, tetracaine, lidocaine, propranolol, and puromycin; calicheamicin or analogs or derivatives thereof; antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin; as well as duocarmycin A, duocarmycin SA, CC-1065 (a.k.a. rachelmycin), or analogs or derivatives of CC-1065), dolastatin, auristatin, pyrrolo[2,1-c][1,4] benzodiazepins (PDBs), indolinobenzodiazepine (IGNs) or analogues thereof, antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)), anti-mitotic agents (e.g., tubulin-targeting agents), such as diphtheria toxin and related molecules (such as diphtheria A chain and active fragments thereof and hybrid molecules); ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Other suitable conjugated molecules include antimicrobial/lytic peptides such as CLIP, Magainin 2, mellitin, Cecropin, and P18; ribonuclease (RNase), DNase I, Staphylococcal enterotoxin-A, pokeweed antiviral protein, diphtherin toxin, and Pseudomonas endotoxin. Therapeutic agents that may be administered in combination with anti-C1 IA antibody, anti-CRP antibody and/or anti-C4 antibody or antibody-drug conjugates of the present invention as described elsewhere herein, such as, e.g., anti-cancer cytokines or chemokines, are also candidates for therapeutic moieties useful for conjugation to an antibody disclosed in the present invention. The term "cytotoxic agent" as used herein, refers to any agent that is detrimental to cells.

In another alternative embodiment, the anti-C1 IA antibody, anti-CRP antibody and/or anti-C4 antibody may comprise conjugated nucleic acid or nucleic acid-associated molecule. In one such embodiment, the conjugated nucleic acid is a cytotoxic ribonuclease, an antisense nucleic acid, an inhibitory RNA molecule (e.g., a siRNA molecule) or an immunostimulatory nucleic acid (e.g., an immunostimulatory CpG motif-containing DNA molecule). In another alternative embodiment, anti-C1 IA antibody, anti-CRP antibody and/or anti-C4 antibody may be conjugated to an aptamer or a ribozyme or a functional peptide analog or derivate thereof. In another alternative embodiment, anti-C1 IA antibody, anti-CRP antibody and/or anti-C4 antibody drug conjugates comprising one or more radiolabeled amino acids are provided. A radiolabeled anti-C1 IA antibody, anti-CRP antibody and/or anti-C4 antibody may be used for both diagnostic and therapeutic purposes (conjugation to radiolabeled molecules is another possible feature). Non-limiting examples of labels for polypeptides include ³H, ¹⁴C, ¹⁵N, 355, ⁹⁰Y, ⁹⁹Tc, and ¹²⁵1, ¹³¹1 and ¹⁸⁶Re. In one embodiment, the anti-C1 IA antibody, anti-CRP antibody and/or anti-C4 antibody may be conjugated to a radioisotope or to a radioisotope-containing chelate. For example, the anti-C1 IA antibody, anti-CRP antibody and/or anti-C4 antibody can be conjugated to a chelator linker, e.g. DOTA, DTPA or tiuxetan, which allows for the antibody to be complexed with a radioisotope. The antibody may also or alternatively comprise or be conjugated to one or more radiolabeled amino acids or other radiolabeled molecules. A radiolabeled anti-C1 IA antibody, anti-CRP antibody and/or anti-C4 antibody may be used for both diagnostic and therapeutic purposes. Non-limiting examples of radioisotopes include ³H, ¹⁴C, ¹⁵N, ³⁵S, ⁹⁰Y, ⁹⁹Tc, ¹²⁵1, ¹¹¹In, ¹³¹1, ¹⁸⁶Re, ²¹³Bs, ²²⁵Ac and ²²⁷Th.

### Kits

It is an aspect of the present disclosure to provide a kit or device for performing the method, having a simple and inexpensive design, being quick and easy to use and not requiring the assistance of specialists or the use of specialised equipment.

In yet another aspect the disclosure relates to a kit comprising (i) one or more antibodies that bind to human C1 IA (anti-C1 IA), one or more antibodies that bind to human CRP (anti-CRP) and/or one or more antibodies that bind to human C4 (anti-C4) and (ii) instructions for use of said kit.

In a further aspect the disclosure relates to a kit for performing the method disclosed above, said kit comprising (i) one or more antibodies that bind to human C1 IA (anti-C1 IA), one or more antibodies that bind to human CRP (anti-CRP) and/or one or more antibodies that bind to human C4 (anti-C4) and (ii) instructions for use of said kit.

In a preferred example the one or more anti-C1 IA may be IgG anti-C1 IA antibody and/or IgM anti-C1 IA antibody. The one or more IgG anti-C1 IA antibody may be selected from the group consisting of IgG1 anti-C1 IA antibody, IgG2 anti-C1 IA antibody, IgG3 anti-C1 IA antibody, IgG4 anti-C1 IA antibody and mixtures thereof.

In a preferred example the one or more anti-CRP may be IgG anti-CRP antibody or IgM anti-CRP antibody. The one or more IgG anti-CRP antibody may be selected from the group consisting of IgG1 anti-CRP antibody, IgG2 anti-CRP antibody, IgG3 anti-CRP antibody, IgG4 anti-CRP antibody and mixtures thereof.

In a preferred example the one or more anti-C4 may be IgG anti-C4 antibody and/or IgM anti-C4 antibody. The one or more IgG anti-C4 antibody may selected from the group consisting of IgG1 anti-C4 antibody, IgG2 anti-C4 antibody, IgG3 anti-C4 antibody, IgG4 anti-C4 antibody and mixtures thereof.

It may be contemplated that the concentration of each of the proteins (i.e. C1 IA, CPR and/or C4) is determined separately.

The kit may be selected from the group consisting of ELISA, immunoturbiditymetry, nephelometry, immunodiffusion, agglutination kit, Western Blot and SDS page. The principles of immunodiffusion are disclosed in Mancini et al. 1964.

The one or more anti-C1 IA, anti-CPR and/or anti-C4 may be immobilized on a solid support selected from the group consisting of membranes, plastic, glas, metal, porcelain and combinations thereof. The agglutination kit may be the Eldon Card™.

In terms of immunodiffusion the kit may comprises at least two gel layers, wherein a first gel layer comprises one or more anti-C1 IA and wherein a second gel layer comprises one or more anti-CPR. In another embodiment the kit may comprise at least three gel layers, wherein a first gel layer comprises one or more anti-Cl IA, wherein a second gel layer comprises one or more anti-CPR and wherein the third layer comprises one or more anti-C4. Clearly the gel order may be reversed.

In another example the one or more anti-C1 IA, anti-CPR and/or anti-C4 may be conjugated to a detectable marker. The detectable marker may be a fluorescent marker or a fluorescein derivative or selected from the group consisting of chromogens, catalysts such as enzymes, a secondary antibody, fluorescent compounds, chemiluminescent compounds, radioactive labels, metals, magnetic particles, dye particles, organic polymer latex particles, liposomes or other vesicles containing signal producing substances and the like.

The detectable signal is capable of being observed by any kind of visual or instrumental means known to the person skilled in the art however, such means may be selected from the group consisting of magno(magne)tometer, spectrophotometer, ELISA-reader and/or CCD camera.

Further disclosed is a kit for measurement of the concentration of human C1 IA, human CRP and/or human C4 in at least one sample. Such kit may consist of a dipstick for determining the concentration of human C1 IA, human CRP and/or human C4 in at least one sample however, other options may be but is not limited to Activity Assay (such as zymography), immunologic assays or a Colour Reaction kit.

The present disclosure further contemplates a kit for performing the method of the present invention. The kit is conveniently in compartmental form with one or more compartments adapted to receive a sample from a subject such as whole blood, serum, purified cells, biopsies or other applicable sample material. That compartment or another compartment may also be adapted to contain heparin where the sample is whole blood.

Generally, the kit is in a form which is packaged for sale with a set of instructions. The instructions would generally be in the form of a method - i.e. for diagnosing cancer in a subject.

In one example the kit comprises antibodies that bind to human C1 IA, human CRP and/or human C4 an immune-assay or specific binding fragments of said antibodies for use as a diagnostic reagent.

The contemplated kit may be in a multicomponent form wherein a first component comprises a multiplicity of blood collection tubes, a second component comprising an antibody-based detection means for human C1 IA, third component comprising an antibody-based detection means for human CRP, fourth component comprising a set of instructions and optionally a fifth component comprising an antibody-based detection means for human C4.

The assay may also be automated or semi-automated and the automated aspects may be controlled by computer software.

The assay may be automated or semi-automated for high throughput screening or for screening for a number of immune effecters from the one subject. The automation is conveniently controlled by computer software. The present disclosure contemplates a computer program product, therefore, for assessing the presence or absence or the level of human C1 IA, human CRP and/or human C4, said product comprises:
(1) code that receives, as input values, the identity of a reporter molecule associated with a labelled antibody or mRNA
(2) code that compares said input values with reference values to determine the level of reporter molecules and/or the identity of the molecule to which the reporter molecule is attached; and
(3) a computer readable medium that stores the codes.

Still another aspect extends to a computer for assessing the presence or absence or level of human C1 IA, human CRP and/or human C4, said computer comprises:
(1) a machine-readable data storage medium composing a data storage material encoded with machine-readable data, wherein said machine-readable data I comprise input values which identify a reporter molecule associated with a labelled antibody or mRNA;
(2) a working memory for storing instructions for processing said machine- readable data,
(3) a central-processing unit coupled to said working memory and to said machine readable data storage medium, for processing said machine readable data to compare said values to provide an assessment of the identity or level of reporter molecules or of molecules to which they are attached; and
(4) an output hardware coupled to said central processing unit, for receiving the results of the comparison.

### Composition and pharmaceutical composition and use thereof

In a further aspect the present disclosure relates to a composition comprising, as the active ingredient, one or more antibodies that bind to human C1 IA (anti-C1 IA), one or more antibodies that bind to human CRP (anti-CRP) and/or one or more antibodies that bind to human C4 (anti-C4).

In another aspect the disclosure relates to a pharmaceutical composition comprising, as the active ingredient, one or more antibodies that bind to human C1 IA (anti-C1 IA), one or more antibodies that bind to human CRP (anti-CRP) and/or one or more antibodies that bind to human C4 (anti-C4) and a pharmaceutically acceptable carrier, diluent and/or excipient.

In an embodiment the one or more anti-C1 IA antibody may be IgG anti-C1 IA antibody and/or IgM anti-C1 IA antibody. The one or more IgG anti-C1 IA antibody may be selected from the group consisting of IgG1 anti-C1 IA antibody, IgG2 anti-C1 IA antibody, IgG3 anti-C1 IA antibody, and IgG4 anti-C1 IA antibody In a preferred embodiment the anti-C1 IA antibody is IgG₁ and/or IgG₃ anti-C1 IA antibody, since these types of antibodies activate the complement system. Also it may be contemplated that the composition and pharmaceutical composition comprise a mix of different antibodies binding to C1 IA since such a mix may target slightly different parts of the C1 IA protein and thereby provide an improved effect. In a further embodiment the one or more anti-CRP antibody may be IgG anti-CRP antibody and/or IgM anti-CRP antibody. The one or more IgG anti-CRP antibody may be selected from the group consisting of IgG1 anti-CRP antibody, IgG2 anti-CRP antibody, IgG3 anti-CRP antibody, IgG4 anti-CRP antibody and mixtures thereof. In a preferred embodiment the anti-CRP antibody is IgG₁ or IgG₃ or e.g. IgM anti-CRP antibody, since these types of antibodies activate the complement system. Also it may be contemplated that the composition and pharmaceutical composition comprise a mix of different antibodies binding to CRP since such a mix may target slightly different parts of the CRP protein and thereby provide an improved effect.

In an embodiment the human C1 IA may comprise or consist of the amino acid sequence set forth in SEQ ID NO: 1.

In an embodiment the human CRP may comprise or consist of the amino acid sequence set forth in SEQ ID NO: 2.

In an embodiment the human C4 may comprise or consists of the amino acid sequence set forth in SEQ ID NO: 3.

The antibodies (i.e. the one or more antibodies that bind to human C1 IA, the one or more antibodies that bind to human CPR and/or the one or more antibodies that bind to human C4) may be selected from the group consisting of of polyclonal antibody, a monoclonal antibody, a synthetic antibody, a recombinant antibody, a chimeric antibody, a heterochimeric antibody, or a humanized antibody and an oligoclonal antibody.

In a preferred embodiment the antibody may be produced by recombinant methods or by a hybridoma method; such methods will be known to the person skilled in the art.

The compositions are optimized for parameters such as physiological tolerance and shelf-life.

An example relates to a pharmaceutical composition as described above, further comprising one or more additional therapeutic agents.

In another example are said one or more additional therapeutic agents selected from agents that induce apoptosis. The pharmaceutically acceptable carrier may be a buffer solution, which to a substantial degree maintain the chemical integrity of the one or more antibodies and is being physiologically acceptable for infusion into patients.

Thus, an aspect of the present disclosure relates to a pharmaceutical composition comprising a radioimmunoconjugate, and a pharmaceutically acceptable carrier, diluent and/or excipient.

Acceptable pharmaceutical carriers include but are not limited to non-toxic buffers, fillers, isotonic solutions, etc. More specifically, the pharmaceutical carrier can be but are not limited to normal saline (0.9 %), half-normal saline, Ringer's lactate, 5 % Dextrose, 3.3 % Dextrose/0.3 % Saline. The physiologically acceptable carrier can contain a radiolytic stabilizer, e.g., ascorbic acid, human serum albumin which protect the integrity of the radiopharmaceutical during storage and shipment.

In another aspect the disclosure relates to the pharmaceutical composition disclosed above for use as a medicament.

In a further aspect the invention relates to the pharmaceutical composition disclosed above for use in the treatment of cancer, inhibiting growth of cancer and/or inhibiting proliferation of cancer in an individual.

The composition may be administered intratumourrally applying e.g. an Ommaya catheter, intracerebrally, intraspinally, intrathecally and/or intravenously.

The composition may, for example, be administered to the individual every day, every second day, once a week, once every second week, once every third week, once a month, once every second month, once every third month, once every fourth month, once every fifth month or once every sixth month.

The efficient dosages and dosage regimens for anti-Cl IA antibody, anti-CRP antibody and/or anti-C4 antibody or ADC depend on the disease or condition to be treated and may be determined by the persons skilled in the art.

A physician having ordinary skill in the art may readily determine and prescribe the effective amount of the pharmaceutical composition required. In relation hereto, when referring to a pharmaceutical composition it is to be understood also to comprise a composition as such, or vice versa. For example, the physician could start doses of the anti-Cl IA antibody, anti-CRP antibody and/or anti-C4 antibody employed in the pharmaceutical composition at levels lower than that required in order to achieve the desired therapeutic effect and gradually increase the dosage until the desired effect is achieved. In general, a suitable dose of a pharmaceutical composition of the present invention will be that amount of the compound which is the lowest dose effective to produce a therapeutic effect according to a particular dosage regimen. Such an effective dose will generally depend upon the factors described above.

For example, an "effective amount" for therapeutic use may be measured by its ability to stabilize the progression of disease. The ability of a compound to inhibit cancer may, for example, be evaluated in an animal model system predictive of efficacy in human tumors. Alternatively, this property of a composition may be evaluated by examining the ability of the compound to inhibit cell growth or to induce cytotoxicity by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected.

An exemplary, non-limiting range for a therapeutically effective amount of an anti-C1 IA antibody or an anti-CRP antibody according to this invention is an estimated dosage of between 10-20 ml with an antibody content of 100-200 mg for a 70 kg person.

Administration may e.g. be intravenous, intramuscular, intra peritoneal, or subcutaneous, and for instance administered proximal to the site of the target. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation.

In one example, the efficacy-safety window is optimized by lowering specific toxicity such as for example by lowering the drug-antibody ratio (DAR) and/or mixing of anti-C1 IA antibody ADC, anti-CRP antibody ADC and/or anti-C4 antibody ADC with unlabeled anti-C1 IA antibody, anti-CRP antibody and/or anti-C4 antibody.

In one example, the efficacy of the treatment is monitored during the therapy, e.g. at predefined points in time. In one embodiment, the efficacy may be monitored by measuring the level of C1 IA, CRP and/or C4 in a sample containing tumor cells, by visualization of the disease area, or by other diagnostic methods described further herein, e.g. by performing one or more PET-CT scans, for example using a labeled anti-C1 IA antibody, anti-CRP antibody and/or anti-C4 antibody, fragment or mini-antibody derived from the anti-C1 IA antibody, anti-CRP antibody and/or anti-C4 antibody of the present invention.

If desired, an effective daily dose of a pharmaceutical composition may be administered as two, three, four, five, six or more sub-doses administered separately at appropriate intervals throughout the day, optionally, in unit dosage forms. In another embodiment, the anti-C1 IA antibody, anti-CRP antibody and/or anti-C4 antibody are administered by slow continuous infusion over a long period, such as more than 24 hours, in order to minimize any unwanted side effects.

While it is possible for a compound of the present invention to be administered alone, it is preferable to administer the compound as a pharmaceutical composition as described above.

An effective dose of an anti-C1 IA antibody, anti-CRP antibody and/or anti-C4 antibody, bispecific antibody or ADC of the invention may also be administered using a weekly, biweekly or triweekly dosing period. The dosing period may be restricted to, e.g., 8 weeks, 12 weeks or until clinical progression has been established.

For example, in one embodiment, the anti-C1 IA antibody, anti-CRP antibody and/or anti-C4 antibody, bispecific antibodies or ADC's may be administered by infusion in a weekly estimated dosage of between 10-20 ml with an antibody content of 100-200 mg for a 70 kg person. Such administration may be repeated, e.g., 1 to 8 times, such as 3 to 5 times. The administration may be performed by continuous infusion over a period of from 1 to 24 hours, such as of from 1 to 12 hours.

In another embodiment, the anti-C1 IA antibody, anti-CRP antibody and/or anti-C4 antibody, bispecific antibodies or ADC's may be administered by infusion every three weeks in an estimated dosage of between 10-20 ml with an antibody content of 100-200 mg for a 70 kg person. Such administration may be repeated, e.g., 1 to 8 times, such as 3 to 5 times. The administration may be performed by continuous infusion over a period of from 1 to 24 hours, such as of from 1 to 12 hours.

In one embodiment, an anti-C1 IA antibody ADC, anti-CRP antibody ADC and/or anti-C4 antibody ADC or bispecific antibodies may be administered as a single dose corresponding to an estimated dose of about 10 - 20 ml with a content of 200 - 400 mg of immunoglobulin for an average weight of 70 kg, for example every week or every third week for up to twelve times, up to eight times, or until clinical progression. The administration may be performed by continuous infusion over a period of from 1 to 24 hours, such as of from 1 to 12 hours. Such regimens may be repeated one or more times as necessary, for example, after 6 months or 12 months.

The dosage may be determined or adjusted by measuring the amount of compound of the present invention in the blood upon administration by for instance taking out a biological sample and using anti-idiotypic antibodies which target the antigen binding region of the anti-C1 IA antibody, anti-CRP antibody and/or anti-C4 antibody of the present invention.

In one embodiment, the anti-C1 IA antibody, anti-CRP antibody and/or anti-C4 antibody may be are administered as maintenance therapy, such as, e.g., once a week for a period of six months or more.

As non-limiting examples, treatment according to the present invention may be provided as a daily dosage of a compound of the present invention in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the present invention are dictated by and directly dependent on (a) the unique characteristics of the active compound and the particular therapeutic effect to be achieved, and (b) the limitations inherent in the art of compounding such an active compound for the treatment of sensitivity in individuals.

Administering (such as but not limiting to injection or infusing) anti-C1 IA to an individual suffering from cancer is likely to result in neutralizing of the inhibitory effect of C1 IA on the Clqrs complex thereby activating this complex to C1r and C1s which again will activate C4 to C4b. C4b will activate C2 to C2b and will initiate the classical pathway provided that this antibody is capable of activating the complement system. Therefore it may be preferred that the anti-C1 IA is IgG1 and/or IgG3 since these types of antibodies activate the complement system (see also Figure 37).

Administering (such as but not limiting to injection or infusing) anti-CRP to an individual suffering from cancer is likely to result in neutralizing the inhibition of H factor and then C3b will be released. C3b can then activate via C5b via c5 convertase resulting in activation of C6, C7, C8 and C9, wherein C9 will constitute multiple components of the C9. This will happen on the surface of the cell (see also Figure 37).

Administering (such as but not limiting to injection or infusing) anti-CRP to an individual suffering from cancer is likely to result in an accelation of the complement cascade reaction (see also Figure 37).

The one or more anti-C1 IA, one or more anti-CPR and/or one or more anti-C4 may be conjugated to a chemotherapeutic drug, isotope and/or monoclonal antibodies. The chemotherapeutic drug may be selected form the group consisting of chemotherapy drugs, alkylating agents and platinium drugs. The alkylating agents may be selected from the group consisting of nitrogen mustards, such as mechlorethamine (nitrogen mustard), chlorambucil, cyclophosphamide (Cytoxan®), ifosfamide, and melphalan, nitrosoureas such as streptozocin, carmustine (BCNU), and lomustine, alkyl sulfonates such as busulfan, triazines such as dacarbazine (DTIC) and temozolomide (Temodar®), ethylenimines such as thiotepa and altretamine (hexamethylmelamine). The platinum drug may be selected from the group consisting of cisplatin, carboplatin and oxalaplatin.

In an example the cancer may be selected from the group consisting of malignant carcinoma, squamous carcinomas (oesophageal cancer, larynx cancer, bronchial carcinomas rectal carcinomas, pancreas carcinoma); adenocarcinomas, such as colon carcinomas of various types and sub types, pancreas carcinomas, ductal pancreas carcinoma, acinar pancreas carcinoma all glandular epithelial structures in which malignant adenocarcinomas such as breast carcinomas, bronchial carcinomas, ranging from lung alveolar carcinomas, breast carcinomas, e.g., ductal, lobular, etc., hepatocellular carcinomas, kidney carcinomas bladder carcinomas, malignant brain tumors (e.g. glioblastomas or astrocytomas), astrocytoma, glioblastomas grade III-IV, gliomas, glioblastoma multiforme, oligodendrogliomas, ependymoma, and medulloblastoma. In a preferred embodiment the cancer is a malignant brain tumor.

A further embodiment of the present invention relates to a pharmaceutical composition of the present invention for treating cancer types expressing human C1-IA, human CRP and/or human C4. In a preferred embodiment the cancer types express human C1-IA and human CRP on their surface.

An aspect relates to the pharmaceutical composition of the present invention for use in depleting cells that express human C1-IA, human CRP and/or human C4 on their surface.

In another aspect the present disclosure relates to a method for inhibiting growth and/or proliferation of a tumor cell expressing C1-IA, CPR and/or C4, comprising administration of a therapeutically effective amount of the composition or the pharmaceutical composition disclosed above to an individual in need thereof.

Another aspect relates to the use of a pharmaceutical composition comprising anti-C1 IA, anti-CPR and/or anti-C4 for the manufacture of a medicament for the treatment of cancer. A further aspect relates to a method for treating a patient suffering from a cancer comprising administering to said patient an effective amount of a pharmaceutical composition comprising anti-C1 IA, anti-CPR and/or anti-C4. Yet an aspect relates to the use of anti-C1 IA, anti-CPR and/or anti-C4 in the treatment of cancer.

A further aspect relates to a diagnostic composition comprising one or more anti-C1 IA, one or more anti-CPR and/or one or more anti-C4.

Therapeutic use of a pharmaceutical solution may be for treatment against malignant cells expressing human C1-IA, human CRP an/or human C4, including but not limited to a cancers types selected from the group consisting of osteosarcoma, soft tissue sarcomas, breast cancer, lung cancer, head and neck cancer, melanoma, pancreas cancer, prostate cancer, leukemia and brain cancer.

The therapy could be based on immunotherapy, antibody drug conjugate, immunotoxin or radioimmunotherapy including but are not limited to, beta-particle-radiation or alpha-particle-radiation or a combination of these.

The therapy could be administered either as a monotherapy or in combination with other therapies, preferentially standard treatments. Such other therapies may be pretreatment, surgery, chemotherapy (including doxorubicin, vinblastin and gemcitabine), immunotherapy, photodynamic therapy, proteasome inhibitor (including bortezomib), histone deacetylase inhibitors (including vorinostat and suberoylanilide hydroxamic acid), vitamin D3 and vitamin D3 analogs, cell cycle checkpoint inhibitors (including UCN-01 and 2-(4-(4-Chlorophenoxy)phenyl)-1H-benzimidazole-5-carboxamide), hypoxic cell radiosensitizers (including metronidazole and misonidazole), apoptosis inducers (including withaferin A) radiosensitizers, radioimmunotherapy or a combination of two or more of these.

By administered is meant intravenous infusion or intravenous injection. More specifically, the pharmaceutical composition of the present invention can be administered directly in a vein by a peripheral cannula connected to a drip chamber that prevents air embolism and allows an estimate of flow rate into the patient.

In one embodiment the pharmaceutical composition of the present invention can be administered in a repeated fashion.

In another embodiment of the present invention the pharmaceutical composition of the present invention could be administered in a repeated fashion but with different modalities, e.g., beta-radioimmunotherapy could be followed by alpha-radioimmunotherapy or vice versa or cytotoxic drug conjugate followed by immunoconjugate etc.

An embodiment of the present invention relates to the use of the pharmaceutical composition of the present invention administered in combination with or in addition to other therapy. In an embodiment of the present invention the other therapies is selected from pretreatment, chemotherapy, monoclonal antibody therapy, surgery, radiotherapy, and/or photodynamic therapy. In another embodiment of the present invention the other therapies are bone marrow transplantation or stem cell transplantation and/or therapy.

In a particular aspect, anti-C1 IA antibody, anti-CRP antibody and/or anti-C4 antibody or ADC may be administered prophylactically in order to reduce the risk of developing cancer, delay the onset of an event in cancer progression or reduce the risk of recurrence when a cancer is in remission and/or a primary tumor has been surgically removed. In the latter case, the anti-C1 IA antibody, anti-CRP antibody and/or anti-C4 antibody could, for example, be administered in association with (i.e., before, during, or after) the surgery. Prophylactic administration may also be useful in patients wherein it is difficult to locate a tumor that is believed to be present due to other biological factors.

In one embodiment of the present invention are the uses and methods of treatment of the present invention performed in vitro or ex vivo.

A further embodiment of the present invention relates to intracavitary administration of one or more antibodies that bind to human C1-IA, one or more antibodies that bind to human CRP and/or one or more antibodies that bind to human C4, conjugates or compositions of the present invention.

Examples of such administrations are intraperitoneal, intrapleural, and intracranial administrations.

A further aspect of the present invention relates to a method of inhibiting the growth of a cancer cell, comprising; contacting the cancer cell with an effective amount of an antibody of the present invention, thereby inhibiting the growth of the cancer cell.

In one example of the present disclosure are the methods carried out in vitro.

The administration of the antibodies and pharmaceutical compositions of the present invention can be done through many different routes of administration including topical, oral, through the gastrointestinal tract, intradermal, subcutaneous, nasal, intravenous, intramuscular, enteral or parenteral.

The pharmaceutical compositions may be formulated with pharmaceutically acceptable carriers or diluents as well as any other known adjuvants and excipients in accordance with conventional techniques. A pharmaceutical composition used in the present invention may also include diluents, fillers, salts, buffers, detergents (e. g., a nonionic detergent, such as Tween-20 or Tween-80), stabilizers (e.g., sugars or protein-free amino acids), preservatives, tissue fixatives, solubilizers, and/or other materials suitable for inclusion in a pharmaceutical composition. The actual dosage levels of the active ingredients in the pharmaceutical compositions used in the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical arts. history of the patient being treated, and like factors well known in the medical arts. The pharmaceutical composition may be administered by any suitable route and mode. Suitable routes of administering a compound used in the present invention in vivo and in vitro are well known in the art and may be selected by those of ordinary skill in the art. In one embodiment, the pharmaceutical composition used in the present invention is administered parenterally. The terms "parenteral administration" and "administered parenterally" as used herein refers to modes of administration other than enteral and topical administration, usually by injection, and include epidermal, intravenous, intramuscular, intra-arterial, intrathecal, intracapsular, intra-orbital, intracardiac, intradermal, intraperitoneal, intratendinous, transtracheal, subcutaneous, subcuticular, intra-articular, subcapsular, subarachnoid, intraspinal, intracranial, intrathoracic, epidural and intrasternal injection and infusion. In one embodiment, the pharmaceutical composition used in the present invention is administered by intravenous or subcutaneous injection or infusion. Pharmaceutically acceptable carriers include any and all suitable solvents, dispersion media, coatings, antibacterial and antifungal agents, isotonicity agents, antioxidants and absorption-delaying agents, and the like that are physiologically compatible with a compound of the present invention. Examples of suitable aqueous and non-aqueous carriers which may be employed in the pharmaceutical compositions of the present invention include water, saline, phosphate-buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts. Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions used in the present invention is contemplated. Proper fluidity may be maintained, for example, by the use of coating materials, such as lecithin, by the maintenance of the required particle size in the case of dispersions, and by the use of surfactants. Pharmaceutical compositions used in the present invention may also comprise pharmaceutically acceptable antioxidants for instance water-soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal-chelating agents, such as citric acid, ethylenediamine tetraacetic acid (EDTA), sorbitol, tartaric acid, phosphoric acid, and the like. Pharmaceutical compositions used in the present invention may also comprise isotonicity agents, such as sugars, polyalcohols, such as mannitol, sorbitol, glycerol or sodium chloride in the compositions. The pharmaceutical compositions used in the present invention may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The compounds used in the present invention may be prepared with carriers that will protect the compound against rapid release, such as a controlled release formulation, including implants, transdermal patches, and micro-encapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, poly-orthoesters, and polylactic acid alone or with a wax, or other materials well known in the art. Methods for the preparation of such formulations are generally known to those skilled in the art. In one embodiment, the compounds used in the present invention may be formulated to ensure proper distribution in vivo. Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions used in the present invention is contemplated. Other active or therapeutic compounds may also be incorporated into the compositions.

Pharmaceutical compositions for injection must typically be sterile and stable under the conditions of manufacture and storage. The composition may be formulated as a solution, micro-emulsion, liposome, or other ordered structure suitable to high drug concentration. The carrier may be an aqueous or a non-aqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating such as lecithin, by the maintenance of the required particle size in the case of dispersion and by the use of surfactants. In many cases, it will be preferable to include isotonic agents, for example, sugars, polyalcohols such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the active compound in the required amount in an appropriate solvent with one or a combination of ingredients e.g. as enumerated above, as required, followed by sterilization microfiltration. Generally, dispersions are prepared by incorporating the active compound into a sterile vehicle that contains a basic dispersion medium and the required other ingredients e.g. from those enumerated above. In the case of sterile powders for the preparation of sterile injectable solutions, examples of methods of preparation are vacuum-drying and freeze-drying (lyophilization) that yield a powder of the active ingredient plus any additional desired ingredient from a previously sterile-filtered solution thereof. Sterile injectable solutions may be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, examples of methods of preparation are vacuum-drying and freeze-drying (lyophilization) that yield a powder of the active ingredient plus any additional desired ingredient from a previously sterile-filtered solution thereof. The pharmaceutical composition used in the present invention may contain one or more antibodies, bispecific antibodies or ADC's of the present invention, a combination of an antibody, a bispecific antibody or ADC according to the invention with another therapeutic compound, or a combination of compounds used in the present invention.

It should be noted that embodiments and features described in the context of one of the aspects of the present invention also apply to the other aspects of the invention.

### Items

C1 IA, and other cancer cell related blocking immunogens for cancer diagnostic, monitoring and cancer treatment

### Abstract

A combination of C1 inactivator (C1 IA) and added effect on cancer, by a novel combination of C Reactive Protein, called CRP, having been identified recently that those two proteins, C1 inactivator and C Reactive Protein (CRP) together as a novel realized combination may be used both as a diagnostic test system based on demonstrating the presence of CRP, antibodies and antisera with specificity against C1 IA and CRP antibodies, and a human cancer therapy of certain types of cancer such as carcinomas and malignant brain tumors using human antisera or antibodies and preferably monoclonal or recombinant human antisera combining C1 IA and CRP in the cancer treatment as well as mixed together as anti human C1 IA and CRP with specificity against those proteins. CRP may be identified as a protein with different locations in a Grabar immune electrophoreses., so that CRP can be located in or around the alpha protein or in the gamma protein region depending on the solvens and other possibilities to change the location in a Grabar immunoelectrophoresis, and is therefore not considered a new CRP due to a different location in this test method, but most probably changes in the isoelectric point of the protein.

According to this invention it is surprising that CRP could be harvested together with C1 IA from carcinoma cultures and from pleural and ascites fluid because of metastases from patients with breast cancer and with ovarian carcinomas. But due to the fact that carcinomas often have been described to contain areas with dead or dying carcinoma cells appearing as tumor necrosis in solid tumors such as for instance carcinomas, it can now be understood why CRP also appears in carcinoma cultures and can be derived from dead or dying carcinoma cells that together with carcinoma cells showing the presence of C1 IA also at the same time often at the same time these harvested carcinoma cells will also contain dead or dying cells giving rise to CRP at the same time. The concomitant use of anti human C1 IA, anti human CRP and anti human C4 for monitoring cancer diseases is therefore a reasonable approach in detecting, monitoring, and treating carcinoma in humans with antibody and rather monoclonal and/or recombinant human anti human antibodies.

### Brief Description of disclosed aspects

The present disclosure relates to a combination of a kit for diagnostics and a kit for treatment of human cancer such as carcinomas, and brain tumors as well as certain other malignant solid cancers where it can be found that C1 IA and CRP, to processes for its isolation and characterization, to diagnostic test methods and materials based on the principle of demonstrating the presence of CRP, to antibodies and antisera with specificity against C1 IA and CRP, to the preparation of such antibodies and compositions containing same, to matrix-immobilized antibodies, and to the therapy of human cancer using for instance monoclonal and/or recombinant antisera or antibodies with specificity against C1 IA and CRP.

Important aspects are based upon the principle of utilizing antibodies directed specifically against blocking and masking proteins, in particular C1 IA AND CRP, associated with human cancer diseases and present on the membranes of cancer cells. According to the invention, this principle is utilized for treatment of cancer in vivo and for extracorporal treatment of cancer patient serum.

C1 IA AND CRP is a human cancer associated protein which may be isolated from body fluids of cancer patients, including serum, and from human malignant cancer cells, also from cell cultures. Repeated culturing and harvesting cycles of cell cultures have been performed and C1 IA and CRP isolated from the culturing media, indicating that the cancer cells themselves are able to produce C1 IA and CRP.

C1 IA AND CRP has been found to possess biological properties similar to the properties of human complement component C1 inactivator (also termed "C1 esterase inhibitor"), which is an alpha₂ neuramino glycoprotein found in the body fluids of various species, including man (Pensky et al., J.Biol.Chem., 236, 1674, 1961, Ratnoff et al., J. Exp. Med. 129. 315, Pensky et al., Science 163, 698, 1969, Nagaki et al., Int.Arch.Allergy 46, 935, 1974), but to be protein-chemically non-identical with human complement component C1 inactivator.

In the following, human complement component C1 inactivator (C1 esterase inhibitor) will be termed "C1 IA", whereas the term "C1 inactivator", used in various contexts, shall designate the group consisting of C1 IA and CRP.

C1 IA & CRP has been found to possess biological properties similar to the properties of C1 IA, including the inhibitory effect on the initial human complement component C1 activation of C4 and C2 (i.e., inhibition of C1 esterase hydrolyzing effect), the inactivation of plasmin, and the lack of effect upon the clotting time of plasma (i.e., this lack is common to C1 IA and which can not be excluded, may be also common to CRP). In accordance with the principle upon which the present invention is based, it is believed that these inhibitory effects of C1 IA & CRP play an important role in the cancer cell's defense against destruction by the human immune system.

In the literature, it has been described that a C1 IA-like protein is present on the membranes of human cancer cells (e.g., Osther et al. 1973 and 1974)

According to the present invention, said C1 IA present on human cancer and as indicated above, it has been found to be a novel with C1 IA called C1 IA and CRP. Important new developments are based upon these findings.

A tentative characterization of CRP, distinguishing C1 IA & CRP from C1 IA and other known proteins, is given in the below section termed "Characterization of C1 IA & CRP". By isoelectric focusing in LKB Multiphor, two separate bands were found by focusing in an ampholine of purified C1 IA and C1 IA & CRP, the ampholine being PAG® plate for thin layer polyacrylamide gel electrofocusing, pH gradient ranging from 4.0-8.6, 2 hours with cooling to 0.degree. C. at 500 V, 50 mamps. After the electrophoresis, the polyacrylamide gel was cut out and subsequently run in an angle of 90.degree. against 2% agarose gel in diemal buffer, ionic strength 0.02, pH 8.6 at 18.degree. C. for 12 hours, the said agarose gel containing rabbit antihuman C1 IA. By this, two precipitation lines were obtained which do not cross, whereas identical runs, but with oligospecific antiserum against both C1 IA and C1 IA & CRP, can be prepared as monoclonal antibodies or recombinant antibodies recresulting in two precipitation lines which cross. A rabbit immunized with C1 IA is not able to distinguish between C1 IA and CRP, whereas the other animals mentioned are able to identify the two proteins occurring when C1 IA methods are used for the purification of C1 IA using known chromatographic methods, where C1 IA and CRP can be isolated from pleura - or ascites fluid from patients suffering from metastatic cancer for instance of type carcinomas, such as breast cancer types and ovarian cancer types . The fact that rabbit antiserum is not able to distinguish between C1 IA & CRP and C1 IA plays an important role in the present context and is therefore emphasized. In reading the present specification, it is important to remember that a response, in immunological tests, based upon reaction with rabbit antihuman C1 IA (which is a commercially available reagent) will be a response to "C1 inactivator" or, in other words, a response to the sum of C1 IA & CRP and C1 IA.

C1 IA and probably also CRP has been isolated from media from culturing of several types of human cancer cells, including carcinomas irrespective of origin, because antibody produced in animals from the harvested culture medium produce antibodies, especially to C1 IA and CRP .

C-reactive protein (CRP) is an annular (ring-shaped), pentameric protein found in blood plasma, whose levels rise in response to inflammation. It is an acute-phase protein of hepatic origin that increases following interleukin-6 secretion by macrophages and T cells. Its physiological role is to bind to lysophosphatidylcholine expressed on the surface of dead or dying cells (and some types of bacteria) in order to activate the complement system via the C1Q complex. This binding to lysophosphatidylcholine which actually is expressed in dead or dying cells, may also be present in certain cancer cells or certain percentage of cancer cells of type carcinoma or from malignant brain tumors. CRP is composed of 5 identical, 21,500-molecular weight subunits. It is detectable on the surface of about 4% of normal peripheral blood lymphocytes. Acute phase reactant CRP is produced in the liver; CRP detectable on lymphocytes is produced by those cells (Kuta and Baum, 1986). Kilpatrick and Volanakis (1991) reviewed the molecular genetics, structure, and function of CRP. Cytogenetic location: 1q23.2 Genomic coordinates: (GRCh38): 1:159,712,288-159,714,608 (from NCBI).

On the basis of in vitro and in vivo experiments, it has been proposed that the function of CRP relates to its ability to recognize specifically foreign pathogens and damaged cells of the host and to initiate their elimination by interacting with humoral and cellular effector systems in the blood. Thus, the CRP molecule has both a recognition and an effector function (Kilpatrick and Volanakis, 1991).

Robey et al. (1984) demonstrated that CRP binds with high affinity to chromatin. It has been proposed that one of its major physiologic functions is to act as a scavenger for chromatin released by dead cells during the acute inflammatory process.

Interleukin-6 (IL6; 147620) and tumor necrosis factor alpha (TNFA; 191160) are inflammatory cytokines and the main inducers of the secretion of C-reactive protein in the liver. CRP is a marker of low-grade inflammation that may have a role in the pathogenesis of atherosclerotic lesions in humans (Blake and Ridker, 2002). The effects of TNF-alpha are mediated by 2 receptors: type 1 (TNFR1; 191190) and type 2 (TNFR2; 191191). The Nurses' Health Study (NHS) and the Health Professionals Follow-up Study (HPFS) are prospective cohort investigations involving a large number of U.S. female registered nurses and U.S. male health professionals, respectively. Pai et al. (2004) examined plasma levels of soluble TNFR1, soluble TNFR2, interleukin-6, and C-reactive protein as markers of risk for coronary heart disease among women and men participating, respectively, in these 2 studies.

Among participants who provided a blood sample and who were free of cardiovascular disease at baseline, 239 women and 265 men had a nonfatal myocardial infarction or fatal coronary heart disease (see 607339) during 8 years and 6 years of follow-up, respectively. Pai et al. (2004) found elevated levels of inflammatory markers, particularly C-reactive protein, indicating an increased risk of coronary heart disease. Although plasma lipid levels were more strongly associated with an increased risk than were inflammatory markers, the level of C-reactive protein was a significant contributor to the prediction of coronary heart disease.

For the isolation and purification of C1 IA & CRP, several methods may be used. Common to all useful methods is that they comprise separation techniques aimed at isolating and purifying a pseudoglobulin material having the properties described in the section termed "Characterization of C1 IA & CRP" from the medium in which it is found. Suitable methods comprise adsorption and gel filtration techniques which are well-known to the skilled art worker. As it has been shown that C1 IA & CRP can be prepared from media from the culturing of human cancer cells, and also that no C1 IA can be isolated from media from culturing of such human cancer cells, one unambiguous method for concentrating, isolating and purifying C1 IA & CRP is to use adsorption and gel filtration methods with concomitant immunological assay using rabbit antihuman C1 IA, for obtaining, from culturing media of the cancer cell types mentioned above, the protein reacting immunologically with the rabbit antihuman C1 IA. In such procedure, care should be taken to avoid pH values below 5.5 and above 10.5 and heating temperatures exceeding 56.degree. C., as it has been shown, vide the section termed "Characterization of C1 IA & CRP", that such conditions give rise to alterations of the biological properties of C1 IA & CRP. When designing a suitable method for concentrating or purifying C1 IA & CRP, the skilled art worker will also make use of other of the characterizing features stated in the section "Characterization of C1 IA & CRP", including the estimated molecular weight of C1 IA & CRP, which is 110,000-130,000. Evidence of the presence of C1 IA & CRP in any material is obtainable using immunological tests against antibodies or antisera giving a reaction with C1 IA & CRP which is readily distinguishable from the reaction with any other protein, including C1 IA. The preparation of such antibodies or antisera is described in the present specification. Suitable methods for concentrating, isolating and purifying C1 IA & CRP employ column chromatography adsorption and gel filtration.

It is noted that C1 IA and CRP have been found not to react with antibodies with specificity against other known antigens, especially not with antibodies against known oncofoetal antigens and, in particular, not with antibodies against alpha₂ foetoprotein or CEA protein.

A preferred method for the preparation of C1 IA & CRP from a C1 IA & CRP-containing medium is described in the section "Preparation of C1 IA & CRP" and involves column chromatography adsorption and subsequent gel filtration. A preferred column for the column chromatography adsorption is an anion exchanger resin column such as Dowex 2 .times. 8, mesh 200-400, and a preferred gel material for the gel filtration is a dextran gel such as Sephadex® G75 Superfine. Conventional unit operations are included in these procedures, e.g., dialysation and lyophilization at appropriate stages.

The medium from which C1 IA & CRP may be concentrated, isolated and purified may be a culturing medium from culturing a C1 IA & CRP-producing type of human cancer cells, or a body fluid such as serum or pleural/ascites exsudate from a patient suffering from a C1 IA & CRP-producing cancer type. As explained above, the cancer cell culturing medium will not contain C1 IA, but C1 IA will be found together with C1 IA & CRP in the body fluid from human cancer patients. For most of the practical utilities of C1 IA & CRP, the presence of C1 IA in any starting medium used does not present any serious problem, because isolates containing C1 IA in addition to C1 IA & CRP are valuable in themselves, such as will be explained below.

When cancer cells are cultivated for the production of C1 IA & CRP, suitable culturing media are Eagle minimum essential medium and RPMI synthetic amino acid medium (vide Publication 73/74 from Flow Laboratories, Irvine Ayrshire, K.A. 12 8 NB, Scotland), but any other medium which will support the growth of the cancer cells may also be used. It has been found that the best yields of C1 IA & CRP are obtained when the culturing medium contains added glutamine in an amount exceeding about 285 mg per liter, preferably exceeding 290 mg per liter. At present, the optimum amount of added glutamine has been found to be about 294 mg per liter, and addition of larger amounts does not seem to give any added advantages. Usual procedures for culturing cancer cells employ a growth phase in Eagle minimum essential medium enriched with added glutamine and a production phase in RPMI medium with added glutamine. Each phase may comprise a few days, e.g., 3-7 days; at present, 3 days is the preferred period.

As mentioned above, C1 IA & CRP is believed to play an important role in cancer cells' defense against destruction by the human immune system. C1 IA & CRP, one of them being a sialoprotein (neuraminoglycoprotein), possesses low antigenicity, and it is believed that C1 IA & CRP, together with other proteins found in cancer cell culturing media and in body fluids from cancer patients, especially orosomucoid, alpha₂ HS glycoprotein, and Zn alpha₂ glycoprotein (also (obsolete) designated Zn alpha₂ glycoprotein), "mask" the cancer cells against identification as "non-self" by the human immune system. Moreover, as has been mentioned above, C1 IA & CRP is able to inhibit activation of C4 by C1, and this is believed to be one of the main reasons why cancer cells are not effectively attacked by the immune system, which could be due to the fact that a solid malignant tumor may easily consist of dead or dying cancer cells, to a cell turn over effect that we do not know much about, but which would explain that CRP could be present at the same time as C1 IA. Actually this phenomenon according to experiences in pathology report says that tumor necrosis is present, this means that dead breast cancer cells can be seen within the tissue sample. Tumor necrosis is often limited to a small area within the sample. Its presence suggests a more aggressive breast cancer.

To investigate the relation between necrosis and hypoxia in for instance breast cancer or breast carcinoma Tomes et al (Tomes L, Emberley E, Niu Y, Troup S, Pastorek J, Strange K, Harris A, Watson PH. Necrosis and hypoxia in invasive breast carcinoma. Breast Cancer Res Treat. 2003 Sep;81(1):61-9) examined the expression of hypoxia-associated markers HIF1, CA IX and GLUT1 by immunohistochemistry in 97 invasive ductal carcinomas. This selected series comprised 48 tumors with extensive necrosis and 49 control tumors without necrosis. Over 90% of necrotic and 30% of non-necrotic tumors expressed at least one hypoxia marker. The group also observed expression of hypoxia associated markers in tumor stroma. Examination of primary human breast fibroblasts in vitro confirmed that CA IX mRNA and protein can be induced by hypoxia. Survival analysis of 53 cases found that the subset of tumors with stromal hypoxia exhibit better prognosis (p=0.027). Tomes' et al's results indicate that necrosis is often accompanied by hypoxia but that hypoxia without necrosis may also be a frequent occurrence. The use of several hypoxia markers may identify a continuum of hypoxia in tumors, which can be sub-classified by different co-expression patterns. Tomes conclude that stromal and epithelial hypoxia may have different biological backgrounds and that stromal hypoxia may affect survival.

In such cases and in many other description of carcinomas it is known that necrosis often can be present and therefore it is quite plausible that in carcinomas isolated, or ascites or pleural fluid from patients with cancer metastases obviously could have necrotic tissue containing dying or dead cells so that the cells, besides the presence of C1 IA coated on a certain part of carcinoma cells also may contain the presence of CRP originated from the dying or dead cells.

Expressed in another manner, the ability of cancer cells to produce C1 IA as a membrane protein may be one of the main reasons why these cancer cells possess the ability of blocking both the afferent limb and the efferent limb of the immune response. At the same time CRP could be present in some of the dead or dying cancer cells, most probably taken place in solid cancers.

The afferent limb is apparently blocked mainly because of the masking effect of C1 IA AND CRP (symbody-effect), and the efferent limb seems to be blocked both at the humoral immune defense level and at the cellular immune response level, the former being a block of the complement system at the stage mentioned further above, and the latter being ascribable to the strongly negative electric charge of the cancer cell membrane due to the negatively charged C1 IA AND CRP and other strongly negatively charged sialo-compounds which will repel the negatively charged lymphocytes.

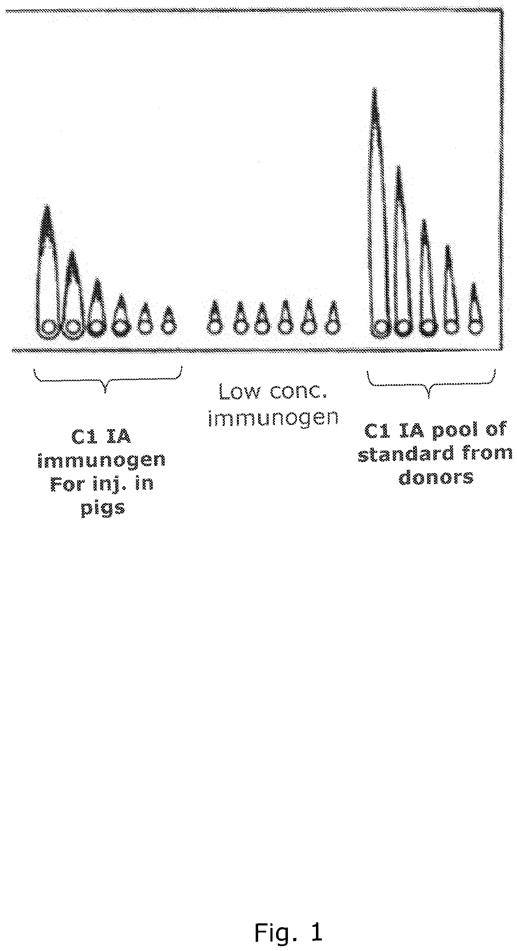

A schematical representation of the human complement system and the blocking and inhibitory action of C1 inactivator thereon is shown in FIG. 1. When a complement reaction has been initiated by the Fc fragment of an immunoglobulin, activated C1 will, in the normal course of a complement reaction or cascade, activate complement components C4 and C2 whereafter the complement reaction will proceed to end point and lysis of the "non-self" material or antigen with which the immunoglobulin had reacted, but C1 inactivator may inhibit C1's activation of C4, in which case C4 will not become attached to the antigen, and the further complement reactions will not proceed.

The body fluids of healthy human beings contain C1 IA as a necessary controller of the complement system. It is well established that the controlling effect of C1 IA in the healthy organism (said control effect manifesting itself via inhibition of C1r and C1s, resulting in lack of C1 activation of C4) is related to the concentration of C1 IA, and that normally, the inactivating effect of C1 IA on the C1 molecule is limited to inactivating any excess activation of C1 which could otherwise lead to a complement cascade in the serum phase, resulting in Quincke edema. Normal values of C1 IA in the serum phase are up to 15- 35 mg%.

It has now been found that patients suffering from verified cancer diseases often show abnormally high serum levels of C1 inactivator, all of which increase is probably ascribable to the C1 IA AND CRP present in the patient's serum as well as complement component C4 elevation in serum of these patients.

In estimating to which extent patients suffering from verified cancer have elevated levels of C1 inactivator (C1 IA), a hundred patients suffering from cancer of various origin, i.e., carcinomas, reticulosarcomas, and in seemingly rare cases in some types of lymphomas, were checked for serum concentration of C1 inactivator, using Laurell rocket technique with rabbit antihuman C1 IA. Control experiments included equivalent examination of serum from healthy subjects and from patients suffering from verified non-malignant diseases. The results are shown in FIG. 2. It appears that the mean values of C1 IA from healthy subjects are much lower than from patients suffering from cancer. Also, patients suffering from non-malignant diseases showed C1 inactivator levels with mean values lower than that of cancer patients' and approximately in the normal range as found in healthy subjects.

The elevated concentrations of C1 inactivator in serum from patients suffering from cancer is believed to be mainly due to the presence of C1 IA liberated from cancer cell clones. By Ouchterlony technique it was verified that the cancer patients found to have raised values of C1 inactivator revealed the precipitation lines for both C1 IA and CRP, and that patients suffering from non-malignant diseases and healthy subjects did not show any significant presence or very low levels of CRP (both measured against oligo-specific pig antihuman C1 IA/C1 IA AND CRP). A typical example of an Ouchterlony serum investigation of this type is shown in FIG. 3.

As previously described Complement C4 is also elevated in cancer patients most probably due to blockage of activation C4 that can not be activated by the Clqrs because of lack of disassociation of this complex due to blocking or inhibiting effect of the presence of C1 IA (elevated in cancer patients due to the presence of cancer cells).

Estimations of the level of complement component C4, performed on the same groups of patients, showed almost the same mean values for healthy subjects and patients suffering from non-malignant diseases, whereas patients suffering from cancer showed significantly elevated C4 levels, vide FIG. 2.

It has been found that values of "C1 inactivator" (C1 IA) exceeding concentrations of approximately 50 mg% (50 mg/100 ml) (as estimated by rocket electrophoresis using rabbit antihuman C1 IA) will often coincide with elevation of C4 indicating that the complement system is blocked at the C4 stage, i.e. at the stage where C1 activation of C4 should occur with consequent consumption of C4 (activated C4 becomes attached to the membranes of the cells to be lysed by the complement system). Investigating progressively growing cancer, it has been found that "C1 inactivator" and C4 rise continuously until death of the patient. It has also been found that subterminal and terminal values of C4 reach values up to 160 mg%, and C1 inactivator values up to approximately 120 mg%. In serum from some patients with verified cancer with primary small cancer cell clones, values of about 40-50 mg% of C 1 inactivator (C1 IA) have been ascertained without any signs of abnormally raised C4 levels; however, when these patients have been subjected to surgery, C1 inactivator levels either decline after a period of about 3 weeks and will remain in the normal range, concomitantly with C4 remaining in normal range (indicative of a successful radical operation), or an elevation of C1 inactivator accompanied with elevated C4 is found. The explanation of the latter phenomenon may be that a supposed radical operation may have elicited a raised concentration of C1 inactivator as well of high CRP in the serum phase due to liberation of CRP from damaged cancer cells and from cancer cells thrown into the blood stream, and that this increase in C1 inactivator concentration caused blocking of the complement system. In these cases C4 will also be elevated. When an elevation of CRP, and possibly at the same time also an elevation of C1 IA occurs in a serum sample, but C4 is not significantly elevated, the patient then has another reason for this pattern, namely an inflammatory disorder or disease such as viral hepatitis, acute or chronic hepatitis B, heart related disease or other inflammatory disease for instance caused by cell death or necrosis due to another disease than cancer.

On the background of the above explanation, it will be understood that a radical increase in the chances of effectively treating cancer would be achieved if the inhibition of the immune response which takes place in case of cancer could be neutralized, that is, if the blocking of the immune defense could be obviated, and if the cancer cells could be "demasked" so that they were more readily recognized by the immunosurveillance of the human immune system. The present disclosure provides such deblocking and demasking.

When for instance a pleural fluid or ascites fluid from a cancer patient with metastases is subjected to purification using methods described for purification of C1 IA, and important aspect is that this form of purification for C1 IA will at the same time contain CRP in the same fractions as C1 IA. Therefore, when using purified C1 IA from cancer patients using methods described in this specification the resulting immunogen which has been used for vaccination of animals such as pigs, an important aspect relates to antisera or antibodies with specificity against both C1 IA and CRP.

Such antisera may be produced by immunizing selectively immunizable animals, e.g., pigs with vaccines containing C1 IA AND CRP as an antigenic component, and recovering serum from the immunized animals. Based upon the finding that orosomucoid, alpha₂ HS glycoprotein, and Zn alpha₂ glycoprotein are proteins which may be isolated from cancer cell culturing media, especially in the early stages of the culturing (before trypsinizing and subculturing), that these three proteins are notoriously proteins showing very low antigenicity, and that orosomucoid is present in high concentrations in pleura exsudate from cancer patients, these additional three proteins are believed to take part in the "masking" of cancer cells, for which reason it is preferred to include also these three proteins in the vaccines to be administered to the host animals for the preparation of the antisera for cancer therapy purposes, in order that the antisera will contain also antibodies against these animals proteins. Furthermore, as it cannot be precluded that C1 IA will in several cases be the predominant C1 inactivator in the fluid phase, whereas C1 IA AND CRP is the C1 inactivator present on the cancer cells, it may be presumed that the most effective antiserum is one which is able to attack both C1 IA and CRP.

In the following, the effect of the antisera or antibodies will be explained in greater detail. For brevity and better survey, the term "INA" (immune neutralizing-deblocking antiserum) used in previous treatment of cancer patients with porcine anti human antibodies (that have been immunized with C1 IA and CRP) was used to treat patients treated with the so-called INA antibody, is now by injection or infusion of the so-called INA antibody was capable of causing shrinkage of carcinomas (advanced carcinoma metastases) concomitantly with decrease in C1 IA and C4 as found in some of the patients treated with porcine antibody against C1 IA, which the patients have also been treated with injection or infusion of anti C1 IA and anti CRP. When applying monoclonal or may be rather recombinant anti C1 IA antibody, the effect of the treatment may be even more efficient when the composition is anti C1 IA and anti CRP antibody either given as a mixture of these two proteins or injection as anti C1 IA and injection as CRP separately, meaning as a treatment test kit either containing of a mixture consisting of recombinant anti C1 IA and CRP antibody composition, or two kits where one kit consists of recombinant anti C1 IA administered separately and another kit consisting of anti CRP administered separately.

The antihuman C1 IA AND CRP antibodies react with C1 IA AND CRP on the cancer cell membrane, by an antigen-antibody reaction. For example, the interaction between INA and the cancer cells may be shown by marking INA (e.g. with FITC (Fluorescein Isothio cyanate)) and incubating the human cancer cells with the marked INA. The cancer cells will show positive marking. Cells from the same culture pre-incubated with neuraminidase do not show any marking after incubation with marked INA antiserum, which indicates that the antigenic determinants of C1 IA AND CRP are decomposed by neuraminidase. When INA blocks the determinants of the C1 IA AND CRP on the cancer cell membranes, the cancer cells become accessible to attack by the human humoral immune defense system, which is demonstrated, e.g., in the following manner: Human cancer cells are incubated with INA and thereafter incubated with serum from the same patient or from other patients with the same type of cancer. After incubation for e.g. 6 hours, the cancer cells become detached from the culture flask, and after e.g. 18 hours, all the cancer cells are dead. Mesothelial cells and fibroblasts treated in the same manner continue to grow in the culture flasks, undisturbed by the incubations, and in control cultures without incubation with INA, the incubation with serum did not affect the cancer cells. This proves that anti C1 IA may act as neutralization or elimination of the inhibiting or blocking factor related to C1 IA on cancer cells thus killing a certain amount of cancer cells and anti CRP will bind to CRP coated dying cancer cells or dead cancer cells thus eliminating another group of the cancer cells which also appears to be a part of the solid carcinoma mass, thus Clqrs will be activated both when antibody against C1 IA is bound to certain group of cancer cells and Clqrs will be activated when antibody against CRP is bound to another group of cancer cells thus activating complement reaction against both groups of cancer cells found in the carcinoma. Thus INA that was produced in pigs as described above might be able to neutralize this blocking effect activating Clqrs on the C1 IA coated cancer cells and the remaining shrinkage of the carcinoma could be due to the activation of Clqrs caused by the binding of a anti CRP to the other part of cancer cells. When C1 IA AND CRP is neutralized by INA in vitro, its inhibitory effect on C1 activation of C4 is neutralized which means that the blocking of the action of the immune system ceases, C4 becomes attached to the cancer cell membranes, and the further action of the complement system takes place resulting in a lysis of the cancer cells; after the activation of C4, the complement reaction cannot be stopped by any influence from C1 IA and CRP, vide FIG. 1.

### Brief added Description

Novel Biological Methods for Diagnosing and Monitoring Immune Blocking Cancer and Immune De-blocking Cancer Treatment For several years Dana-Genetic's researchers have focused on the de-blocking immune responses in cancer patients taking place in the innate immune system, which includes complement.

The general known functions of the vertebrate innate immune system include:
- Recruiting immune cells to sites of infection, through the production of chemical factors, including specialized chemical mediators, called cytokines.
- Activation of the complement cascade to identify bacteria, activates cells, and promotes clearance of antibody complexes or dead cells.
- Identification and removal of foreign substances present in organs, tissues, the blood and lymph, by specialized white blood cells.
- Activation of the adaptive immune system through a process known as antigen presentation
- Acting as a physical and chemical barrier to infectious agents.

We found that the innate immune system actually appears to be blocked by cancer cells, and among these especially carcinoma cells in humans. We then identified the reasons for the blocking and have found ways to de-block the cancer cells. This was suggested even in an early pilot study involving treatment of a few patients suffering from advanced metastatic carcinoma (patients in end stage cancer).

This proprietary de-blocking immune treatment approach on carcinomas enables a new methodology removing or neutralizing the otherwise protective coating of carcinoma cells with C1 inhibitor also called C1 inactivator (C1 IA), which acts as a blocking protein inhibiting the activation of complement component Clqrs that normally is activated thus enables the innate immune system by killing microorganisms and cause lysis or rejection of foreign cells (e.g., for instance rejection observed after allogeneic transplantation), and C Reactive Protein (CRP) that under certain conditions interfere with complement component C5 convertase blocking C5b. C1 inactivator presence on carcinoma cells led to measurement of serum C1 inactivator and C4, which was found to be a possible predictor of cancer. Held together with CRP a proprietary diagnostic and cancer treatment monitoring system was developed.

The C1 IA blocking of the complement Clqrs prevents Clrs activation of C4 conversion to C4b, preventing further complement cascade and therefore prevents lysis or cell death. In this manner the carcinoma will survive unrecognized and blocked from the immune surveillance system. Carcinomas are the dominant type of cancer in humans (∼80% of all cancers).

Furthermore, it was found that an additional blocking protein also was present. By harvesting the C1 inactivator from human carcinoma cell cultures we now know that the additional blocking factor appeared to be C Reactive Protein.

The above described blocking of carcinomas and malignant brain tumors will be utilized to create a future diagnostic - and monitoring Test kit as well as treatment approach.

Besides these findings in carcinomas and certain sarcomas, we also identified the blocking C1 inactivator on malignant brain cancers such as astrocytomas and glioblastomas (Osther K et al, Demonstration of a complement inactivator on cultured cells from human malignant brain tumors, Acta Neurol. Scandinav. (1974),50: 681-689).

The initial attempt using de-blocking antibodies in the clinical pilot study consisted of treatment of carcinoma patients with purified pig immunoglobulin G antibodies against C1 inactivator and anti C-Reactive Protein, which were injected I.V. in six (6) patients with advanced carcinomas and metastases approaching end stage of their disease. As described above this pilot study was based on the findings of C1 inactivator coating cultured human carcinoma cells as a "Complement Blocking Factor". However, the treatment using pig IgG proteins had to be discontinued after a few treatment attempts. Otherwise, patients treated with pig IgG quickly produced antibodies against pig proteins as indicated by a significant increase in serum IgM after infusion with pig IgG.

Our present specification is first to use monoclonal antibodies in our diagnostic and monitoring testing, which in clinical laboratories can be based on ELISA, immunoturbidimetry, nephelometry; and kits such as rapidimmunodiffusion, and a visual agglutination can be used in Doctors' office.

Based on the blocking effect described above our second intention is to develop Recombinant Technology to produce human de-blocking antibodies for treatment of cancer.

The titer of the C1 IA immunogen was at that time estimated by testing the immunogen on Laurell Rocket immunoelectrophoresis (Walker JM. Rocket immunoelectrophoresis. Methods Mol. Biol. 1984;1:317-23), and quantified comparing a serum C1 IA control made by using a batched control consisting of serum C1 inactivator from a representative number of healthy donors. Testing was performed on Laurell rocket immunoelectrophoresis (see figure 1).

Dana Genetic Monoclonal Antibodies to be used in Cancer Diagnostic and Monitoring Test Kit

Background for the development of Dana Genetic's Immune Diagnostic/Monitoring Blocking Test Kit.

The antibodies used were rabbit anti human C1 inactivator and rabbit anti C4 serum from Behring Institute, Marburg, Germany. The C1 inactivator and C4 testing was done using serum from human carcinomas from different types of cancer ranging from lung cancer, breast cancer, other gynecologic cancers, prostate cancer, kidney cancers, bladder cancer, melanomas and other carcinoma types . These serum samples were compared to serum from patients with non-malignant diseases and from healthy donors . The serum samples were from departments of oncology, gynecology, and department of surgery from Copenhagen based hospitals, as well as from Copenhagen Municipal Blood banks, see figure 2.

Our initial diagnostic testing comparing levels of serum C1 inactivator (serum C1 inhibitor) and serum complement component C4 was based on using rabbit anti human polyclonal anti C1 inhibitor antibody and rabbit anti human polyclonal antibody against C4, and the immune method for measurement was Laurell Rocket Immunoelectrophoresis (Walker JM. Rocket immunoelectrophoresis. Methods Mol. Biol. 1984; 1:317-23), see results in figure 2.

### Measurement of C1 inactivator Coat on Carcinoma Cell Cultures and Sub-cultures

These results are based on our laboratory findings during culturing carcinoma cells from carcinoma biopsies from patients with different types of carcinomas and metastases as well as from body fluid adjacent to areas of metastases in carcinoma patients as listed above.

The carcinoma biopsies were explanted into cell culture flasks and on micro slides intended for incubation of the cells with rabbit anti human C1 inactivator (C1IA) labeled with fluorescein isothiocyanate (FITC), also called rabbit anti human C1 inhibitor - FITC. The cell samples cultured on micro slides with adherent anti C1 IA-FITC was measured on a Leitz MPV2 cytophotometer mounted on a phase contrast microscope equipped for measuring immunofluorescence on single cells on microslides from cultured and sub-cultured carcinoma cell cultures.

As shown in figure 3, a number of 37 carcinoma cell cultures from 37 cancer patients were measured using this method. The cell number showing relative concentration of C1 inactivator (C1 inhibitor) is expressed in percentage for each cell culture as measured by FITC excitation giving a specific significant signal (control signal identified when establishing cut-off borderline and deducted from specific excitation).

Sub-cultures from 24 explants (which had shown specific C1 inactivator coat by significant high signal in the cytophotometer) was again tested more than 3 days after having been trypsinized, washed and explanted in culture medium in in Leighton tubes (two (2) micro slides from the same patient, where one micro slide was measured and showed no excitation signal 1-2 hours after incubation with rabbit anti C1 inactivator-FITC, and the other paired micro slide with the same cells were tested after having been incubated for more than 3 days, showed again presence of C1 inactivator on the cell surfaces as indicated by the measurements, which can be observed in the second column. C1 inactivator is also present on certain cells in healthy individuals, such as monocytes. However these cells are not adherent and would be eliminated during the buffer washings, whereas the cancer cells will be adherent to the microslides. Also Kupffer cells in any liver tissue are known to be producing C1 IA.

These results coincide and correspond well with the findings of the level of C1 inhibitor (C1 inactivator) and C4 in serum samples from carcinoma cells, compared to serum from healthy donors and from patients with non-malignant tumors. Thus indicating that the production of C1 inactivator from the carcinoma cells corresponds well with the findings of higher level of C1 inactivator (C 1 IA) in serum, and postulated by us to be caused by the presence of carcinoma cells in the patients. The complement C4 measurements reflect the blocking of C4 which is illustrated in figure 3, which most probably is overproduced by the liver due to the un-balanced complement system.

The relative numbers of cells showing positive C1 inactivator signal was expressed as a percentage of cells tested positive from the first testing, indicating that the C1 inactivator coat was re-established 3 days after trypsinization (which was proven to remove the C1 inactivator coat), again indicating for us that these carcinoma cells were capable of re-producing the C1 inactivator on their cell membrane during the 3 days of incubation. Pre-saturation studies were done on 12 carcinoma cell cultures which were among those tested positive for C1 inactivator before.

Paired samples on micro-slides were pre-incubated with un-labeled rabbit anti human C1 inactivator, then washed and incubated with FITC labeled anti C1 inactivator. These cell samples did not show any specific excitation signal indicating the specific binding of the rabbit anti C1 inactvator to the cell membrane. A number of 12 biopsies from non-malignant adenomas and fibromas were tested for C1 inactivator coat and showed no specific C1 inactivator coat on their cell membrane (See figure 3).

### Dana Genetic's Immune Diagnostic/Monitoring Blocking TRIADE KIT

This "TRIADE Kit" contains monoclonal antibodies for separate quantitative determinations in human blood of
1. C1 inactivator
2. C Reactive Protein (CRP)
3. Complement component C4

In theory cancer patients, and in particular carcinoma patients are most probably expected to show the following pattern in the TRIADE Kit:
C1 IA, CRP, Complement C4 = possible carcinoma or other cancer type

As the first product related to cancer, Dana Genetic will introduce the Blocking Test Kit (TRIADE Kit) intended for
1. Diagnosing early cancer (e.g., carcinomas)
2. Monitoring start and follow-up of any cancer treatment ranging from surgery, chemotherapy, irradiation treatment, immune - oncological effect of monoclonal antibodies and various other treatment aspects such as inhibitors (Tyrosine Kinase Inhibitors, etc.) e.g., Sunitinib (TKI).
3. Monitoring De-Blocking immune treatment of Cancer.

Dana Genetic's development of Human Recombinant Antibodies as the De-blocking Immune Therapy for patients with carcinoma, certain sarcomas, leukemia and malignant brain tumors

Developing human recombinant antibodies against C1 inactivator and C Reactive Protein will be done in human transfected cell cultures capable of being manufactured in an industrial site. The antibodies will be tested in in vitro laboratory studies involving cultured carcinoma cells and malignant brain tumor cells such as astrocytomas and glioblastomas.

Dana Genetic will develop a Human Recombinant anti C1 inactivator IgG, and Human Recombinant anti C-Reactive Protein (anti CRP) IgG and get this de-blocking product tested for safety, toxicology and pharmacology using competent CRO services, designed to offer a CRO validation, where the testing will include:
1. Characterizing any adverse effects
2. Identifying potential target organ toxicity
3. Determining dose-levels
4. Defining mechanisms of action
5. Assessing risk to humans, animals and the environment Based upon previous pilot studies involving a few cancer patients, there will be performed validation of the Recombinant Human anti C1 inhibitor and the Recombinant anti C-Reactive Protein using separate activity and stability studies. Using the previous pilot study treating cancer patients with Pig anti C1IA/CRP IgG as a model, Dana Genetic will initiate the below treatment studies with "Human recombinant anti-C1 IA/CRP IgG" replacing pig anti human C1 IA/CRP IgG:
   - Safety studies in patients with advanced metastatic carcinoma in approved Clinical Research Clinics (CRO)
   - Phase I - II, continued expanded clinical safety and efficacy studies including escalation of doses and frequency (CRO)
   - Phase III clinical trials on several oncology and neurosurgery departments in patients with advanced metastatic carcinomas (e.g., breast carcinoma, gastro intestinal carcinoma, pancreas carcinomas, kidney carcinoma, with metastases (RCC), etc. in three centers or more.

The development and manufacturing of the "Recombinant Human Antibodies" as de-blocking antibodies against the protective coating of carcinoma cells with C1 inactivator and, as recently found, against C Reactive protein harvested from human carcinoma cell cultures will be used as "De-blocking immune treatment". This novel method builds on targets we have identified in the type of cancers listed above, irrespectively of where and in what organ carcinomas are located.

This treatment model will be used as "De-blocking immune treatment" against such cancers as human carcinomas from different types of cancer ranging from lung cancer, breast cancer, other gynecologic cancers, prostate carcinomas, kidney cancers, bladder cancer, melanomas and other carcinoma types.

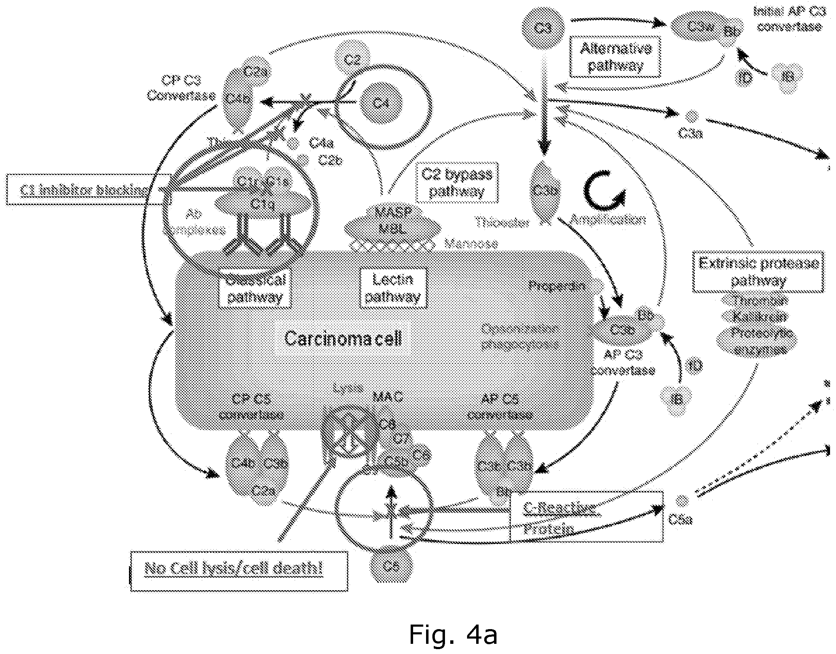

As illustrated in figure 4a, WT rC1q essentially retained the ability of serum C1q to trigger activation of C1s-C1r-C1r-C1s, yielding 95% activation upon incubation for 1 h. WT rC1q and its variants were each mixed with the proenzyme C1s-C1r-C1r-C1s tetramer and the resulting complexes were tested for their ability to self-activate upon incubation at 37 °C in the absence of C1 inhibitor.

C1q As expected, addition of C1-inhibitor abolished the activation process in all cases, yielding activation values of 0.5-5% after incubation for 1 h.

There are three areas of blocking of the immune system on the carcinoma cell. The Clqrs complex normally activated by an antigen - antibody reaction on a surface of a foreign cell, is blocked on the cell membrane of the carcinoma cell due to C1 inactivator blocking of the Clqrs complex to Clrs on the cell surface. This inhibition causes blockage of C4 conversion to C4b in the innate immune system inhibiting the complement cascade to take place on the carcinoma cell as shown in figure 4a.

In relation to the inhibitory reaction by C Reactive Protein (CRP) on the carcinoma, it is anticipated that this reaction resembles the reaction observed by CRP during myocardial injury occurring in the coronary blood vessels inhibiting C5 convertase from activating C5 to C5b. The inhibition of C5b prevents the complement cascade from taking place from C5b through C9 preventing lysis or killing of the cell (see figure 4a).

This inhibition causes other CRP-initiated signals through interactions with neutrophil Fc receptors to have an overall anti-inflammatory effect. Thus, the main biological function of CRP in relation to carcinoma cells appears to be the same reason as in the cardiovascular system, where it constitutes additional functions, such as participation in atherogenesis and pathogenesis of myocardial injury after myocardial infarction (Volanakis JE, Mol Immunol. 2001 Aug;38(2-3):189-97).

### Dana Genetic immune blocking - TRIADE KIT

The Dana Genetic TRIADE KIT may be a valuable approach as a possible cost efficient method for early detection of various types of cancer (e.g.,carcinomas), prior to significant manifestation of a cancer disease in the patient.

Therefore Dana Genetic intends to introduce this proprietary Dana Genetic -Kit to be used as a future diagnostic /monitoring tool to be offered to the physicians, and specialists as a possible predictor of early stages of cancer and especially carcinomas, which constitute approximately 80% of all cancer types in humans.

### Background for the selection of blocking proteins in the Dana Genetic Diagnostic/Monitoring Kit

The test kit will be developed as a laboratory test, such as ELISA, immunoturbidimetry, nephelometry, etc.; and as a "Doctors' Office test", rapid immunodiffusion, and visual agglutination kit.

The Dana Genetic Diagnostic/Monitoring Kit is considered primarily as an approach to an "immune blocking diagnostic test" based upon the three immune blocking components identified in the innate immune system (see figure 4a). The components are:
- C1 inactivator
- C Reactive Protein
- Complement component C4

The anticipated abnormal values demonstrated below are preliminary estimation, based on our previous findings in cancer patients, as demonstrated in figure 1.

### (Normal values: C1 IA: 15 - 40 mg%, CRP: 0 - 10 mg%, C4: 12 - 60 mg%)

- Carcinomas and other forms of cancer, certain sarcomas, and certain blast leukemias;
   ∘ C1 IA increased, CRP increased C4 increased.
- B -Cell lymphoproliferative disorders, B-Cell malignant disease (B-cell lymphoma, Hodgkin's and Non-Hodgkins lymphomas, Chronic lymphatic leukemia, other monoclonal gammopathies; acquired angioedema (often due to the above diseases)
   ∘ C1 IA decreased (<15 mg%!), CRP increased (>10 mg%), C4 decreased (<12 mg%) (Gompels MM, J Clin Pathol 2002;55:145-147)).
- Autoimmune disorders, SLE, rheumatoid arthritis, ulcerative colitis, Crohns disease, etc,
   ∘ C1 IA decreased (<15 mg%!), CRP increased >10 mg%), C4 decreased (<12 mg%) (Gompels MM. J Clin Pathol 2002;55:145-147).
- Heriditary angioneurotic edema, type I or type II
   ∘ C1 IA decreased (<15 mg%!), normal CRP (0-10 mg%), C4 decreased (<12 mg%) (Gompels MM. J Clin Pathol 2002;55:145-147).

### C1 Inactivator

C1 inactivator (also called C1 inhibitor) is the only known physiological inhibitor of the classical complement pathway in blood and tissue. However, C1-inhibitor is also a major regulator of the contact-kinin system by blocking of activated FXII (FXIIa) and plasma kallikrein,

Serum C1 inactivator level: Normal range between 15-40 mg%.

C1 inactivator is routinely used for treatment of patients with hereditary angioedema HANE or HAE. The amount of infused C1 inactivator for HANE (HAE) conditions is efficient for treating these attacks with doses around 20 Units/kg body weight, and has been evaluated for repeated attacks with no significant change in dose range using Berinert (a plasma C1 Inhibitor).

Monoclonal C1 inhibitors or inactivators are also used for the treatment of this condition.

When secondary angioedema appears in relation to lymphoproliferative disorders or malignant lymphogenic diseases as described above, the patients found deficient in C1 IA should also receive adequate and immediate treatment with C1 inactivator.

### C Reactive Protein

Small amounts of C Reactive protein (CRP) are always present in humans, but significantly elevated in patients suffering from a variety of diseases including certain types of cancer and as we recently found especially elevated in serum in patients with carcinoma or carcinoma metastases.

CRP was recently found to be part of the "immune blocking proteins" involving the complement system and found to be one of the two proteins that we initially isolated from human carcinoma cells detected by using an additional testing method, a variant of the Radial Mancini immunodiffusion (RID test named a "Double Mancini immunodiffusion test" (see figure 5).

The presence of this other "blocking" protein, now known as being CRP was tested by measuring the size of the precipitation ring developed using Mancini technology as shown in figure 5 .

This presence of CRP in carcinoma tissue has recently become a new focus for researchers regarding certain cancers, such as carcinomas and has been found to be increased relative to how progressive a malignant cancer might be.

Factors predictive of survival have been identified in patients from the Western world with metastatic clear cell renal cell carcinoma (mCCRCC) as evidenced in studies where patients were treated with Sunitib, a Tyrosine Kinase Inhibitor used for treatment of kidney. It was indicated by CRP decrease in patients showing remission of their kidney cancer. These results suggest a possible relation to tumor conditions a response to treatment with sunitib found in the "Western world".

However, this CRP prediction could not be used as a prognostic tool when studying survival in Japanese patients with mCCRCC treated with first-line sunitinib for treatment evaluation in Japan (Kawai Y, Osawa T, Kobayashi K, Inoue R, Yamamoto Y, Matsumoto H, Nagao K, Hara T, Sakano S, Nagamori S, Matsuyama H. Factors Prognostic for Survival in Japanese Patients Treated with Sunitinib as First-line Therapy for Metastatic Clear Cell Renal Cell Cancer. Asian Pac J Cancer Prev. 2015;16(14): 5687-90).

Originally, CRP was defined as a substance, observed in the plasma of patients with acute inflammations, as for instance in infections that reacted with the C polysaccharide of the pneumococcus as discovered by Tillett et al in 1930 (Tillett WS and Francis T. J Exp Med. 1930 Sep 30;52(4):561-71). It is one of those plasma proteins that originally were called "acute phase reactant" because of a pronounced rise in concentration after tissue injury or inflammation; In the case of CRP, the rise may be 1000-fold or more. CRP is composed of 5 identical, 21,500 molecular weight subunits. CRP is produced in the liver and detectable on lymphocytes. CRP is well known to be a systemic marker in response to cytokines, and has also been identified to be increased in viral diseases such as chronic hepatitis B (Shima M1, Nakao K, Kato Y, Nakata K, Ishii N, Nagataki S. Comparative study of C-reactive protein in chronic hepatitis B and chronic hepatitis C. Tohoku J Exp Med. 1996 Mar;178(3):287-97) and HIV infections (Noursadeghi M1, Miller RF. Clinical value of C-reactive protein measurements in HIV-positive patients. Int J STD AIDS. 2005 Jun;16(6):438-41).

### C4

Complement component C4 is a protein involved in the complement system. When the Clqrs complex is bound to an antibody -antigen reaction and converted the C1qrs complex to C1rs, the converted Clrs is then activating C4, which is then cleaved into C4b that in conjunction with complement component C2a via C3 convertase activates C3, - and the complement cascade will be activated all the way to C9. See figure 4b, where the de-blocking effect is eliminated.

Component C4b will under this process be bound to the cell membrane during the complement cascade process as well as component C5 converted to C5b. These components will then bind in sequence on the cell membrane through C9, which then will penetrate the cell membrane and cause lysis of the cell (cell death). If C4 is not activated by C1qrs, which can be inhibited by C1 inactivator, will prevent Clrs to activate C4. This will result in an increase of C4 that cannot be activated to C4b, and C4 will be the blocking component identified indirectly by an increase of circulating C4, meaning increased C4 in blood.

Therefore, in regards to the innate immune blocking C1 inactivator, C4 will be indirectly blocked (see figure 4a.

In autoimmune disorders complement factor C4 will often show decreased level of serum C4 below normal values. In Systemic Lupus erythematosis (SLE) complete deficiency of Complement C4 may be found and is suggesting a strong genetic risk factor for SLE. C4 is encoded by two different genes, C4a and C4b, which show considerable gene copy number (GCN) variation. This study investigates the association of total C4, C4a and C4b "GCN" with SLE (Pereira KM, et al. Low C4, C4A and C4B gene copy numbers are stronger risk factors for juvenile-onset than for adult-onset systemic lupus erythematosus. Rheumatology (Oxford). 2016 Jan 22).

### Dana Genetic immune blocking- Monitoring Use

In the early studies on the 6 patients treated with pig anti C1 IA/CRP IgG antibody, we monitored the effect of the pig IgG "De-blocking" treatment by measuring serum C1 inactivator (C1 IA) and complement component C4.

Among the effects which have been observed after administration of the porcine INA to human cancer patients are:
(1) Decrease in serum concentration of "C1 inactivator" (C1 IA), as measured with rabbit antihuman C1 IA.
(2) Decrease in serum concentration of C4.

In addition, several manifestations of remissions, such as decrease in tumor mass have been noted. However, C1 IA and C4 are important and easily determinable indicators of the course of the disease before, during, and after administration of INA, and, additionally, of any type of cancer therapy, such as is explained above. However, an indication of significant increase of IgM following a few days after administration of the porcine INA was most probably caused by immunization of the patient against porcine infused proteins, making it impossible to infuse the INA treatment more than one (1) or at the highest two (2) times, and further treatment with the porcine INA was the of course impossible due to the presence of immune anti porcine antibodies.

As an additional manifestation of remission it is seen that the low amount (low percentage) of T lymphocytes often found in patients suffering from cancer, especially upon cytostatic treatment or irradiation, will rise to normal values within a few weeks from the start of INA treatment.

The preparation of specific antibodies against C1 IA AND CRP, also opens up the possibility of establishing an unambiguous diagnosis of cancer. This diagnosis is based upon the ascertainment of the presence or absence of C1 IA AND CRP in human body fluids, for practical purposes especially in human serum as well as testing the level of C4.

The presence or absence of C1 IA AND CRP in a sample is ascertained by subjecting the sample to immunological reaction with antihuman C1 IA AND CRP under such conditions that the result of the immunological reaction is easily distinguishable from any response to C1 IA in the sample. This means that INA antiserum and other materials containing antibodies or immunologically active modifications or derivatives thereof are extremely valuable diagnostic materials. Further discussions of the diagnostic aspect of the present invention are given below.

### Recombinant anti C1 IA and recombinant anti CRP

When having produced monoclonal or recombinant antibodies against human C1 IA and/or CRP, depending on the titer of the anti human C1 IA and the titer of the anti human CRP, may consist of a purified composition of monoclonal, mixed monoclonals against C1 IA and mixed monoclonals against CRP according to the invention for therapy of human cancer, preferably being IgG solution that among other things will activate the complement system when finding its target on the cancer cells, an amount of 100 - 1000 mg IgG dissolved in about 2 ml isotonic saline (or equivalent, e.g. isotonic glucose). Such composition could practically be supplied as, e.g. one kit of mixed anti C1 IA and anti CRP in for instance a 2 ml vial either freeze-dried or in solution as an injectable either for use in intravenous infusion diluted in the normal amounts of isotonic saline or equivalent. In the case of a malignant brain tumor,e.g., astrocytoma grade II, III or glioblastoma corresponding to grade IV, the antibody may be given intraspinally or intrathecally or even through an Ommaya catheter applied into the tumor of the brain. Another preferred composition is a lyophilized human IgG immunoglobulin for reconstitution. Preferred administration ranges are about 2-15 mg of immunoglobulin per kg of patient body weight per day with daily to weekly intervals, and thereafter about 2 - 10 mg/kg body with daily intervals or weekly intervals - or even monthly intervals depending on the monitored titer of serum C1 inactivator level which should not be lower than 15 mg%. A lower level than 15 mg% or minimal amount of C1 IA in the serum can be adjusted by i.v. infusion of C1 IA to counteract any possibility of angioneurotic edema. C1 IA (and C4) can be monitored frequently - for instance daily after injection(s) of recombinant anti C1 IA and CRP.

During treatment with C1 IA and CRP antiserum or antibodies, the patient is carefully monitored as described in the section "Cancer Therapy with Immune Serum According to the Invention", and especially important measured parameters are serum levels of C1 IA AND CRP and C4. By the successful treatment C1 IA AND CRP would be depleted but C1 IA serum level should preferably not be lower than 15 mg%. C4 level in serum can be lowered to less than normal lower range values, due to the consumption of the C4 in the complement reaction occurring in the patient.

During the period in which anti C1 IA anti CRP treatment is administered, simultaneous treatment with cytostatics is not preferred according to the present invention due to the immune-suppressing effect of cytostatics. On the other hand, if cytostatic treatment is used, the patient should preferably be treated with INA during e.g. the last fortnight of the cytostatic treatment and continuously for a period of 1 or 2 months upon ceased cytostatic treatment in order to normalize the exhausted immune system including re-establishment of normal values of T lymphocytes and B lymphocytes.

Cytostatics coupled to antihuman C1 IA AND CRP as selective carriers may, however, prove useful as they may be administered in much lower amounts than conventional cytostatics.

In order to monitor and adjust the immunoglobulin level of the patient, especially IgG must be assessed continuously, as the IgG concentration reflects both the human and the pig IgG content in serum. The purpose of such monitoring and adjustment is to secure that a sufficient amount of INA antiserum is administered for controlling and neutralizing the immune defense blocking and masking proteins, in particular C1 IA AND CRP, both in the humoral phase and on the cancer cell membranes. The necessary amount of anti C1 IA and anti CRP antiserum may, hence, depend upon both the total mass of the tumor as measured with conventional methods, x-ray, CT scanning, MR scanning, ultrasound, etc. and the activity (C1 IA AND CRP-producing ability) of the cancer cells.

The early therapy experience obtained so far where only mainly porcine anti C1 IA and anti CRP antibody in the form of INA, done in the seventies indicates that if one has access to the human or humanized monoclonal or rather recombinant anti human C1 IA and anti CRP may be able to prevent the tumor from further progressing, or after more than one, and may be several administered doses given over some weeks to months may induce minor responses, partial responses or even major responses of tumor. However, if no response is obtained within a certain period of time, weeks to months, it may be necessary to find other treatment modules for the patient such as other known and well tested monoclonal antibodies than those antibodies identified in this invenetion or chemotherapy routine treatment may be necessary.

Evidence of the fact that anti C1 IA AND CRP in INA serum is able to combine with cancer cells in vivo has been obtained:
Observations using anti C1 IA (and/or anti CRP) coupled to tracers such as 99technecium given intravenously may also indicate that an isotope labeled antibodies injected into a patient can be tracing metastases.

For the preparation of an antiserum or immunoglobulin product for diagnostical use in vivo, after isotope marking, e.g. with, preferably, technetium, which has a very short half life (4 hours) the vaccine used is preferably one which contains no C1 IA.

Preferred embodiments of a diagnostic kit are given below in the section "Diagnostic Kits". Diagnostic kits according to the invention contain an antihuman C1 IA AND CRP material, the reaction of which with human C1 IA AND CRP in immunological test methods is readily distinguishable from the reaction with human C1 IA.

### Reliability.

It would be preferable that both serum C1 IA and serum CRP presence are tested as well as serum C4 level are used as monitoring kit called TRIADE Kit for initial testing for the presence of cancer (carcinoma) or C1 IA monitored 1 to 2 times a week during i.v. treatment with recombinant human anti human C1 IA, and monthly follow up of the patient serum of CRP and C4.

Serum C1 IA, serum CRP and serum C4 may be detected by the test at such an early stage of a cancer that it is not possible to ascertain or verify the cancer by any other known method. This may account for test results which would, at a first glance, be considered "false positive". Experience has shown that even among serum samples from about 200 blood donors, 2% of the samples. If serum C1 IA, serum CRP are elevated but serum C4 is within normal range should lower the false positive percentage from 2% to less than 1%.

More specifically, the experience from the serum investigations so far performed using the immunodiffusion diagnostic kit are the following:
The following cancer types have been found to be rather consistently C1-IAC-positive: carcinomas such as carcinoma mammae (breast carcinoma), carcinoma corporis uteri (uterin carcinoma), cancer pulmonis (bronchogenic carcinoma), cancer pancreatis (pancreatic cancer), cancer ventriculi (stomach cancer), hepatoma (primary liver cancer), cancer coli (colorectal cancer), renal cancer (kidney cancer), including pelvic carcinoma (carcinoma pelvis renis), and carcinoma vesicae urinariae (urinary bladder carcinoma), sarcomas (a few osteosarcomas have been investigated and found negative). Testicular teratomas are strongly C1 IA AND CRP-positive. Melanomas: not all melanomas are found C1 IA AND CRP-positive.

A few investigated cases of Hodgkins were found C1 IA AND CRP-positive. Neoplasma in infants: nephroblastoma were found C1 IA AND CRP-positive. Neuroblastoma were found C1 IA AND CRP-positive. Tumors within the central nervous system, for example astrocytoma and glioblastoma should be tested because cell cultures from malignant brain tumors have been found to be C1 IA positive (Osther K, Højgaard K, Dybkjær E, Demonstration of a complement inactivator on cultured cells from human malignant brain tumours, Acta Neurol. Scandinav.(1974, 50:681-689). Malignant diseases in blood and lymphsystem: acute myeloblastic leucaemia (AML) and chronic myeloid leucaemia (CML) are found to be C1 IA AND CRP-positive, except during the remission phase. Myelomatosis is also found C1 IA AND CRP-positive. Acute lymphoblastic leucaemia (ALL) and chronic lymphoblastic leucaemia are found C1 IA AND CRP-positive in 5-20% of total verified cases; it is noted that most of the cases of ALL so far investigated were cases in remission, whereas a few cases of active stages of ALL investigated were found to be significantly C1 IA AND CRP-positive. One case of acute lymphosarcom leucaemia was found to be C1 IA AND CRP-negative. Lymphomas did not show significant C1 IA increase.

It is to be noted that one valuable feature of the diagnostic test is that it permits a meaningful monitoring of the patient during any attempt of therapy. Experience has shown that cytostatic treatment of several diseases will not lower the C1 IA AND CRP titre in the patient, if the tumors are not showing regression of tumor load by the treatment.

In other words, if the test shows that there is no decrease in serum C1 IA AND CRP during an attempt of therapy, this is an indication that the therapy is not effective. It can also be said, based upon the above explanation, that the cancer types and stages which are C1-IAC-positive must be presumed to be those which are susceptible to successful therapy with anti-Cl IA AND CRP antiserum.

Elevated serum C1 IA, elevated CRP, but decreased C4 and inflammatory autoimmune disorders and hepatitis!

It is interesting to note that an "autoimmune" disease such as LED (lupus erythromatosus disseminatus), and hepatitis, such as hepatitis B shows a positive C1 IA AND CRP-reaction in but have a low normal to significantly decreased serum complement C4 level. Low C4 is actually found in rheumatoid arthritis, psoriatic arthritis, various autoimmune disorders, hepatitis B, vascular collagen diseases. In investigations of the serum from about 200 patients from a neurological department, with a diversity of diseases ranging from cerebral thrombosis, cerebral infarcts, to diseases such as multiple sclerosis gave about 2% positives, just like the result found in the blood donor group. The blood donor group consists of 200 blood donors, and as mentioned above 2%. C1 IA AND CRP-positive serum samples and 10 doubtfully positive serum samples were found. The sera from some of these C1 IA AND CRP-positive blood donors have been re-examined after some weeks, and at the re-examination, some of the previously positive sera from blood donors were later found C1 IA AND CRP-negative. Sera from about 50 patients with different types of allergic diseases ranging from asthma and exzema to urticarial rash (except Quincke type) were found C1 IA AND CRP-negative.

### From 760'

C1 IA, and CRP coating cancer cells

### Technical Background

As described previously in US provisional patent application 62/388,720 with priority date February 5, 2016 (02.05.2016) and furthermore, in provisional patent application 62/495,203 with priority date September 7, 2016 (09.07.16) combined with the more recent data reveals a significant picture of the concept what happens when a de-blocking treatment of the innate immune system takes place by neutralizing the inhibitory effect of the C1 inhibitor (C1IA) or any other factor that may interfere or neutralize the inhibitory effect of this protein, for instance with aprotinin or other factor(s) as for instance compounds that would have a neuraminidase effect on C1 IA by treating the cancer with anti C1 IA such as antihuman anti C1 inhibitor (anti C1IA) present on the cancer cells, such as carcinoma cells, and malignant brain tumors such as malignant glioma cells in mammals as for instance rats or human malignant glioma - also called glioblastoma or glioblastoma multiforme, allowing activation of the inate immune system by inducing complement cascade.

The present disclosure also describes, as part of another "blocking", masking protein, or a complement interfering and in some cases is co-localized with complement, called C-reactive protein (CRP) towards which de-blocking treatment encompasses the important addition of neutralization of CRP on the cancer cell, which according to our findings as for instance shown in the examples that both rat gliomas, such as rat NS-1 glioma and human glioma/glioblastoma. These cancer cells show a significant coating with CRP as evidenced in the examples presented herein in this provisional patent application. The treatment of cancer cells coated with CRP is among other things done by using antihuman antibodies against CRP or any peptide or nanopeptide or chemical compounds that may eliminate the CRP on the cancer cell the use of an additional protein (a pentameric protein) called C-Reactive protein or CRP identified and documented by showing significant coating using anti CRP antibody sandwich methods also showed coating of the same malignant cancer cells as found being coated with C1 IA such as e.g., rat glioma cells and human glioblastoma cells.

The treatment consists of un-coating certain cancer cells, such as for instance having been identified by immune staining of proteins such as for instance malignant rat gliomas NS1 and PG2, as well as immune staining of these proteins in cancer cells such as human malignant glioma/glioblastoma or glioblastomas as documented in the examples of this invention, which clearly indicates that that these proteins can be found coating both the rat glioma cells and human glioblastomas, and which has previously been described to be present on certain carcinoma cells.

### Brief Description

The present disclosure relates to a combination of a kit for diagnostics and a kit for treatment of human cancer such as carcinomas, and brain tumors as well as certain other malignant solid cancers where it can be found that C1 IA and CRP, to processes for its isolation and characterization, to diagnostic test methods and materials based on the principle of demonstrating the presence of CRP, to antibodies and antisera with specificity against C1 IA and CRP, to the preparation of such antibodies and compositions containing same, to matrix-immobilized antibodies, and to the therapy of human cancer using for instance monoclonal and/or recombinant antisera or antibodies with specificity against C1 IA and CRP.

Important aspects of the present invention are based upon the principle of utilizing antibodies directed specifically against blocking and masking proteins, in particular C1 IA AND CRP, associated with human cancer diseases and present on the membranes of cancer cells. According to the invention, this principle is utilized for treatment of cancer in vivo and for extracorporal treatment of cancer patient serum.

C1 IA AND CRP is a human cancer associated protein which may be isolated from body fluids of cancer patients, including serum, and from human malignant cancer cells, also from cell cultures. Repeated culturing and harvesting cycles of cell cultures have been performed and C1 IA and CRP isolated from the culturing media, indicating that the cancer cells themselves are able to produce C1 IA and CRP.

C1 IA AND CRP has been found to possess biological properties similar to the properties of human complement component C1 inactivator (also termed "C1 esterase inhibitor"), which is an .alpha..sub.2 neuramino glycoprotein found in the body fluids of various species, including man (Pensky et al., J.Biol.Chem., 236, 1674, 1961, Ratnoff et al., J. Exp. Med. 129. 315, Pensky et al., Science 163, 698, 1969, Nagaki et al., Int.Arch.Allergy 46, 935, 1974), but to be protein-chemically non-identical with human complement component C1 inactivator.

In the following, human complement component C1 inactivator (C1 esterase inhibitor) will be termed "C1 IA", whereas the term "C1 inactivator", used in various contexts, shall designate the group consisting of C1 IA and CRP.

C1 IA & CRP has been found to possess biological properties similar to the properties of C1 IA, including the inhibitory effect on the initial human complement component C1 activation of C4 and C2 (i.e., inhibition of C1 esterase hydrolyzing effect), the inactivation of plasmin, and the lack of effect upon the clotting time of plasma (i.e., this lack is common to C1 IA and which cannot be excluded, may be also common to CRP). In accordance with the principle upon which the present invention is based, it is believed that these inhibitory effects of C1 IA & CRP play an important role in the cancer cell's defense against destruction by the human immune system.

In the literature, it has been described that a C1 IA-like protein is present on the membranes of human cancer cells (e.g., Osther, K., Hojgaard, K., and Dybkjaer E., Acta neurol. Scand, 1974 50, 681, Osther, K., the Lancet, Mar. 2, 1974, p. 359, Osther, K., Linnemann, R., Acta path. microbiol. scand. 1973, 81, p. 365).

According to the present disclosure, said C1 IA present on human cancer and as indicated above, it has been found to be a novel with C1 IA called C1 IA and CRP. Important new developments are based upon these findings.

### Anti C1 IA and Anti CRP Treatment of certain types of cancer

For the treatment of cancer cells in mammals (including humans) biomacromolecules engineering for research and for therapeutic use is one of the integral parts of the present innovation. Human or humanized antibody production using phage display technology and a wide range of antibody gene engineering, affinity maturation and humanization services can be provided by Creative Biolabs, (Creative Biolabs., USA, 45-1 Ramsey Road, Shirley, NY 11967; Europe, Ringstrasse 4, 64401 Gross-Bieberau, Germany). This company is well-recognized in manufacturing scFv/Fab and full-size IgG antibodies. In addition, the same company also gives OEM services for bulk scale antibody manufacturing, including bacterial production of scFv, diabody, tandem scFv, miniantibody and Fab, and mammalian cell expression of minibody, chimeric IgG and IgG, which also are available in the industry of producing antibodies, which includes mouse and rat monoclonal antibody production using hybridoma technology.

The invention will now be described in further details in the following non-limiting examples.

### Examples

### Example 1: PREPARATION OF C1 IA AND CRP.

### Obtainment of human cancer cells for culturing.

Human cancer cells may be obtained from pleural or ascites fluid from patients suffering from cancer with metastases to pleura and/or ascites. The pleural/ascites fluid should be handled under sterile conditions, and infectious pleural/ascites fluids should be excluded. Separation of the cells from the fluid may be obtained by centrifugations at 1000 rpm for 10 minutes at room temperature. The precipitate contains the cells. The supernatant is discanted and stored at -20°C for later isolation of immunogenically valuable proteins therefrom as described in later sections.

Another source are solid human tumors removed from cancer patients by operation, for example, malignant brain tumors from patients suffering from primary malignant brain tumors. The cancer cells may be obtained from such tumors by trypsination. For example, the brain samples are washed several times in Eagle minimum essential medium (MEM) whereafter the tissue is cut into 1-2 mm cubes and resuspended in the medium after further washings and centrifugations, e.g., for 10 minutes at 900 rpm, trypsin solution may be added, whereafter incubation for e.g. 30 minutes at 25°C is carried out, and the cell suspension may be filtered through sterile gauze admixed with a minimum of Eagle MEM with 15% bovine serum added, and centrifugated as mentioned above. The precipitate contains the cells, and the supernatant is decanted.

Each of the above precipitates may be used individually in the following procedure, or the precipitates may be pooled. Furthermore, human cancer cells obtained from other sources may be treated analogously and used per se or pooled with cancer cells obtained as described above.

### Culturing of the cancer cells.

Cancer cells obtained as described above are explanted into 1000 ml Roux flasks containing Eagle MEM enriched with added glutamine (about 294 mg per liter) and containing 15% inactivated foetal calf serum and are incubated in incubator without CO₂ addition. For parallel test cultures, about 0.5 ml of the cell suspension is explanted directly on slides in a Leighton tube in order to examine a presence of C1 IA AND CRP on the cell membrane by immunofluorescence cytophotometri (Osther et al., Acta.path.microbiol. scand.B 81, 365, 1973).

When the test cultures show the presence of C1 IA AND CRP, the main culture is used as production culture: The medium is removed, the cell structure is washed with PBS buffer, pH 7.3, and RPMI synthetic amino acid medium from Flow, Scotland, enriched with glutamine (about 294 mg/liter) but without any other admixture, is introduced, and incubation is performed for 3 days at 37.degree. C., whereafter the medium is harvested under sterile conditions and centrifugated at 1000 rpm for 15 minutes at room temperature, whereafter the supernatant is stored in closed bottles at -20°C until isolation of C1 IA AND CRP is carried out. The cancer cell culture is further cultivated in the above-mentioned Eagle MEM modified medium, and after 3 days of incubation, the medium is again exchanged with glutamine-enriched RPMI medium, which is again harvested after 3 days of incubation and treated and frozen as described above for later isolation. In this manner, alternating culturing in Eagle MEM modified medium and RPMI glutamine-enriched medium and harvesting of the latter is carried out as long as the cancer cell culture is productive. In case of specially good growth (far over approximately 2 times 10⁵ cells per Roux flask), explanting to further Roux culturing flasks is performed, and the resulting new cell cultures are also used for production in the same manner as described above. Throughout the procedure, the C1 IA AND CRP productivity of the culture cells is monitored by means of the test cultures in the Leighton tubes, and to the culturing media, penicillin and streptomycin are added is appropriate concentrations before use.

### Isolation of C1 IA AND CRP.

The RPMI glutamine-enriched medium harvested from cancer cell cultures as described above is salted out to precipitate contaminating proteins, suitably to 40% saturation with saturated (NH₄)₂SO₄ at 0°C with agitation. The medium so treated is centrifugated at 4000 rpm for 15 minutes at room temperature. Also other centrifugation procedures may be used, but the one stated has been found to give excellent results. The precipitate is discarded, and the supernatant is dialyzed for about 48 hours at 4 °C against numerous changes of distilled water and thereafter centrifugated at 3500 rpm at room temperature for 15 minutes. Also other centrifugation procedures are suitable, e.g. using a cooled centrifuge. Especially good purification is obtained in a cooled preparative ultracentrifuge.

### Purification by chromatography methods

In the further working up of C1 IA and CRP from the clear supernatant, column chromatography absorption is the first stage. A preferred anion exchanger resin for this purpose is Dowex 2 x 8, mesh 200-400, but also other ion exchanger materials may be used. The ion exchanger resin is pretreated by boiling in water bath for 2 hours, 5 x 2 hours shift with 0.1 M HCI and thereafter equilibration by numerous shifts with agitation with Tris buffer 0.06 M, pH 7.3 until pH is stabilized about 7.3. Also other Tris buffers and phosphate buffers of different pH and ionic strength have also been used, but the above-mentioned buffer has been found optimal.

The above-mentioned clear supernatant is applied to the Dowex 2 x 8 anion exchanger resin column (K 50/100 with adaptors with a flow of the above-mentioned Tris buffer, pH 7.3, the flow rate being 100 ml per hour at room temperature. The best yield of C1 IA AND CRP is obtained using 160 ml of RPMI medium per 1500 ml of column material. The effluent from the column passes through a flow-through micro cuvette of a spectrophotometer which graphically records the optical density at 580 nm of the effluent (an Isco spectrophotometer was used) and is collected in 10 ml fractions in a fraction collector. In this and the following fractionations, eluted proteins detected as optical density peaks in the spectrophotometer are checked for C1 IA AND CRP by rocket immunoelectrophoresis using rabbit antihuman C1 IA, pig antihuman C1 IA AND CRP antihuman (absorbed) C1 IA AND CRP (prepared as described further below) in the gel and for contaminating proteins by Freeman crossed immunoelectrophoresis using antihuman whole protein serum in the gel and Grabar immunoelectrophoresis using the two above-mentioned types of antisera. The contaminating trace of other proteins are suitably checked for such as the presence of orosomucoid, transferrin, .alpha₂ HS glycoprotein, inter-alpha.-trypsin inhibitor, prealbumin, and albumin.

The main part of C1 IA AND CRP is absorbed in the ion exchanger. By repeated passage through the ion exchanger in Tris buffer, the remainder may be absorbed.

Most of the contaminating proteins appear in a first major elution peak in the elution with Tris buffer. A typical spectrophotometrical graph of this elution is shown in Figure 5. After a suitable number of fractions following the appearance of the peak, a straight ascending concentration of NaCl until an end point of 0.09 M NaCl is added to the column. The above-mentioned "suitable" number of fractions is a number of fractions which will secure proper separation of the first peak and the subsequently eluted protein. Also other models of additional salt concentration, including stepwise increase, may be used, but with no additional advantage

C1 IA AND CRP is liberated from the ion exchanger at the end point at about 0.09 M NaCl (as shown by a minor peak in the spectrophotometrical graph, vide Figure 5), and the fractions containing C1 IA AND CRP are pooled and dialyzed for about 24 hours at 4°C against distilled water. Thereafter, the dialyzed product is lyophilized (shell lyophilization using vacuum and gentle external heating at 40°C was found to be a suitable procedure).

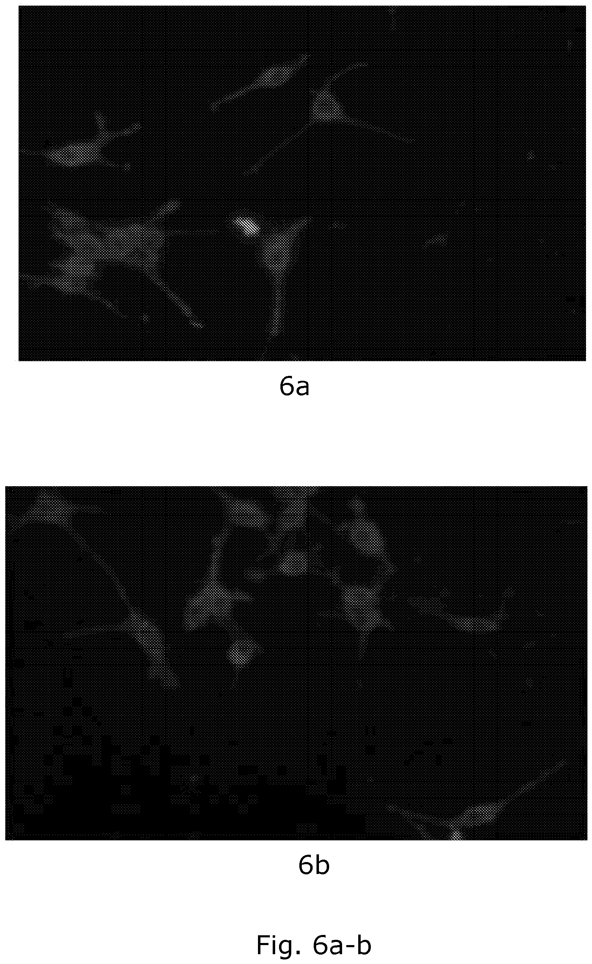

The lyophilisate is resuspended in 0.06 M Tris buffer, pH 8.6, and applied on a Sephadex® G75 Superfine gel filtration dextrane column such as a Pharmacia K 15/50 column, using the Tris buffer, pH 8.6, as effluent buffer. The flow rate is suitably about 10 ml per hour. The effluent was monitored with the spectrophotometer and examined by immunoelectrophoresis techniques as described above. A typical spectrophotometrical recording of the optical density of the effluent is shown in Figure 6, in which the first peak represents C1 IA AND CRP. The fractions rich in C1 IA AND CRP are collected, dialyzed exhaustively against distilled water (48 hours) and lyophilized as described above. In the following, this product is designated "C1 IA AND CRP (Sephadex®G75 Superf.)" or "C1 IA AND CRPsemipurified ". According to immunoelectrophoretical estimation, this product is pure and non-aggregated.

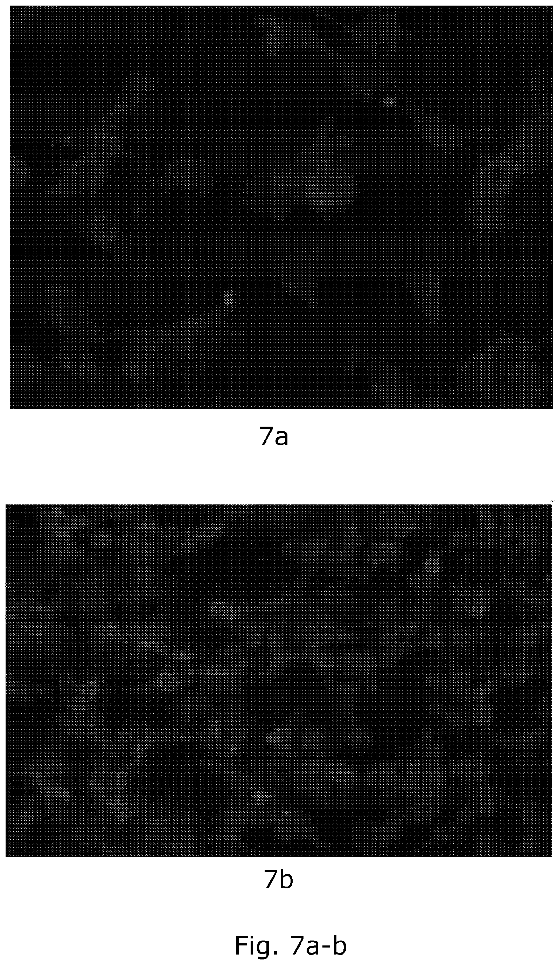

In an alternative purification procedure, the above-mentioned lyophilisate of the dialyzed C1 IA AND CRP-containing fractions from the Dowex 2 x 8 ion exchanger is resuspended in 0.06 M Tris buffer, pH 8.6, and applied on a Whatman DE-52 cellulose anion exchanger column (e.g. K 25/60) with adaptors. The anion exchanger is eluted with a straight ascending gradient of sodium chloride from 0 to 0.15 M NaCl Repeated passages through the Whatman ion exchanger were carried out until optimal purification had been obtained as ascertained by the above methods. The C1 IA AND CRP purified in this manner is aggregated to some extent, as ascertained by immunoelectrophoresis, vide Figure 7.

For use as immunogenic agent in a vaccine which is suitable for the first vaccination of suitable host animals, the C1 IA AND CRP-rich fractions from the Whatman ion exchanger were pooled with the fractions from the Sephadex® G75 Superfine column, and the pooled fractions were dialyzed and thereafter lyophilized as described above. In the following, this combined product is designated "C1 IA AND CRP.sub.aggregated protein + C1 IA AND CRP Sephadex® G75 Superf.". This product is ready for resuspension for possible admixture with other proteins as described below and/or admixture with C1 IA alone to give a specific antiserum with optimal function.

### Example 2: CHARACTERIZATION OF C1 IA AND CRP.

### Antisera

Rabbit antihuman C1 IA (antibody content 0.7 mg/ml), rabbit antihuman C4 (antibody content 1.0 mg/ml), rabbit antihuman C3 (antibody content 1.2 mg/ml), rabbit antihuman C3 activator (antibody content 0.5 mg/ml), rabbit antihuman .alpha ₂HS glycoprotein (antibody content 0.35 mg/ml), rabbit antihuman inter-.alpha.-trypsin inhibitor (antibody content 1.0 mg/ml), rabbit antihuman orosomucoid (antibody content 0.9 mg/ml), rabbit antihuman transferrin (antibody content 2.5 mg/ml), rabbit antihuman albumin (antibody content 1.1 mg/ml), rabbit antihuman prealbumin (antibody content 0.25 mg/ml), rabbit antihuman .alpha₁ foetoprotein (0.2 mg/ml), rabbit antihuman plasminogen (antibody content 0.25 mg/ml) were from Behringwerke. Rabbit antihuman IgG (antibody content 0.4 mg/ml), rabbit antihuman IgM (antibody content 0.4 mg/ml), and rabbit antihuman whole serum were from Dakopatts.

### Experimental procedures

### Immunoelectrophoresis

Quantitative electrophoresis in antibody-containing 1% agarose (1.5 mm thick) was run with 2.5 V/cm for 18-20 hours at 20°C (Laurell C. B., Quantitative Estimation of Proteins by Electrophoresis in Agarose Gel Containing Antibodies. Ann. Biochem. 15:45-52, 1966). Qualitative immunoelectrophoresis was run according to the method of Grabar & Scheidegger. Antigen-antibody crossed electrophoresis was run a.m. Freeman (Clarke, H. C. & Freeman, T. A Quantitative Immunoelectrophoresis Method (Laurell Electrophoresis) pp. 503-509 in Protides of the Biological Fluids. Vol. 14 Elsevier, Amsterdam, 1966., Laurell, C. B. Antigen-antibody Crossed Electrophoresis. Ann. Biochem. 10:358-361, 1965). Initial separation by electrophoresis in agarose with 10 V/cm for 1.5 hours at 20 °C. After turning the electric field 90°C electrophoresis was run into antibody-containing agarose gel with 2.5 V/cm for 18 to 20 hours at 20°C.

### Immunodiffusion

Was run in 1% agarose a.m. Ouchterlony gel diffusion technique (Ouchterlony, O. Progr. Allergy, 6:30, 1962). The diffusion was carried out at room temperature for 3 days.

### Tandem-crossed immunoelectrophoresis

Was run a.m. Kroll (Kroll, J. Tandem-crossed Immunoelectrophoresis, in A Manual of Quantitative Immunoelectrophoresis (Eds. Axelsen, NH, Kroll, J., Weeke, B.) Universitetsforlaget, Oslo, 1973, pp. 57-59). The principle is an initial electrophoresis of two antigen samples in the same run separation in agarose gel with 10 V/cm for 1.5 hours. After turning the electric field 90.degree., electrophoresis is run into antibody containing gel with 2.5 V/cm for 18-20 hours at 20°C.

### Immunological properties of C1 IA AND CRP

### Grabar-Scheidegger immunoelectrophoresis.

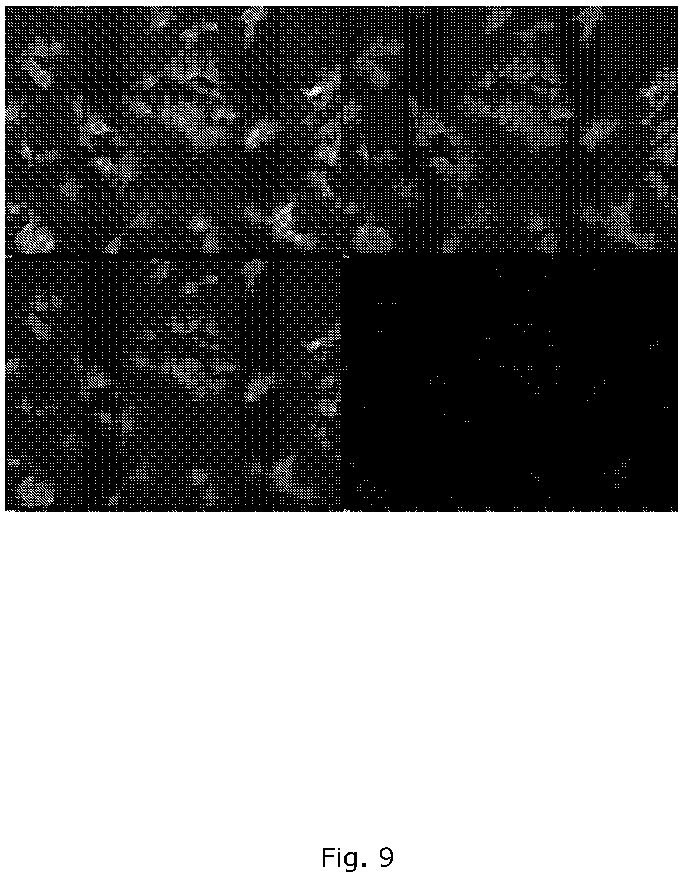

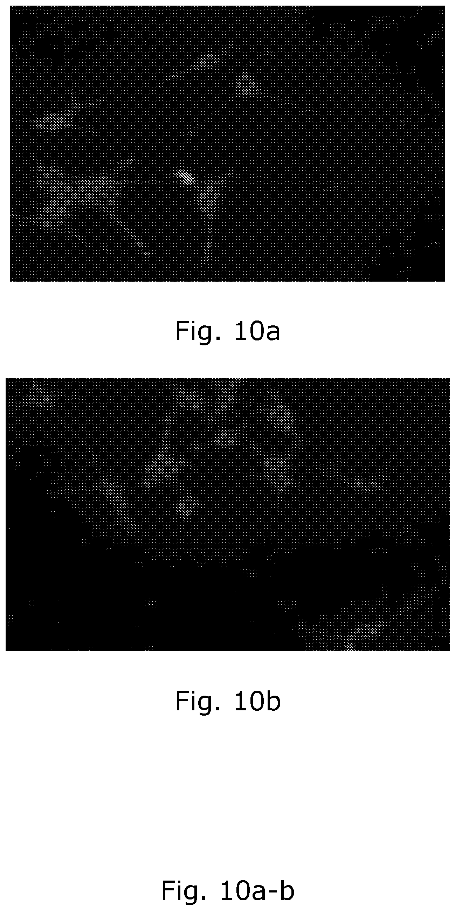

In Grabar-Scheidegger immunoelectrophoresis against rabbit antihuman C1 IA, a gull wing precipitate results from C1 IA, whereas C1 IA AND CRP does not show gull wing precipitation. (cf. Figure 9 of US 4,132,769). (The "gull wing" is described by Hirschfeld (1960)). The C1 IA AND CRP precipitate extends into the beta zone on a Grabar immune electrophoresis when immunoelectrophoresis is run in the presence of calcium lactate, vide Figure 10 of US 4,132,769.

### Laurell rocket immunoelectrophoresis.

In Laurell rocket immunoelectrophoresis against rabbit antihuman C1 IA, a slight difference between C1 IA and C1 IA AND CRP is found in the configuration of the rockets, which are somewhat more blunt for C1 IA AND CRP than for C1 IA, approximately as seen in Figure 7.

### Tandem-crossed immunoelectrophoresis a.m. Kroll.

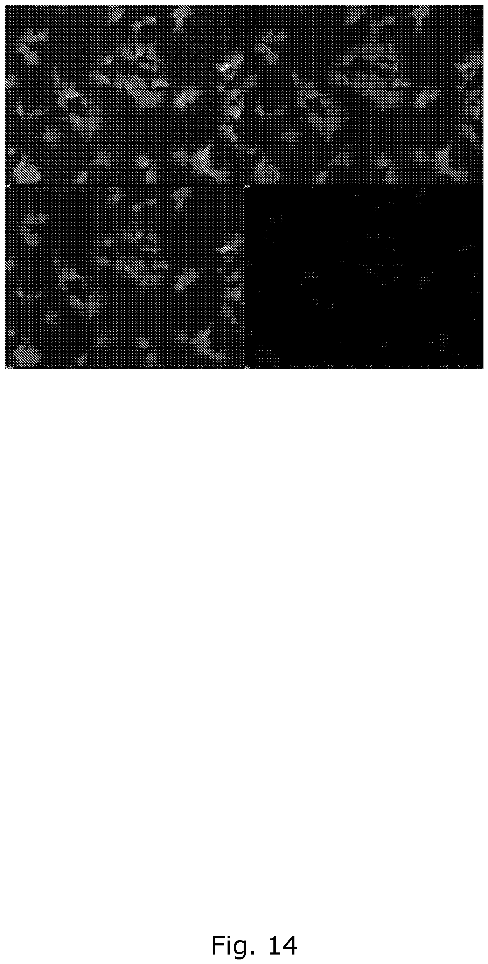

Using rabbit antihuman C1 IA, full identity was found between C1 IA and C1 IA AND CRP in tandem-crossed immunoelectrophoresis; cf. Figure 14 of US 4,132,769.

### Crossed Freeman immunoelectrophoresis.

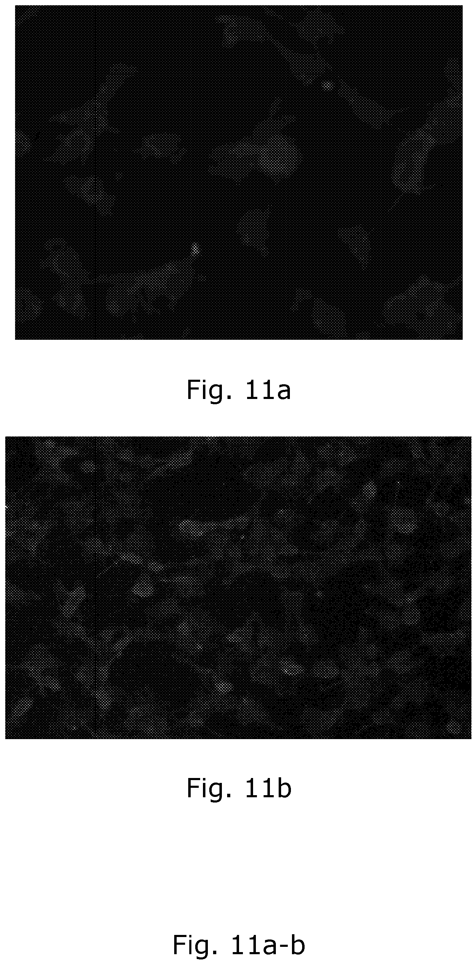

Using rabbit antihuman whole protein serum, purified C1 IA and purified C1 IA AND CRP give identical precipitates in crossed Freeman immunoelectrophoresis (see Figures 11, 12 and 14 of US 4,132,769).

On Grabar-Scheidegger immunoelectrophoresis against rabbit antihuman C1 IA (vide Figure 10 of US 4,132,769) it was shown that C1 IA AND CRP treated with neuraminidase as described above gives no precipitate.

### Ouchterlony immunodiffusion against oligospecific antiserum.

In Ouchterlony immunodiffusion against antiserum produced on pigs of "Dansk Landrace", which host animals have been vaccinated with vaccines containing C1 IA AND CRP, e.g. the type 1 or 2 vaccine described in the section "Vaccination of Host Animals", or against antiserum from any other animal which is able to distinguish between C1 IA and CRP from C1 IA and which has been vaccinated with vaccines containing C1 IA and CRP and C1 IA in effective immunogenic amounts, C1 IA AND CRP gives a precipitate distinguishable from the C1 IA precipitation. For example, serum from cancer patients will give two precipitates distinguishable from each other, whereas serum from healthy donors (or patients with non-malignant diseases) will give only one precipitate, this being the C1 IA precipitate. One of the precipitates from cancer patient serum will correspond to the C1 IA precipitate from the cancer patient serum. An immunodiffusion of this type is shown in Figure 3. In the two central holes, oligospecific antiserum was applied, and in the circumferential holes, serum from healthy donors ("DONOR") and various patients were applied. Serum from each sample was applied in each of two opposite holes. In the present case, the antiserum used was one obtained from a pig vaccinated with a type 1 vaccine (vide the section "Vaccination of Host Animals").

From Figure 3, it will be seen that a strong interior precipitate was formed which was identical for donor and for all the patients. This is the precipitate of C1 IA. However, patient 3 (verified cancer) serum gave an extra precipitate easily distinguishable from the C1 IA precipitate. This is the C1 IA AND CRP precipitate. Also, all serum samples gave a common weaker exterior precipitate, this being the orosomucoid precipitate.

It is evident that the precipitate formed by C1 IA AND CRP is easily distinguishable from the C1 IA precipitate. While the immunodiffusion shown in FIG. 3 were actually experiments made for diagnostic purposes, analogous precipitation patterns have, of course, been demonstrated with mixtures of pure C1 IA and C1 IA AND CRP, and it will be understood that the Ouchterlony technique is only one example of the immunological identification or assay methods (Laurell, Mancini, etc. etc.) which give distinction between C1 IA and C1 IA AND CRP when oligospecific antiserum is used.

### Other properties of C1 IA AND CRP.

Estimation of the molecular weight of C1 IA AND CRP by the gel filtration technique.

A Sephadex® G200 gel filtration column (Pharmacia K 25/50) was equilibrated with Tris buffer, pH 8.6. A solution of C1 IA AND CRP Sephadex® G75 Superf., purified human albumin (chromatographed and checked for purity by immunoelectrophoresis), hemoglobin (prepared by hemolysis of erythrocytes and purified, checked for purity), and purified IgG (molecular weight 130,000-150,000) was applied on the column, and the effluent was monitored spectrophotometrically, the identity of the peaks being ascertained by immunoelectrophoresis. The first peak consisted of IgG, and before it had fully decreased, the second peak (C1 IA AND CRP) began to appear. When the C1 IA AND CRP peak had passed, a number of substantially empty fractions were noted, whereafter the next peak appeared which was the albumin. Almost immediately after the albumin peak, the hemoglobin peak appeared.

Based upon the spectrophotometrical graph, it is evident that the molecular weight of C1 IA AND CRP must be greater than the molecular weight of the albumin which is 60,000, and just below the molecular weight of the IgG. As the molecular weight of IgG cannot be stated more accurately than 130,000-150,000, a similar uncertainty applies to the estimation of the C1 IA AND CRP molecular weight which, based on the position of the C1 IA AND CRP peak, is 110,000-130,000.

### Example 3: DEMONSTRATION OF THE CANCER ASSOCIATION OF C1 IA AND CRP.

Pleural or ascites exsudates from patients suffering from far advanced carcinomas were used as sources for carcinoma cell culturing. Contaminated fluids were excluded. The cell cultures grown in Roux flasks and in Falcon plast flasks were distributed for clonal growth or more diffuse growing. The morphology of the clones showed cell and nuclei polymorphism, one big or several nucleoli, more cells had several nuclei. Many mitosis were seen. Of these mitosis several were atypical. The attachment of the cells to plast and glass were sufficient.

27 Of the 37 human carcinoma cultures were subcultured from 1 to 4 times. A mean of 1.8 subcultures of 27 cultures. The cell ines were accordingly cultured and used for production of C1 IA AND CRP for a period ranging from 2 to 5 months. 10 Carcinoma cell cultures were not subcultured, and consequently used for production of C1 IA AND CRP for about 2 months.

The cell-free C1 IA AND CRP was harvested about once a week, alternatingly released into MEM medium containing 15% inactivated foetal calf serum and into RPMI amino acid medium. Consequently, it was possible to harvest 4 times a month from the same cell culture. In a total, 235 isolations of C1 IA AND CRP were performed. Of these isolations, 37 were performed directly from the cell-free pleural and ascites fluids.

101 Isolations were performed from MEM medium containing 15% inactivated foetal calf serum and 97 isolations were performed from RPMI medium. On each carcinoma cell culture, C1 IA AND CRP was isolated ranging from 2 to 14 times. A mean of 5.4 isolations per cell culture. During the growth period in RPMI medium, the cell growth was depressed, while during the period in MEM medium containing inactivated foetal calf serum the cells regained their normal growth cycle in vitro. In several cell cultures it was necessary to let the cell culture develop more sufficiently before changing to RPMI medium again. The amount of cells in a culture was optimally held at about 2 x 10⁵ cells per Roux flask. The cell cultures were constantly checked for development of the individual cell types. The cell types from pleura and ascites consisted of carcinoma cells, fibroblasts and mesothelial cells. In flasks with clonal growth of the carcinoma cells it was not difficult to establish the approximate quantity of these cells. But in rather diffusely growing cell cultures it was more difficult to establish the approximate quantity of carcinoma cells. The amount of cells producing C1 IA AND CRP was related to the amount of cells coated with C1 IA AND CRP as found by cytophotometry, from idem cell culture lines.

A total of 37 patients were used as donors. The age of the patients ranged from 33 to 80 years. The patient group consisted of 34 females and 3 males. The 37 patients were attending hospital for removal of pleural and ascites fluids. All patients had far advanced carcinomas of various origin. All patients had previously during their disease been treated with radiation from 1 to 3 series. All patients had been treated with cytostatics and cortisone or prednisone. 25 of the patients were during the donor period treated with corisone or prednisone.

Of the carcinoma primary cell lines, an average of 51.3% of the cells showed specific immunofluorescence with anti C1 IA-FITC as measured by cytophotometry. The average distributions of the immunofluorescence measurements of carcinoma cell cultures is demonstrated in Figure 3. Values scored below 40 w.u. are defined as non-specific signals.

An average of 58.4% of the cells from the subcultured cell lines show specific immunofluorescence. When comparing the primarily cultured carcinoma cells with the corresponding subcultured cell lines, the amount of cells exhibiting C1 IA AND CRP coat varies significantly.

In some of the subcultured cell lines the amount of C1 IA AND CRP coat cells is higher, when compared with their origin culture.

Control specific immunofluorescence as performed with presaturation of the cell culture with non-labelled anti C1 IA, followed by incubation with anti C1 IA-FITC and neutralized anti C1 IA-FITC (with purified C1 IA), did not show values exceeding the border line and thus, not a single cell was found showing specific immunofluorescence. The control cell cultures (non-malignant cells) measured by cytophotometry did not show any C1 inactivator coat. The measured values of the cells scored, were all under 40 w.u. The values of the control cell cultures are distributed roughly as the values for control of immunofluorescence specificity. The control cell cultures showed a reliability for at least 63% and the malignant cell cultures a reliability for at least 90%. The significant difference is less than 0.002 (Fischer's exact test).

Control MEM mediae containing 15% inactivated foetal calf serum and control RPMI mediae (not used for cell culturing) were subjected to immunoelectrophoresis before and after a Dowex elution. The investigated mediae did not reveal any precipitation lines against antihuman C1 IA antiserum from rabbit. The only precipitation lines detected were found against antihuman albumin antiserum from rabbit. These control experiments gave accordingly evidence to the fact that the mediae used for cell culturing did not contain any proteins reacting with anti C1 IA before being used for cell culturing.

MEM mediae and RPMI mediae harvested from non-malignant cell lines, subjected to immunoelectrophoresis did not reveal any precipitation lines against antihuman C1 IA antiserum from rabbit. Using Ouchterlony immunodiffusion no precipitation lines were detected against antihuman C1 IA antiserum from rabbit.

Accordingly, no detectable C1 IA was released from control non-malignant cell cultures into the mediae, when jusing immunoelectrophoretic and immunodiffusion methods for detection.

### Example 4: PURIFICATION OF C1 IA AND CRP/C1 IA FROM PLEURAL/ASCITES FLUID FROM CANCER PATIENTS.

Pleural/ascites fluid from patients suffering from cancer with metastases to pleura and/or ascites is centrifuged as described in the above section "Preparation of C1 IA AND CRP", and the precipitate is explanted to cancer cell cultures as described above. The supernatant, possibly after storage at -20°C (vide above), is allowed to clot spontaneously for 2 hours at room temperature, and thereafter is allowed to stand overnight in refrigerator for complete spontaneous clotting. The supernatant is again centrifuged to remove fibrine and frozen at -20°C until the further purification takes place.

2 ml of the pleural/ascites fluid thus treated are admixed with 2.5 g of DEAE Sephadex ® A50 anion exchanger which had been preheated to 100.degree. C. for 30 minutes and thereafter cooled to room temperature. The mixture is stirred at 8°C for one hour. Thereafter, the mixture is placed in a column and washed with 5 times. 40 ml 0.15 M NaCl with suction from the column bottom. The C1 IA AND CRP/C1 IA protein material is absorbed in the ion exchanger. The ion exchanger material in the column is washed with 200 ml NaCl, 2 M, and trisodium citrate, 0.01 M, pH 7.0.

The latter eluate containing C1 IA AND CRP/C1 IA was admixed with saturated (NH₄)₂SO₄ (pH adjusted to 7.0 with NaOH) until 50% saturation at 8°C in the course of one hour. Thereafter, centrifugation (10 minutes at 3000 rpm at room temperature) was performed, and to the supernatant (NH₄)₂SO₄ (as above, pH 7.0) was added until 65% saturation at 8.degree. C. in the course of one hour. Again, centrifugation (10 minutes at 3000 rpm at room temperature) was performed, and the precipitate containing C1 IA AND CRP/C1 IA was dialyzed overnight against distilled water at 4-6°C and thereafter redissolved in 0.15 M NaCl.

The solution was lyophilized, either by shell lyophilizing or by lyophilizing in vacuum with external heating as described in the section termed "Preparation of C1 IA AND CRP".

Increased stabilization of monoclonal antibodies and of recombinant antibodies to proteins described herein and in recombinant proteins made for other purposes.

For the purpose of stabilizing monoclonal antibodies, recombinant antibodies against C1 inactivator, C Reactive Protein (CRP), and C4, one can add a certain amount of non reducing types of sugar components such as non-reducing disaccharides , which could be any non-reducing disaccharides such as e.g., trehalose, or disaccharides consisting of sugar-alcohol combinations such as e.g., isomalt. Furthermore, larger molecule such as Dextran can be used, because it is also a noreducicing sugar combination.

*Monoclonal antibodies such as monoclonal anti C1 IA, anti CRP, and anti C4 as IgM* An alternative to using monoclonal antibodies types IgG (e.g., IgG1, IgG3), it is within this invention to use monoclonal anti IgM antibodies against C1 IA, against CRP and against C4. They can also be mixed such as for instance mixing IgM anti C1 IA and IgM anti CRP, and possibly IgM anti C4.

Any of these combinations of IgM could be used in agglutination test methods for instance as marked or labeled with for instance fluorescein isothiocyanate (FITC) or another labeling method. When using the system which is used for Blood typing such as ELDON CARD (Eldon Biologicals A/S, Sandtoften 10, DK-2820 Gentofte, Denmark), it is within this invention possible to coat the "Eldon" card among other things with IgM anti C1 IA, and/or IgM anti CRP, and/or IgM anti C4 polyclonal or monoclonal antibodies. These IgM type monoclonal antibodies will due to their poly-binding to antigens, be capable of bridging more than one antigen together, and thereby most probably be identifiable on Eldon Card (and if necessary using FITC excitation light source), to see agglutination in whole blood or in serum, and thereby finding a borderline of visible agglutination where a pre-established (by validation) borderline between significant agglutination and non-significant agglutination can be evaluated, may be even with the use of miocroscope or alike to see small agglutinations, as for instance using FITC excitation light. It may be anticipated that blood samples from patients, suspected for having a malignant cancer, or even may be easier to identify, for instance in regards to certain leukemias, where the cells already may be coated with C1 IA, etc. so the agglutination would be easily visible.

### Immunogen used for antibody production.

### Type 1:

C1 IA AND CRP.sub.aggregated protein + C1 IA AND CRP Sephadex ® G75 Superf. is given as initial vaccination in a total amount of 20 mg, dissolved in 1 ml 0.15 M NaCl, made into water-in-oil emulsion with equal volume of Freund's complete adjuvant.

As booster dose, 20 mg of C1 IA AND CRP.sub.aggregated protein + C1 IA AND CRP Sephadex® G75 Superfine is admixed with 20 mg of C1 IA AND CRP/C1 IA DEAE Sephadex® A50 + 20 mg of C1 IA AND CRP/C1 IA, alpha₂ HS, Zn .alpha₂, orosom. The mixture is resuspended in 1 ml 0.15 M NaCl and made into water-in-oil emulsion with equal volume of Freund's complete adjuvant. This booster is suitable as first and second booster doses for pigs.

### Type 2:

C1 IA AND CRP (removed by Kurt Osther, 2.3.2017 protein + C1 IA AND CRP Sephadex® G75 Superf. is given as initial vaccination to production animals of mice used for use in the preparation for fusion with plasma cells for the production of monoclonal antiserum for diagnostic and for recombinant anti C1 IA and recombinant anti C Reactive Protein (anti rec. CRP) for therapeutical purposes.

### Future Treatment with anti C1 IA and CRP

There will only be used humanized monoclonal antibodies against C1 IA, against CRP and monoclonal against complement component C4.

For the treatment of cancer patients with anti C1 IA and anti CRP: There will only be used either mixed human or humanized monoclonal antibodies and preferably recombinant human anti human C1 IA and/or CRP.

### Example 5: CANCER THERAPY WITH IMMUNE SERUM.

In the following, three preliminary attempts to utilize de-blocking sera in short-time treatment of human cancer are reviewed. In addition to the treatment reported below, also other cancer patients have been treated and remission, i.e., a decrease in tumor mass, has been ascertained. However, the treatments here reported were the first ones in which a more complete specification of the effect of the treatment was possible.

### De-blocking immune sera used for the patients treated with pigs.

INA (Immune Neutralizing Antiserum) de-blocking sera were produced on pigs as described above. The pig serum contained antibody with a titer of 2 mg/ml against C1 IA AND CRP in total serum, estimated as described in the section "Vaccination of Host Animals". The means concentration of 1gG in the pig total serum was 1200 mg/100 ml, as estimated using antihuman 1gG. The antibody titer against C1 IA AND CRP pig serum was approximately 3 mg/ml. The sera were fractionated on a sterile Sephadex® G200 column using sodium phosphate buffer at physiological pH, and fractions containing immunoglobulin were isolated, dialyzed, and lyophilized. At the start of treatment, the lyophilized protein was resuspended in isotonic saline. The purified pig anti C1IA/CRP de-blocking serum was estimated to contain 100-1000 mg IgG/ml (by rocket immunoelectrophoresis using antihuman IgG).

### Routine investigations of patient during immunotherapy with INA.

Monitoring of the patient with ECG, pulse rate, external and internal temperature control.

Serum concentrations of immunoglobulins, complement component C4, and C1 inactivator (C1 IA + C1 IA AND CRP) were estimated consecutively before, during and after treatment. Serological tests were performed by rocket immunoelectrophoresis a.m. Laurell, C. B., Analyt. Biochem., 15, 45 (1966), using the following test sera: Rabbit antihuman C1 IA (antibody content 0.7 mg/ml), and rabbit antihuman C4 (antibody content 1.0 mg/ml), both from Behringwerke. Rabbit antihuman IgG (antibody content 0.4 mg/ml) and rabbit antihuman IgM (antibody content 0.4 mg/ml) from Dakopatts, Copenhagen. Immunoglobulin coat and secretion of the patient's lymphocytes were investigated consecutively in accordance with the following method.

Lymphocytes are separated from a citrate-containing blood sample from the patient by Isopague-Ficoll gradient centrifugation followed by washings in Hanck's stock solution with centrifugation after each washing. The resulting lymphocyte pool is divided in two portions. One portion is examined for presence of coat of immunoglobulins as described in Osther, K. and Dybkjaer, E., Scand. J. Haemat., 13, 24 (1974). The other portion is trypsinized using Trypure-PBS buffer for 2 minutes at 37.degree. C. The trypsine effect is stopped by repeated washings in Eagle MEM containing 15% inactivated foetal calf serum. Thereafter, the lymphocytes are checked for presence of immunoglobulin. When found negative, the lymphocytes are incubated in Hanck's stock solution at 4.degree. C. overnight. Thereafter, the lymphocytes are separated and then incubated in FITC-marked antihuman immunoglobulins. FITC (Fluorescein Isothiocyanate) was from BBL, Cockeysville, Md., USA. The antisera were conjugated with FITC a.m. Fothergill, J. E. (Properties of conjugated serum proteins. In Nairn, R. C., (ed.), Fluorescent Protein Tracing, pp. 5, 3rd ed. E. & S. Livingstone Ltd., Edinburgh and London (1969)). Immediately thereafter, the immunofluorescence of the lymphocytes is measured by cytophotometry as described in Osther, K. and Dybkjaer, E. (Scand. J. Haemat., 13, 24 (1974)).

See also Sandahl Christiansen, J, et al and Osther K. Acta Pathol. Microbial. Scand. Sect.C 84:313-318, 1978, and the figures related to patients in US 4,132,769.

### PROCEDURE OF TREATMENT.

Intracutaneous test with pig anti C1 IA/CRP de-blocking serum diluted 1:10 was set and reaction noticed, if any. The patient was pretreated with antihistamine in order to avoid an iatrogenic histamine release in the patient.

The pig anti C1 IA/CRP de-blocking serum, diluted in saline, was administered intravenously. Drop count and total dose were administered according to the condition of the patient.

### RESULTS

A basic introductory of the use of monitoring serum C1 IA and C4 level is exemplified a 2.5 year old child (male) with metastatic reticulosarcoma treated with chemotherapy, surgery, and "single dose of pig anti C1 IA/CRP de-blocking IgG.

### Patient No. 6 (see the patient history on the other patients below)

An early example of monitoring C1 IA and C4 during de-blocking treatment was done on a 2.5 year old child with progressing metastatic reticulosarcoma with infiltration of testicles and bone marrow.

Four months prior to the "one dose" de-blocking treatment with pig IgG the patient recieved 3.0 mg Vincristine weekly with early partly remission.

The Vincristine treatment had to be stopped due to the development of ataxia and cerebral edema. This interruption of chemotherapy was followed by recurring fast progressing growth of the reticulosarcoma to his testicles and his bone marrow.

During the coming 4 weeks a large orange size tumor in his right testicle required surgery. The surgeon did not remove the other testicle which contained palapable tumor mass. Due to the patient's condition having reciculosarcoma in the resection zone, small papable tumor in the other testicle and bone metastases, it was decided to administer 500 mg pig anti C1 IA/CRP IgG given I.V. as a de-blocking attempt.

The patient was only given one I.V. infusion with ∼500 mg of pig anti C1 IA/CRP purified IgG with no any adverse reaction. The serum concentration of C1 IA decreased from 85 mg% to 50 mg% after an immediate peak in serum C1IA level of 90 mg%. The C4 level was found to decrease from 60 mg% to approximately 20 mg% (Normal standard range found in blood donors were 15-50 mg%). We interpreted this decrease in the two blocked proteins as an indication of a response.

Further pig IgG treatment was impossible due to the significant increase in the patient's serum IgM level caused by antibodies against pig proteins.

It was then decided to give the patient reduced weekly doses of Vincristine (1.75 mg) for 4 weeks and during this period the patient re-developed neurotoxic symptoms, ataxia and paresis. After the second and third weekly injection of Vincristine, the C4 level started a steady increase to close to 100 mg% after approximately 2.5-3 months post surgery indicating a continuous blocking interpreted as the inhibition of immune reaction due to chemotherapy.

Due to these symptoms Vincristine was stopped. Two to three months after stop of chemotherapy the neurological symptoms disappeared.

Following the one time surgery and de-blocking treatment of the patient, the serum level of C1 inactivator remained at 50 mg% after approximately 3 months.

The bone marrow findings showed during the coming 2-3 months no recurrence of sarcomatous infiltration.

Three months after ceased treatment the patient had been checked several times. No lymphomas have re-occurred, left testis is of normal size without sign of tumor. Bone marrow has been checked several times without any signs of tumor cells. Neither had scanning revealed any signs of relapse.

### Summary of 5 more patients treated with pig anti human C1 inactivator/CRP IgG

The lymphocyte IgG and IgM fluorescence is measured in relation to these patients as described in US 4,132,769.

### Patient No. 1

A female patient aged 55 years with an advanced metastatic breast carcinoma with en cuirass metastases in the resection zone carcinoma approaching the end stage of her disease, with metastases in lungs, liver and bone was treated with 2 gram of pig anti human C1 inactivator/CRP IgG infused I.V. after negative skin test for pig proteins. After the I.V. infusion of an amount of 2 gram of porcine IgG (anti C1 IA and anti CRP) the patient started to experience pains in the entire area around the resection zone close to the en cuirass constituting 10-12 tumors averaging of 2-3 cm after 1 to 1 ½ hour of the infusion of the pig IgG. At the same time the entire area of this relatively large tumor metastasis suddenly turned blue with no reaction or redness of the surrounding skin which appeared to be more pale, while the tumors turned increasingly bluish - dark red. Having never seen this before, and on the background of the patient's liver metastases, it was decided to stop the reaction by corticosteroid injection which shortly thereafter halted the immune reaction. The pains seized, but the tumors stayed blue and appeared after some hours to show evidence of rejection. The next morning the en Cuirass tumors were slowly drying out and actually peeling off leaving clear demarcation zones. Around two days after, all the tumors had dried out and the bottom of the lesions healed with normal colored fibrous like scar. It was the impression that the blood supply to each tumor had been stopped at the time of the color change. This tumor rejection was interpreted as an immediate reaction which appeared as a result of a complement cascade. This patient was not tested for serum C1 inactivator or C4.

### Patient No. 2

A 53 years old female patient suffering from disseminated metastases from a breast carcinoma with verified osteolytic metastases in her vertebrae, carcinoma metastases to the bone marrow and en cuirass metastases in the resection zone. Following surgery the patient had previously been treated with irradiation against bilateral supra, infra and axillary lymph nodes, and palliative irradiation against lumbar column.

Because of the fast progression of the metastatic carcinoma, the patient was, after negative skin test initially treated with pig anti C1 inactivator/CRP IgG at a total dose 1 gram I.V. The only side effect immediately after was a rise in body temperature, which disappeared after a few hours. One week after the pig IgG infusion, the patient received two cycles (with one week interval) of a cytostatic treatment consisting of Vincristine (total dose 1.15 mg), 5-fluorouracil (total dose 600 mg), metothrexate (total dose 45 mg), cyclophosphamide (total dose 150 mg) and prednisone (total dose 550 mg). Twenty seven (27) days following the initial infusion with pig IgG and after a negative skin test, a second and a third I.V. treatment were administered with an interval of 7 days (total dose of the two cycles 2 gram pig IgG).

The patient experienced a fever after the third cycle with pig IgG at 40o.4 C, which then decreased following administration of aspirin after some hours to normal range. One month after last pig IgG infusion, the patient was given a total of 5.0 mg Vincristine for a period of two months, 5-fluorouracil (1355 mg), and metothrexate (100 mg) and a perorally administered dose of 70 mg cyclophosphamide is given daily.

Regarding the patient's carcinoma condition, the patient's uric acid increased to extremely high levels, and 15 days after (8 days after first cytostatic cycle) the cutaneous en cuirass metastases flattened, and turned to horny scars. After a period of 3.5 months the osteolytic metastases in her vertebrae was replaced by osteosclerotic alteration. One bone marrow sample now showed a normoblastic marrow, another bone marrow taken after 3.5 months showed small islands of tumor cells surrounded by lymphocytes and connective tissue, and normoblastic marrow.

After the first infusion with pig anti human C1 inactivator/CRP, a decrease of serum C1 IA and C4 from 60 - 40 mg% which was noted at the time of the first cytostatic treatment (one week after infused pig IgG). After the cytostatic treatment C4 increased and C1 inactivator was unchanged around 65 mg%. During the second and third pig IgG treatments, C1 IA level started after a immediate peak level of 85 mg% during second and third infusion of pig IgG to decrease to approximately to 40 - 45 mg% and a C4 decreased to 5 mg% between the second and third pig IgG infusion. The concentration of C1 inactivator then slowly decreased and was after 3.5 months after the first pig IgG treatment at 30 mg%. After the next chemotherapy cycle the C4 showed a steady increase to around 70 mg%. The patient did not have any significant increase in serum IgM during the combined chemotherapy and pig IgG treatment.

### Patient No. 3

A 41 year old male with melanocarcinoma with disseminated far advanced subcutaneous and multiple organ metastases. The patient had previously been treated with DTIC (total dose 660 mg), CCNU (total dose 20 mg) and Hydrea (total dose 5000 mg), without any effect on the cancer, but treatment was stopped due to appearance of cardio-toxicity, haematopoietic toxicity and liver toxicity. Weekly cycles of 1 gram pig anti human C1 inactivator/CRP was infused. The weekly cycles were given with an interval of one week (total dose 2 gram). Temperature increased to 41o.6 C. During the second pig IgG infusion the patient was beginning to develop brain metastases and no further pig IgG treatment was attempted.

Clinically, the metastases grew fast during the cytostatic period of treatment. A probable arrest of the growth of the metastases was noted during 2 weeks following the pig IgG treatment. As described below the patient developed a brain metastasis, which prevented further treatment . The patient died 6 weeks after the last pig IgG treatment without any signs of remission of his cancer.

During the first and second weekly pig anti human C1 IA/CRP IgG infusion, a significant immediate peak of serum C1 inactivator increase from around 60 mg% to 90 mg% was noted, followed by a decrease a few days after to 50 mg%. At the time of the detection of the brain tumor metastases the serum C1 IA had decreased - ending at 45 mg% prior to the patient's death after 4 weeks. C4 fluctuated with an immediate decrease during pig IgG treatment followed by a slight increase in C4 to around 50 mg% at the end stage. Already after the first pig IgG infusion the patient's serum IgM level showed a slight increase, probably due to the pig IgG infusions. The heavy chemotherapy treatment obscured any detectable effect of the pig IgG.

### Patient No. 4

A 51 year old male patient with kidney cancer with lung metastases. At the start at the pig anti human C1 inactivator/CRP treatment, the patient had progressively growing tumors. A skin test with pig IgG was negative. The pig anti human C1 IA/CRP I.V. treatment consisted of infusion of 1 gram of pig IgG in 200 ml of saline per day for the first 5 days, and thereafter 0.5 gram of pig IgG. I.V. treatment was then done daily for 4 weeks leading to arrest of growth of lung metastases . During the next 4 months period after the treatment, minor regression of tumor load was noted, although lung metastases are difficult to measure. The patient did not become immunized against the pig IgG protein, not even after an intermittent pause of a fortnight (holiday). 1 1/2 months after the last pig anti human C1 IA/CRP treatment, no tumor growth was observed. We did not have access to Laurell Immunoelectrophoresis at the department and instead we performed Double Mancini immunodiffusion test. The patient's serum showed a significant size CRP ring at the start of the pig IgG treatment, whereas repeating the same test on a serum sample 1.5 months after termination of the pig anti human C1IA/CRP treatment showed a borderline positive CRP ring, when compared to the initial testing. The patient was then transferred to chemotherapy treatment.

### Patient No. 5

A 19 years old male with metastatic testicular teratoma, who had been on Adriamycin treatment for several weeks, but this treatment had to be interrupted due to side effect affecting the liver and the heart . The patient was then treated with pig anti human C1 inactivator/CRP for 5 days starting the first day with 1 gram pig IgG infused I.V. and the following days with 0.5 gram pig IgG per day for approximately 2 weeks. The patient's serum IgM started to increase approximately two weeks after the last pig IgG infusion. The treatment was then stopped and the patient was planned for rescheduling a new series of chemotherapy and the pig IgG treatment had to be stopped due to the development of antibodies against pig proteins.

Prior to the start of the treatment, the patient had developed large metastases in the lungs and in mediastinum and was considered sub-terminal. During the pig anti C1 IA/CRP treatment period, the lung metastases appeared stable for 2.5 months. After the 2.5 months during which the patient did not receive any treatment, the lung metastases started to progress again. The patient was then started with chemotherapy with Adriamycin, however, the metastases continued to grow and the cancer spread to the liver. The patient did not respond after another month on the Adriamycin. The patient turned cachectic and was then considered terminal.

C1 inactivator and C4 was not measured, because at that time we did not have access to the previous Laurell immunoelectrophoresis equipment. Instead the patient was tested using a double Mancini immunodiffusion (see Double Mancini in figure 5).

The double Mancini immunodiffusion test showed that the patient's serum prior to the treatment with the pig anti human C1 IA/CRP showed a very significant CRP ring. In contrast, after treatment a serum sample from the CRP ring decreased in size. No measurement of the precipitation ring was done. When the patient was restarted on chemotherapy (Adriamycin) some days after, a new serum sample was repeated during the chemotherapy period showed a larger CRP ring than before the first pig anti C1IA/CRP treatment, however no measurement was done.

### Conclusion

The conclusion of the above monitoring of serum C1 IA and serum C4 following a cancer patient during a cancer treatment combining both chemotherapeutic treatment, surgical intervention and cancer immune de-blocking treatment appeared to be very useful when relating the test results with the development and changes in the cancer during the months that the patient was followed.

Because we later on found that the immunogen isolated from carcinoma cell explants actually did not contain two variants of C1 IA, which were our early interpretation of the two proteins harvested from cloned explants from carcinomas from several patients, but later on the two proteins were separated individually and appeared to be a combination of C1 IA and C Reactive Protein (CRP).

The pig IgG antibody used to treat the 6 patients described (including the child, see patient No. 6) was anti C1 IA/CRP as indicated in the Double Mancini test in figure 5.

Therefore C1 IA and CRP must in our opinion have been closely related to the carcinoma explants used for the development of the purified immunogen used for the pigs.

The Human recombinant IgG anti human C1 IA/CRP is developed based on IgG1 and IgG3 in transfected human cells (e.g., HEK 293 subtype cell line) to be formulated for clinical use in the treatment of patients with cancers. The purpose is to start a pilot study and/or a phase I or phase I/II clinical trial in patients with advanced cancer and/or metastases with no other treatment possible.

The Human recombinant anti human C1 inactivator/CRP IgG (either IgG1 or IgG3) is produced using a patented methodology with the recombinant IgG1 and/or IgG3 in human cells transfected with the IgG antibody using cell culturing in a closed "Wave Bio-reactor system" and purified in approved Class 3 clean room laboratory, subjected to strict quality control and validation, and packaged as lyophilized IgG preparation.

The product will be safety and toxicity tested as previously described, and due to the fact that it constitutes purified recombinant human IgG with specificity against C1 inactivator/CRP, it is anticipated that this product will be tolerated without producing antibodies against this type of Human IgG. Furthermore, the previous pilot studies consisting of intravenous infusion of purified pig anti human C1 inactivator/CRP IgG used in 1 to 3 doses did not cause side effects or adverse reactions. However, IgM level was increased following the pig IgG treatment, preventing further treatment.

The Human recombinant anti C1IA/CRP "De-Blocking" antibody can be administered as long at the patient benefits from this treatment. Dana Genetic's "Immune Blocking Diagnostic Test Kit" can be valuable for monitoring of C1 IA, C4 and CRP levels in relation to tumor response.

A possible brain cancer de-blocking treatment approach based on detection of C1 inactivator on malignant primary and secondary brain tumors.

It is until now unknown if diagnosing any change in patients with brain tumors is reflected in Dana Genetic's Test kit for use in serum.

A study on diagnosing malignancy of brain tumors were done by Kurt Osther et al. It was found that C1 inactivator coated malignant cell cultures and certain subcultures explanted from biopsies obtained from a neurosurgery department in Denmark testing various tumors including benign tumors and malignant primary and secondary brain tumors. The testing of the cell cultures and subcultures were based on the use of rabbit polyclonal anti human C1 inactivator-FITC (fluorescein conjugated antibody). The cells were measured using a Leitz MPV2 equipped for measuring immunofluorescence on single cells on a cell layer seeded onto microslides from cultured and sub-cultured brain biopsy specimens.The testing also included adequate control of the specificity of the C1 IA coat on the cells (Osther K, Hoejgaard K, Dybkjaer E, Acta Neurol. Scand. (1974) 50:681-689). This immunofluorescence, representing the C1 IA coating of the cells was clearly demonstrated to be reproducible.

Cell cultures from primary malignant brain tumors and tumors with uncertain classification after Kernohan (Zülch K, Atlas of Gross Neurosurgical Pathology, Kernohan classification from 1949, 1952, pp32 - 33. (Author and editor Klaus Zülch) Springer Verlag Berlin Heidelberg GmbH 1975), - from secondary malignant brain carcinoma metastases, and from benign primary brain tumors were investigated.

A number of 7 out of 8 astrocytomas grade II-IV and one meningeoma showed presence of C1 IA coated brain cells in accordance with histological diagnosis. Two (2) astrocytomas (Kernohan grade uncertain) were not showing any C1 IA coated cells.

A number of 6 out of 7 secondary malignant brain tumor, metastases from various carcinomas, showed C1 IA coated cells.

A number of 8 primary benign tumors did not show any C1 IA coated cells. One patient with necrotic tissue without definite tumor mass showed C1 IA coated cells.

An important difference between secondary brain tumors caused by carcinomas, and primary malignant brain tumors

This brain tumor study showed indications that sub-cultured secondary brain metastases of carcinomas retained the C1 IA coating cells, whereas interestingly, primary malignant astrocytomas lost the C1 IA coat in sub-cultures.

Besides losing the C1 IA coat, sub-cultured primary malignant brain tumors changed appearance resembling fibroblasts. The culture medium did not reflect the same biological conditions when compared to the conditions present in the CNS system.

Traditionally, gliomas are considered to be confined to the central nervous system. (Jimsheleishvili S, Alshareef AT, Papadimitriou K, Bregy A, Shah AH, Graham RM, Ferraro N, Komotar RJ. J Cancer Res Clin Oncol. 2014 May;140(5):801-7). Even highly malignant brain tumors such as glioblastoma mulltiforme do rarely metastasize to the remaining organism outside the CNS. The reason could be due to the fact that the biological environment for primary brain tumor cells outside the CNS is different from inside the CNS so that the environment outside CNS is not conducive for expansion of primary brain tumors hindering metastasizing.

Primary malignant brain tumors are normally well vascularized and have an especially well vascuralized edge of tumor, the most malignant glioblastomas often appears to have necrosis in parts of the tumor. This can be due to the fact that the blood brain barrier is extremely complex. It could be postulated that the lack of a C1 IA blocking coat in brain tumor cells released outside the CNS system might either be unable to survive, because the cells outside CNS change in behaviour and may be recognized by the immune system and subsequently be lysed and/or rejected.

### Example 6: Rat RG-2 and Rat NS-1 gliomas

Recent studies have given significant evidence of the presence of C-reactive Protein and also the presence of C1 inhibitor, also called C1 esterase inhibitor or C1 inactivator (C1IA) on malignant cancer cells and among these malignant cancer cells, malignant brain tumors such as astrocytomas, also called gliomas or glioblastomas. This novel finding has also been identified in rat gliomas such as (rat) glioma RG-2 gliomas and (rat) glioma NS-1 also called CNS-1.

### Rat RG-2 and Rat NS-1 gliomas

These malignant cancer cells have been cultured in disposable Leighton-like chambers as have human gliomas and glioblastomas, such as glioblastoma multiforme. All of these also called astrocytomas at the Rausing laboratories at Lund University in Southern Sweden.

These malignant glioma cell lines were incubated with rabbit anti human C-reactive Protein, found to cross-react with rat C-reactive Protein. A secondary TRITS labeled antibody against rabbit antibody was used to react with bound human antibodies against (rat) C-reactive Protein. See figures 6a and 6b shows rat RG-2. Glioma cells that have been incubated several hours with polyclonal rabbit anti human C-Reactive Protein (anti HU CRP), washed with PBS buffer or culture medium and then incubated with TRITS conjugated anti rabbit antiserum as a secondary antibody to show binding (see red RG-2 cells shown in figures 6a-6b).

### GFP incorporation in RG-2 rat glioma cells

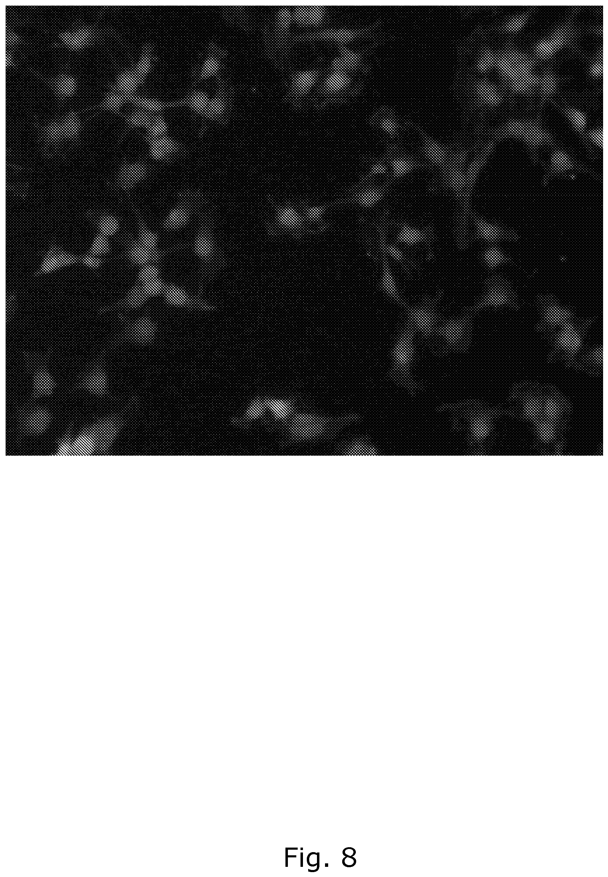

As seen figure 8, the RG-2 glioma cells are visualized using for green fluorescence by incorporation of GFP.

The visualization of the rat RG-2 glioma cells is accomplished by co-located both RG-2 Rat GFP gene pos (live) glioma cells. At the same time the figure 9 shows the presence of the C-reactive Protein coat on the RG-2 glioma cells, as stained with rabbit anti human (anti human antibody, know to cross-reacting with anti rat CRP and secondary TRITS Ab (red outer coat).

This figure 8 shows the green fluorescence especially visible in the nucleus and in the cytoplasma of the glioma cells. The red TRITS labeled antibody appears coating the periphery of the RG-2 glioma cells giving evidence of an plasma membrane - located C-Reactive protein reacting with rabbit anti human (known to cross-react with rat anti C-Reactive Protein).

The crucial importance of the findings of CRP and the C1 inhibitor or C1 Inactivator (C1 IA) shall be viewed from the background of the fact that these recent glioma cell experiments from rat gliomas are known to be very similar to human malignant gliomas and are used as a rat model for human gliomas also called glioblastoma or glioblastoma mulitforme.

The C1 esterase inhibitor also called C1 inhibitor or C1 inactivator (C1 IA) was identified to coat glioma cells such as RG-2 glioma cells as an important finding of this invention as well as the findings of C-reactive protein on glioma cells also is a very important part of this invention.

### Example 7:

### Rat RG-2 and Rat NS-1 gliomas

These malignant cancer cells have been cultured in disposable Leighton-like chambers as have human gliomas and glioblastomas, such as glioblastoma multiforme. All of these also called astrocytomas at the Rausing laboratories at Lund University in Southern Sweden.

These malignant glioma cell lines were incubated with rabbit anti human C-reactive Protein, found to cross-react with rat C-reactive Protein. A secondary TRITS labelled antibody against rabbit antibody was used to react with bound human antibodies against (rat) C-reactive Protein. See figures 10 shows rat RG-2 Glioma cells that have been incubated several hours with polyclonal rabbit anti human C-Reactive Protein (anti HU CRP), washed with PBS buffer or culture medium and then incubated with TRITS conjugated anti rabbit antiserum as a secondary antibody to show binding (see red RG-2 cells shown in figures 10).

Figure 11a and 11b. NS-1 (CNS-1) rat glioma cells expanded in disposable Leighton-Like chambers and incubated with rabbit anti human CRP (known to cross-react with rat CRP). Surplus antibody removed by washing in buffer (medium), and second TRITS labelled anti rabbit antiserum was added. The cells were then washed, and subjected to dark field microscopy with Excitation filters allowing TRITS to be detected. The cells show coating of anti CRP as evidence of the presence of (rat) CRP visualized with the secondary TRITS labelled antibody against rabbit antibody. The cells' nucleus are seen as darker round typically looking nuclei in cells.

### Example 8:

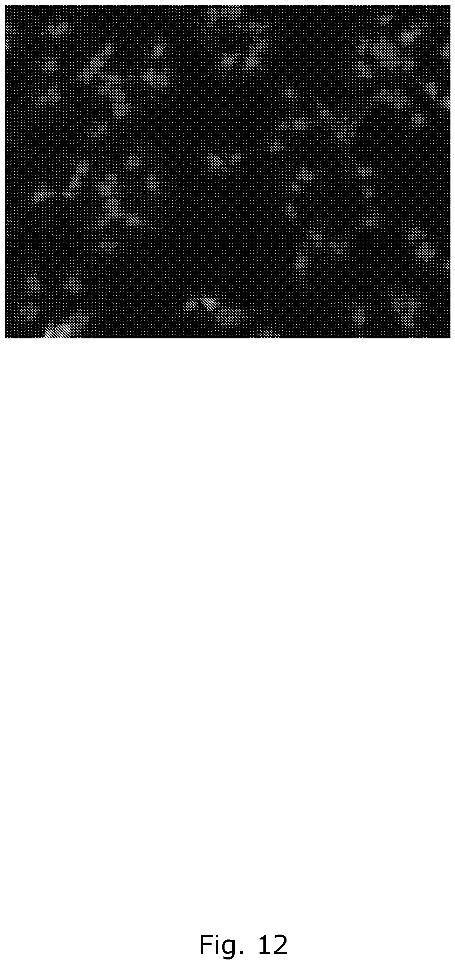

GFP incorporation in RG-2 rat glioma cells. As seen in figure 12, the RG-2 glioma cells are visualized using for green fluorescence by incorporation of GFP.

### Example 9:

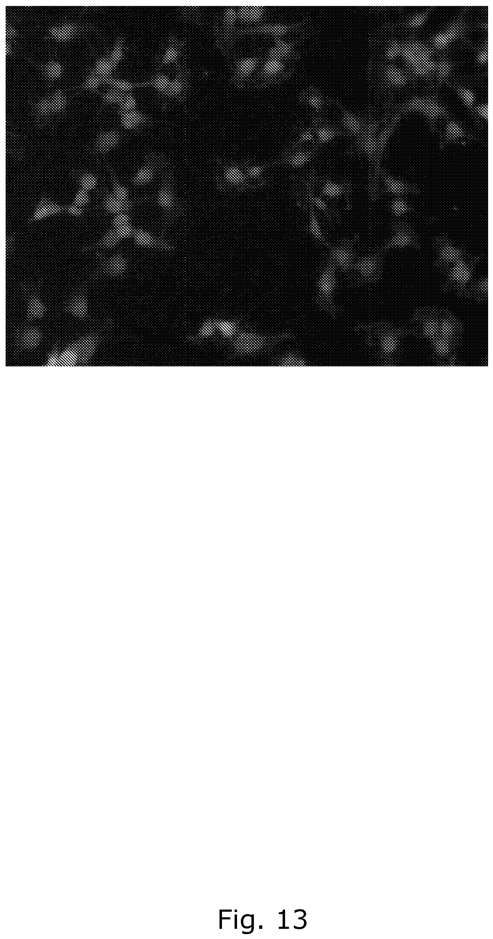

The visualization of the rat RG-2 glioma cells is accomplished by co-located both RG-2 Rat GFP gene positive (live) glioma cells. Figure 13 shows the presence of the C-reactive Protein coat on the RG-2 glioma cells, as evidenced by incubating the cells with rabbit anti human, located on the plasma membrane of the cells (anti human antibody used, are known to cross-react with anti rat CRP. Using the immunostaining sandwich method, a secondary TRITS antibody show the "red" biding of the plasma membranes of the cells.

This figure 13 shows the green fluorescence caused by the GFP incorporatin in these cells and make them visible especially in the nucleus and in the cytoplasma of the glioma cells. The red TRITS labeled antibody appears coating the periphery of the RG-2 glioma cells giving evidence of an plasma membrane coated C-Reactive protein (CRP), which is reacting with rabbit anti human (known to cross-react with rat anti C-Reactive Protein). The method consists of merging the GFP stained visibility as green fluorescence, very noticeable in the nucleus of the cells.

As can be viewed in figure 13, an important finding of this invention as well as the findings of C-reactive protein on rat RG-2 glioma cells also is a very important part of this invention. This was also found in other types of rat glioma cells such as NS-1 rat cells. It is therefore evident that these rat glioma cell (exemplified using RG-2, and NS-1 glioma cells for these investigations). This is an anticipation of what will be found on the coat of human glioma/glioblastoma cells. Again these findings indicate the significant importance of the presence of these proteins, C1 inactivator, and C-Reactive protein (CRP) on certain cancer cells and giving a significant evidence of the fact that it is indeed not the complement reaction itself that promotes the tumor cell growth in these cases, but absolutely for the first time showing that the cancer cells such as for instance gliomas and glioblastomas utilize methods that inhibit the complement system from killing or lysing the cancer cells by hindering the complement reaction from taking place already at the initiation of the complement activity in response to possible antibodies by hindering this activity from taking place, namely by hindering the Clqrs complex constituting the normal classical pathway from disassociate to C1r and C1s

### Example 10:

The C1 esterase inhibitor also called C1 inhibitor or C1 inactivator (C1 IA) was identified to coat glioma cells such as RG-2 glioma cells. As seen in figure 14 C1 inactivator is coating rat RG-2 glioma cells. Dapi stain was used for showing nuclei. Staining with Dapi blue together with anti C1 IA (and performed using sandwich immunostaining with secondary sandwich antibody that was labelled with TRITS give evidence of that the nuclei of these rat glioma cells is not stained with anti C1 inactivator, but the plasma membrane of the rat glioma cell is expressing C1 inactivator as evidenced by the TRITS (red) coloring.

### Example 11:

The crucial importance of the findings of the C1 inhibitor or C1 Inactivator (C1 IA) shall be viewed from the background of the fact that these recent glioma cell experiments from rat gliomas are known to be very similar to human malignant gliomas and appears to have the same type of coat on their glioma cell membrane as the human glioma/glioblastoma as indicated in figures 16a, 16b, and 16c. Therefore we have used as a rat glioma model (actually two rat glioma models, one called RG-2 and one called NS-1) to compare what will happen when to anticipate what will happen to human gliomas also called glioblastoma or glioblastoma mulitforme having this identical coat of protease inhibitor, which is one of the targets for the treatment of human glioma/glioblastoma descibed in this invention. Therefore a series of 3 different human glioma/glioblastoma cells were tested using immunostaining without permeabilization of the cells in either of the rat glioma or in the human glioma/glioblastoma.

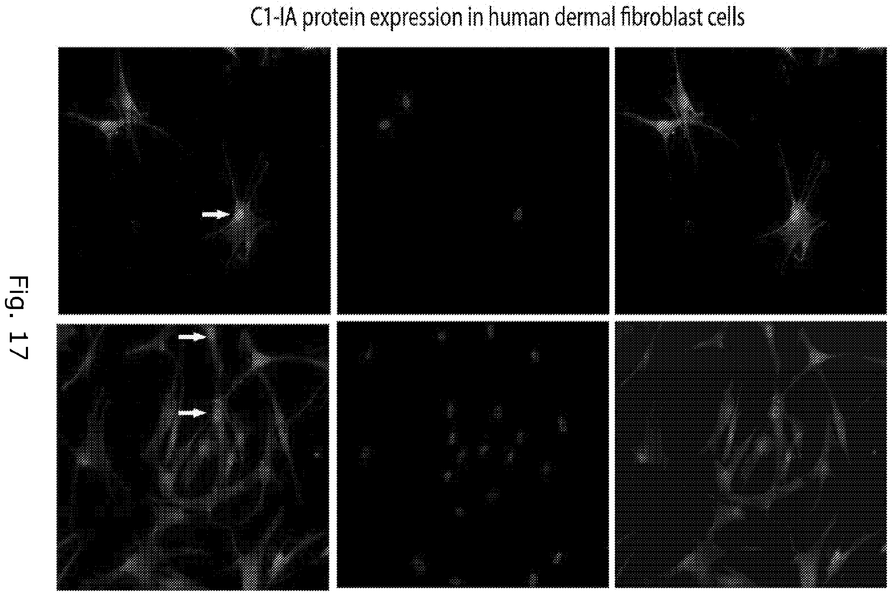

Human Benign skin (dermal) fibroblasts incubated with anti C1 inactivator and with secondary FITC labelled antibody showing a definite difference as opposed to the cancer cells (e.g., rat glioma and human glioma/glioblastoma). The findings in the cells there is a weak expression of C1-IA in nucleus of fibroblast cells but not in cytoplasm or surface of cells. If one compares this image with previous images of human glioblastoma cells and GFP rat cells there is strong expression of C1-IA in cytoplasm or cell membrane but not in nucleus in the cancer cells. A weak C1 IA is definitely located in the nucleus and not on the plasma membrane. In this test the cells were not treated with a permeabilization step (see figure 17).

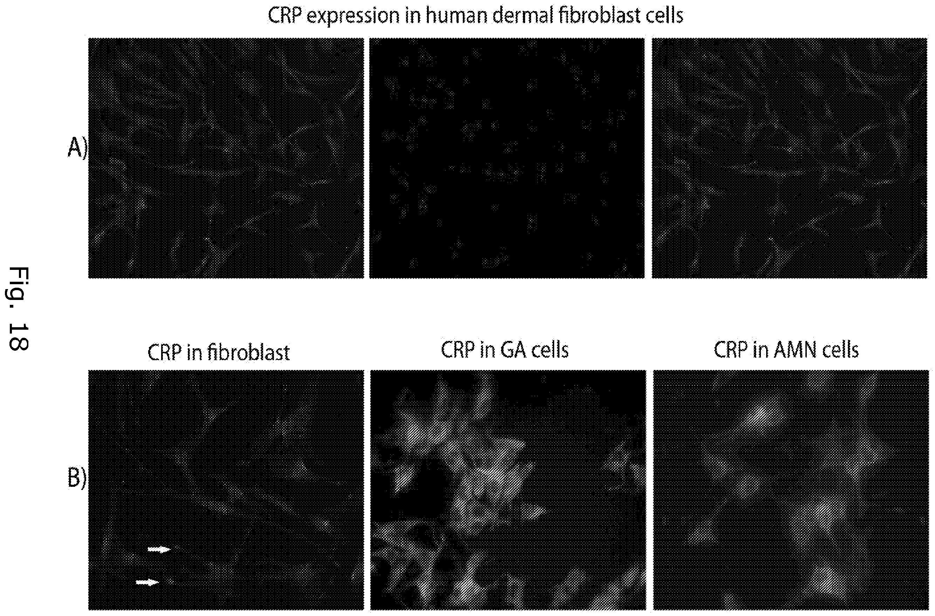

When the human benign skin fibroblasts were incubated with anti CRP and secondary sandwich labelled there is only a very weak signal indicating no specific binding of CRP onto the surface of these benign cells. Without permeabilization step (see figure 18).

### Regarding the findings in human skin fibroblasts re. C1 inhibitor (C1IA)

As described by Gulati et al in besides C1 inhibitor (C1IA), the complement component C1r, C1s synthesized by all cell types of many non malignant cells. In our invention a higher amount of C1 IA in cancer cells may inhibit the C1qrs complex from being disassociated to C1q, C1r and C1s. In benign cells the secretion rates of C1r and C1s were approximately equimolar in fibroblasts and in the disassociated human mesenchymal cells, chondrocytes, whereas the secretion rate for C1s exceeded that for C1r in the other cell types (Gulati P, Lemercier C, Guc D, Lappin D, Whaley K. Regulation of the synthesis of C1 subcomponents and C1-inhibitor. Behring Inst Mitt. 1993 Dec;(93):196-203).

According to these authors, who examined the synthesis of C1q, C1r, C1s and C1-inhibitor in HepG2 cells, human umbilical vein endothelial cells (HUVEC), fibroblasts (skin and synovial membrane), chondrocytes and monocytes and C1q (only found to be synthesised by monocytes) - C1r, C1s and C1-inhibitor were synthesised by all cell types. Molar ratios of C1s to C1r were approximately 2:1 for HepG2 cells, 5:1 for monocytes and 10:1 for HUVEC. Stimulation with interferon-gamma resulted in increased expression of all four proteins. The C1s:C1r ratio did not alter in chondrocytes or fibroblasts, but approached unity in HepG2, monocytes and HUVEC, due to relatively greater stimulation of C1r gene expression.

In 2002, I isolated total RNA from 4 human chondroblast cultures harvested from cell explants from the cartilage tissue in the knee of four (4) patients, two with cartilage defects grade III (no osteoarthritis, and two with grade IV (with beginning osteoarthritic changes).

The total RNA from chondroblasts from femoral condyle of these four patients was isolated and subjected to Affymetrix Gene Chip Analysis testing around 12,000 genes at Professor Klaus Bendtsen's Department of Inflammatory Research, Rigshospitalet, Copenhagen.

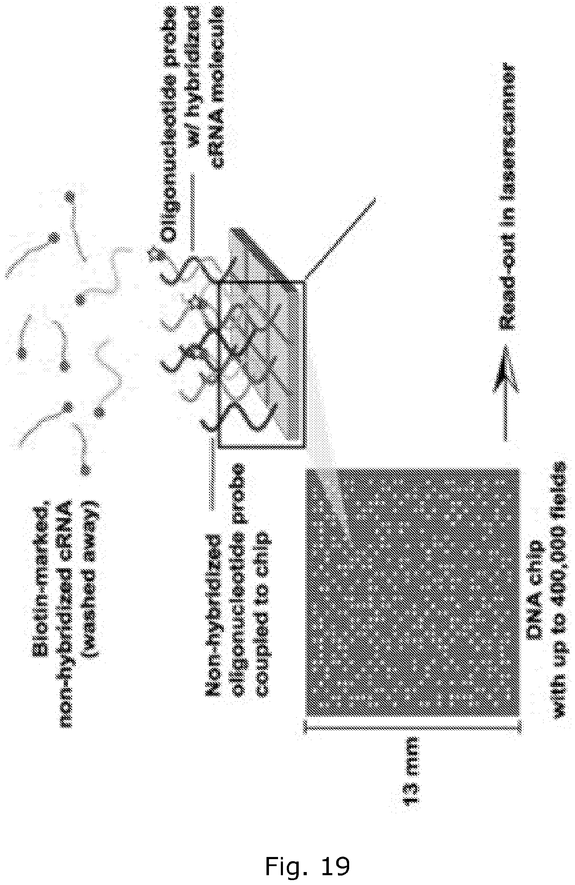

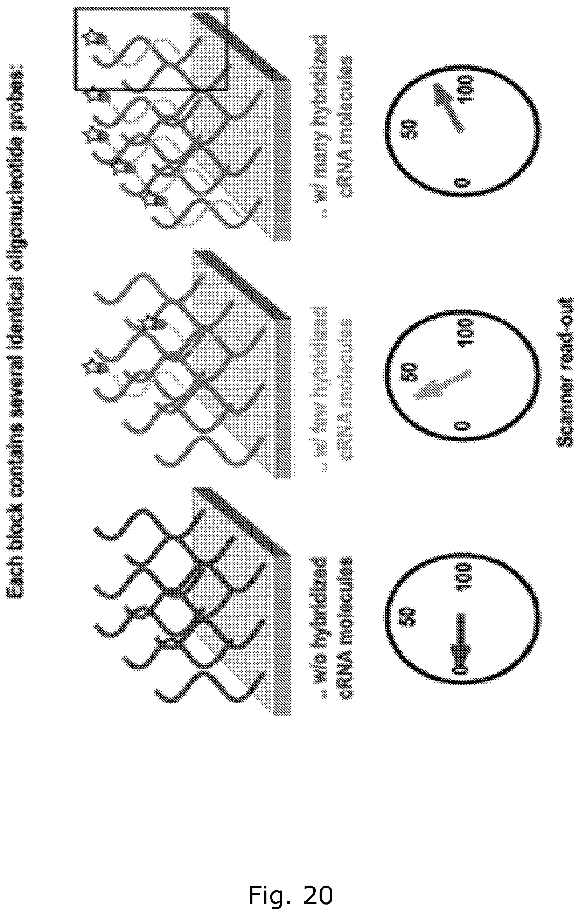

The testing method using the Affymetrix Genchip array is roughly described in figures 19 and 20.

In this library, I tested these human chondroblasts (which resemble chondrocytes) and got a read out on activated and non-activated genes. I used a non-gene activated protein namely collagen type X, which appeared to have low read out values below an arbitrary limit of 100 (≤90). Among these many genes I identified was complement component C1q and looked at the read out on the 4 patients, which showed the following.

### Example 12:

### Affymetrix Gene Chip Array

### Gene Chip Array Library of the Complement system in non malignant human mesenchymal cells

In this library, we tested these human chondroblasts that were cultured and after having reached 70% confluence in 25 cc Falcon flasks, tRNA were harvested in serum-free medium and brought to the Institute for Inflammatory Research, Rigshospital (Danish University Hospital), Copenhagen, the department headed by Professor Klaus Bendtsen. 12,600 cDNA. These human mesenchymal cells (which resemble chondrocytes) were tested on a chip containing approximately a read out on activated and non-activated genes.

I used a non-gene activated protein namely collagen type X, which appeared to have low read out values below an arbitrary limit of 100 (≤90).

Among these many genes identified in the 12,600 gene chip array were several of the complement components. One was complement component C1q and looked at the read out. This contains gene information on 4 patients, where the mesenchymal cells are derived from cartilage biopsies from the patients' knee condyle on a non-bearing zone, and where the cells have been cultured in foetal calf serum-free medium where the cells were cultured until 70% confluence, total mRNA was harvested and prepared for Affymetrix Gene chip analysis - this was prepared at Professor Klaus Bendtsens Institute for Inflammatory Research at Panum Institute, Copenhagen, with a coded ID as follows: KM, HL, MT, and IB, which showed the following.

### Example 12a:

### The C1 inhibitor (C1 inactivator (C1 IA)):

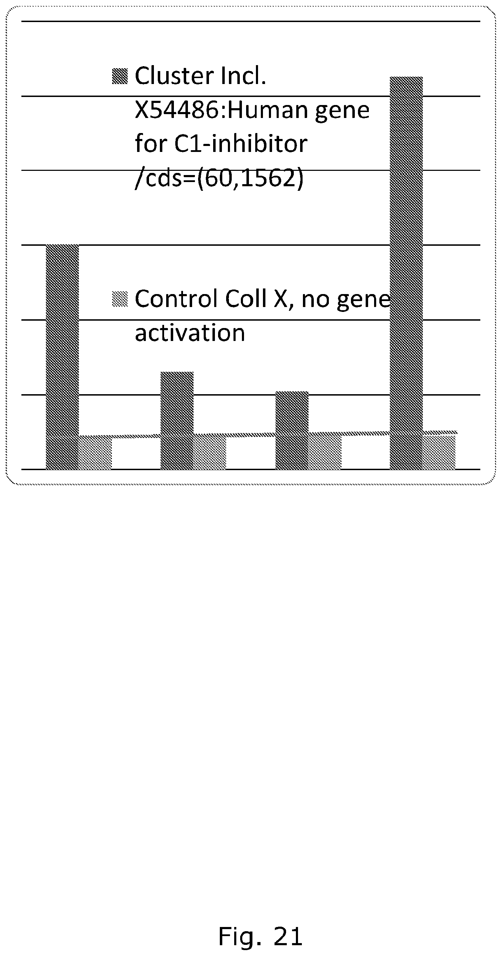

The first gene of interest in tested in the human mesenchymal cells is C1 inhibitor (C1 inactivator (C1 IA) (a protein that is known to be present in cells such as fibroblasts, and among other things, in chondroblast (∼chondrocytes), etc., This was identified after search in the Gene Chip array among ∼12,600 genes. The read out of the C1 inhibitor C1 IA on the 4 patients, showed in figure 21.

### Example 12b:

### Complement component C1q:

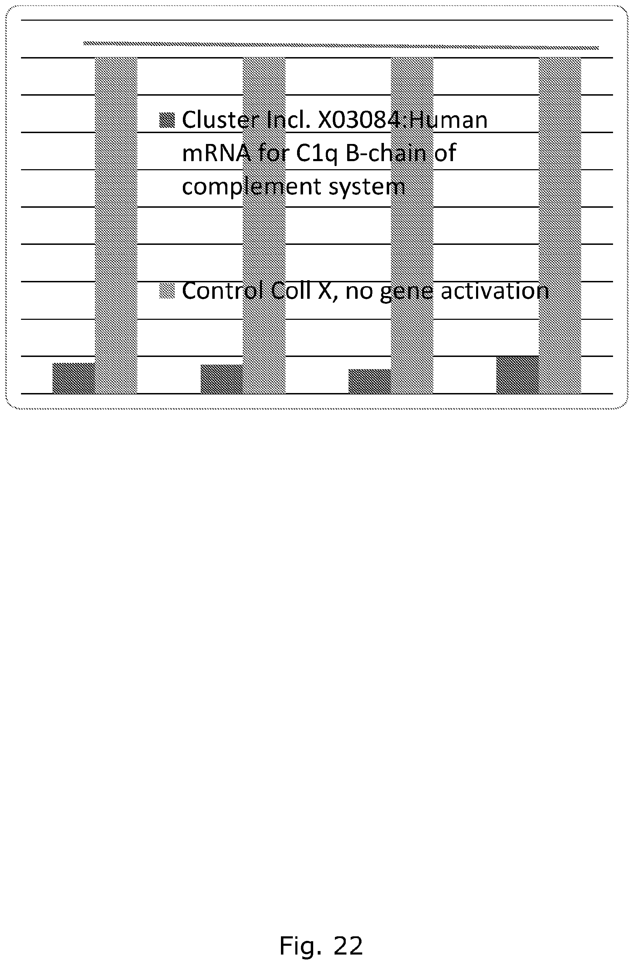

Among these many genes I identified was complement component C1q and looked at the read out on the 4 patients as shown in figure 22. The read out showed that the C1q gene was very low in signal and far below the red border where any signal below this border is considered an inactive Gene, which showed up as follows regarding C1q in benign human mesenchymal cells.

### Example 12c:

### Complement component C1r:

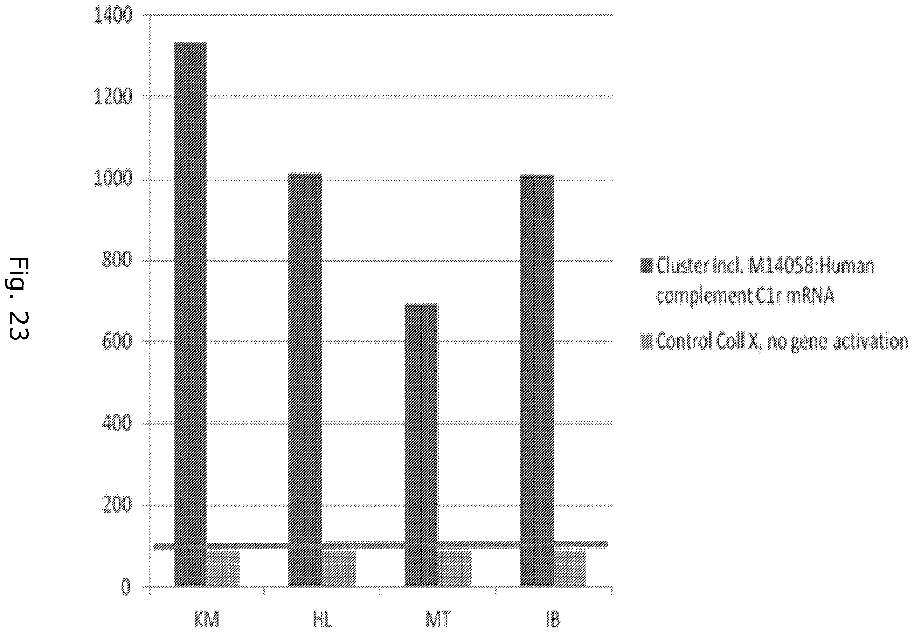

The next gene of interest and examined using the Gene Chip array on mesenchymal cells from the four (4) listed (coded) patients was C1r (disassociated from Clqrs complex) that I identified and looked at the read out of C1r on the 4 patients, which showed the ≥ 1 log over border line, implying that the C1r gene was activated (see figure 23).

Interpretation of the gene activity found in human mesenchymal cells such as chondroblasts in Example 12b that C1q was not disassociated and that C1r was found to be active, most probably at a steady state, most probably balanced by the gene activity in the cells of the inhibitor of the C1 complex, C1 inhibitor (also called C1 inactivator (C1 IA). As can be seen there is apparently no antigen antibody reaction taking place in these cells, also indicated by the fact that C1r has not activated C4 (see example 12d).

Using Affymetrix Gene Chip Analysis, I found that C1q is not activated in chondroblasts. The method for harvesting the chondroblasts from human cartilage explants was a technology described by Kurt Osther et al. and published in 2006 (Osther K, Storgaard P, Clausen Cl The Rationale to use Explant Cultures for ACI. (in Basic Science and Clinical Repair and Reconstruction of Articular Cartilage defects: Current Status and Prospects, Zanasi S, Brittberg M, Nehrer S, Marcacci M, editors),pp313-319, Timeo Bologna 2006).

Furthermore, I found that C1r was significantly elevated in these human chondroblasts, which is consistent with the results published by Gulati P et al. using the methods described by that group in 1993 (Gulati P, Lemercier C, Guc D, Lappin D, Whaley K. Regulation of the synthesis of C1 subcomponents and C1-inhibitor. Behring Inst Mitt. 1993 Dec;(93):196-203)*.* This corresponds overall to my postulated theory around the difference in this regard, when comparing these types of benign cells with glioma/glioblastoma.

Regarding the findings in human chondroblasts of gene activity based on results on testing of four (4) human chondroblasts (∼chondrocytes) re. complement component C4 ,C5, C6, C7, C8, and C9 a continuation of gene chip analysis of complement components in benign mesenchymal cells such as chondroblasts (∼chondrocytes), where the isolation of the chondroblasts from the cartilage harvested from the femoral condyle of 4 patients, where two of these patients had cartilage defects grade III (non osteoarthritic) and two had cartilage defects grade IV (beginning osteoarthritis). No evidence of C-reactive protein (CRP) is coating these mesenchymal cells either, contrary to the findings in both human carcinoma cells and now proven to coat both NS rat glioblastoma cells and in human glioblastomas.

In 2002, I isolated total RNA from 4 human chondroblast cultures harvested from cell explants from the cartilage tissue in the knee of 4 patients, two with cartilage defects grade III (no osteoarthritis, and two with grade IV (with beginning osteoarthritic changes).

The total RNA from chondroblasts from femoral condyle of these four patients was isolated and subjected to Affymetrix Gene Chip Analysis testing around 12,000 genes done at Professor Klaus Bendtsen's Department of Inflammatory Research, Rigshospitalet, Copenhagen.

### Example 12d:

### Complement component C4b U24578:Human RP1 and complement C4B precursor (C4B) genes

### C4b gene

This gene encodes the basic form of complement factor 4, part of the classical activation pathway. The protein would be expressed as a single chain precursor which an antigen antibody reaction had been taken place in the tissue from which these benign mesenchymal cells were derived. Otherwise there would have been a disassociation of C4 to further activate complement component C2, if there had been occurring a proteolytically cleavage into a trimer of alpha, beta, and gamma chains prior to secretion. The trimer would have provided a surface for interaction between the antigen-antibody complex and other complement components, for instance showing binding of some part of the C4 molecule to a cell surface.

The alpha chain was not cleaved to release C4 anaphylatoxin, otherwise a mediator of local inflammation. This gene localizes to the major histocompatibility complex (MHC) class III region on chromosome 6. Varying haplotypes of this gene cluster exist, such that individuals may have 1, 2, or 3 copies of this gene. In addition, this gene exists as a long form and a short form due to the presence or absence of a 6.4 kb endogenous HERV-K retrovirus in intron 9.

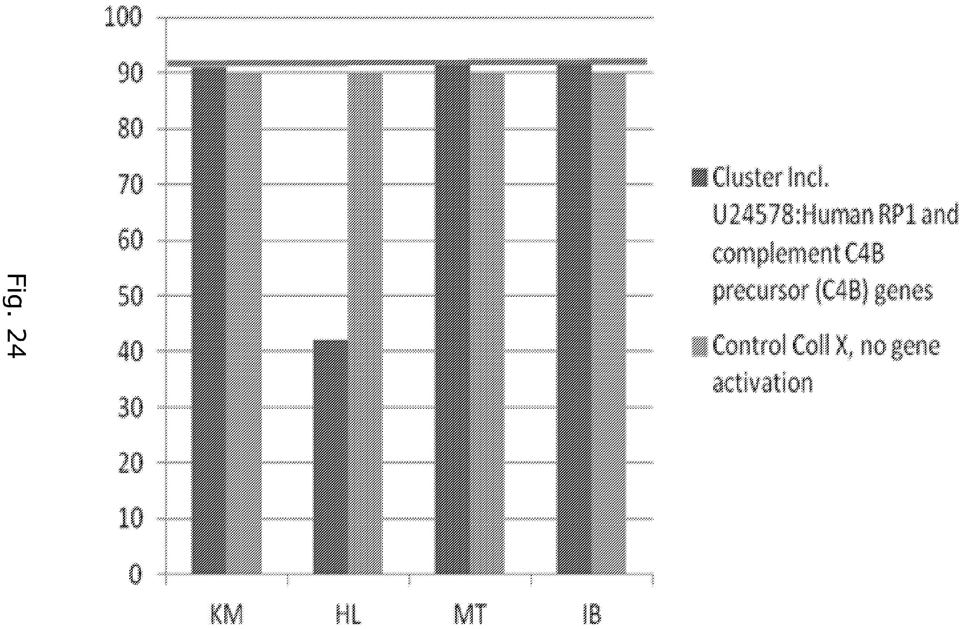

Among these genes I have also identified complement component C4b and looked at the read out on the 4 patients, shown in figure 24.

### Example 12e:

C5 also covers the following subunits of C5

### Summary

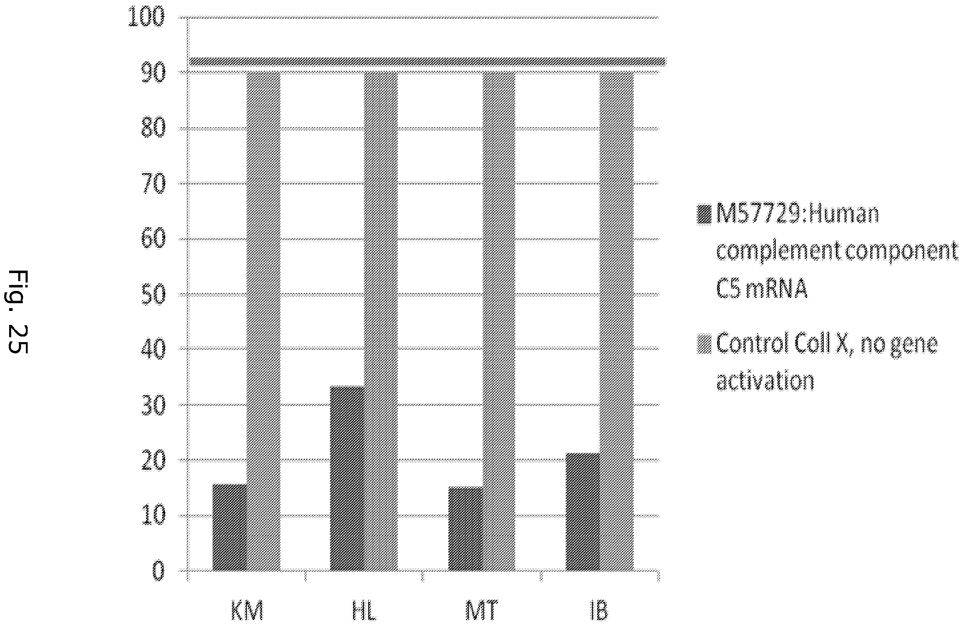

The C5 gene encodes a component of the complement system, a part of the innate immune system that plays an important role in inflammation, host homeostasis, and host defense against pathogens. If C5b macromolecular cleavage product has taken place all the way from C5b to C9 on a cell surface creating membrane attack complex (MAC) to take place killing the cell. Mutations in this gene cause complement component 5 deficiency, a disease characterized by recurrent bacterial infections. See figure 25.

### Example 12f:

### Complement component C6

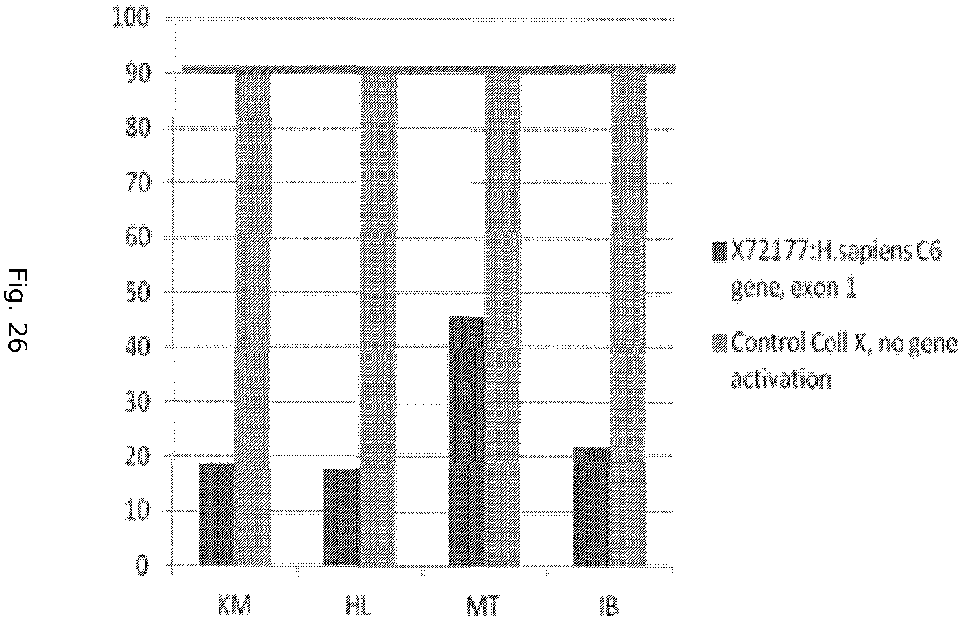

This gene encodes a component of the complement cascade. The protein, Complement component C6, is protein bound to a cell surface when activated and is part of the membrane attack complex that can be incorporated into the cell membrane and cause cell lysis. Mutations in this gene are associated with complement component-6 deficiency. Transcript variants encoding the same protein have been described. See figure 26.

### Example 12g:

### Complement component C7

### Summary

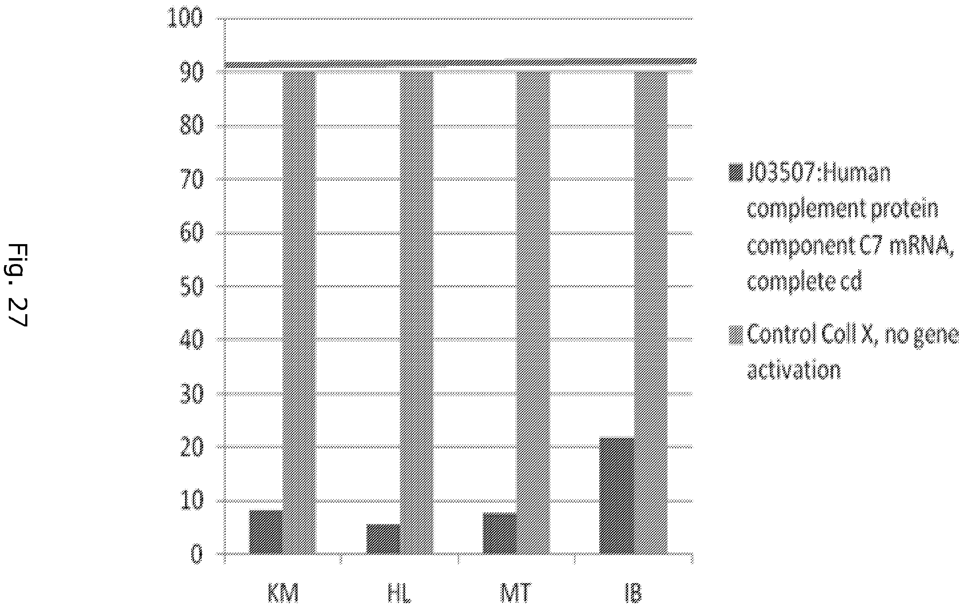

C7 is a component of the complement system. It participates in the formation of Membrane Attack Complex (MAC). People with C7 deficiency are prone to bacterial infection. See figure 27.

### Example 12h:

### Complement component C8 (beta)

This gene encodes one of the three subunits of the complement component 8 (C8) protein. There is no C8 gene activity in these mesenchymal cells. C8 is composed of equimolar amounts of alpha, beta and gamma subunits, which are encoded by three separate genes. C8 is one component of the membrane attack complex, which mediates cell lysis, and it initiates membrane penetration of the complex. The protein mediates the interaction of C8 with the C5b-7 membrane attack complex precursor. In humans deficiency of this protein is associated with increased risk of meningococcal infections. Alternative splicing results in multiple transcript variants. See figure 28.

### Example 12i:

### Complement Component C8 alpha

C8 (alpha) is a component of the complement system and contains three polypeptides, alpha, beta and gamma. This gene, which is not activated in these mesenchymal cells, encodes the alpha subunit of C8. C8, when activated, participates in the formation of the membrane attack complex (MAC). The MAC assembles on bacterial membranes to form a pore, permitting disruption of a cell or a bacterial membrane Mutations in this gene cause complement C8 alpha-gamma deficiency. See figure 29.

### Example 12j:

### Complement component C9

When activated this gene encodes the final component of the complement system. It participates in the formation of the Membrane Attack Complex (MAC). The MAC assembles on bacterial membranes to form a pore, permitting disruption of bacterial membrane organization. Mutations in this gene cause component C9 deficiency. See figure 30.

### Example 12k:

### C-Reactive Protein (CRP)

CRP is in some cases found to be present in relation to activation of the complement system and in connection with such activation. I have found that in the case of malignant rat glioma NS1 and RG2 as well as human glioma/ glioblastoma cells in culture both C1 inhibitor (C1 inactivator) and CRP are both coating these cells as indicated in previous figures using immune staining methods. See figure 31.

### Summary

There was not found any evidence of C-Reactive Protein (CRP) reaction in the mesenchymal cells as seen in figure 31.

When activated this gene encoding the C-Reactive Protein of the complement system can happen during activation of the classical pathway of the complement system is a well known and direct biological function of CRP (Pepys MB, Hirschfield GM. C-reactive protein: a critical update. J Clin Invest 2003;111:1805-12). In contrast, during CRP induced activation of complement and opsonization of apoptotic cells, the actively phagocyting macrophages reduce expression of IL-12 and thereby suppress T-lymphocytes (Kim SJ, Gershov D, Ma X, Brot N, Elkon KB. Opsonization of apoptotic cells and its effect on macrophage and T cell immune responses, Ann NY Acad Sci 2003;987:68- 78). The results suggest that CRP bound to a surface provides secondary binding sites for H most probably resulting in greater regulation of alternative pathway amplification.

Via this action, CRP directly amplifies and facilitates innate immunity, a process that has already been associated with initiation and progression of various inflammatory conditions, cardiovascular disease, cancer, C-reactive protein (CRP) is a substance produced by the liver in response to inflammation. Other names for CRP are high-sensitivity C-reactive protein (hs-CRP) and ultra-sensitive C-reactive protein (us-CRP).

C-reactive protein (CRP) is an acute-phase serum protein and a mediator of innate immunity. CRP binds to microbial polysaccharides and to ligands exposed on damaged cells. Binding of CRP to these substrates activates the classical complement pathway leading to their uptake by phagocytic cells. Complement activation by CRP is restricted to C1, C4, C2 and C3 with little consumption of C5-9.

A high level of CRP in the blood is a marker of any condition that causes inflammation, from an upper respiratory infection to cancer. High CRP levels can indicate that there is inflammation in the arteries of the heart, which can mean a higher risk for heart attack. It is important to remember, however, that CRP is an extremely nonspecific test and can be elevated in any inflammatory condition. As part of this invention it appears that CRP actually is coating cancer cells such as rat glioma cells in culture and such as human glioblastoma cell cultures, as can be observed from the examples of immune-staining above.

CRP may even prevent the formation of the Membrane Attack Complex through C5b to C9 (MAC). The MAC reaction apparently does not take place in the rat malignant glioma and the human glioma/glioblastomas, whereas C5b to C9 is normally assembled on bacterial membranes to form a pore, permitting disruption of bacterial membrane organization. Mutations in this gene cause component C9 deficiency.

Preliminary scanning of Complement factors in patients with Glioblastoma, Henrietta_2_Oscar mRNA Microarray,
Patient AMN with glioblastoma
Patient DZ with glioblastoma
mRNA NCBI Access No. AA144838. serine (or cysteine) proteinase inhibitor, clade G (C1 inhibitor), member 1.

### Example 13:

mRNA testing has been done on two patients with glioblastoma, where the cells from one patient coded AMN and patient coded DZ were tested for the presence of C1 inhibitor (C1 IA) coat, stained with anti human C1 IA and secondary FITC labeled sandwich antibody in Example 11.

The results in this example show mRNA done by microarray testing (Henrietta_2_Oscar AMN and DZ. See figure 32).

### Example 14:

The same two patients with glioblastoma coded AMN and DZ were tested on mRNA microarray assay for some complement components, including mRNA complement component C1r precursor, C4a, C2 and C5 is displayed in logarithm steps. See figures 33-36.

### Conclusion

When studying gene activity of the complement system in human mesenchymal cells using Affymetrix Gene Chip array analysis, there was not any evidence of activation of complement components apart from C1r, which most probably is not giving rise to any activation of the classical pathway of complement, most probably due to no antigen antibody reaction of type IgG, especially I found that C4 is not activated in IgG1, IgG3, IgM and other antibody components in the chondroblasts (∼Chondrocytes). The method for harvesting the chondroblasts from human cartilage explants was a technology described by Kurt Osther et al. and published in 2006 (Osther K, Storgaard P, Clausen Cl The Rationale to use Explant Cultures for ACI. (in Basic Science and Clinical Repair and Reconstruction of Articular Cartilage defects: Current Status and Prospects, Zanasi S, Brittberg M, Nehrer S, Marcacci M, editors),pp313-319, Timeo Bologna 2006)*.*

Furthermore, I found that C5, C6, C7, C8, and C9 in these human chondroblasts are not activated in these benign human mesenchymal (chondroblast) cells, which is consistent in 4 different Affymetrix Gene Analyses done. This corresponds overall to my postulated theory around the difference in this regard in benign cells such as for instance mesenchymal cells, when comparing these types of benign cells with glioma/glioblastoma.

### Example 15:

Rat GFP glioma cells and RG2 or GM1 rat glioma cells were incubated at a concentration of ∼5000 cells in rabbit anti human (anti human antibody crossreacting with rat either C1 IA, or rat CRP or mix of rat C1 IA/rat CRP) at 37 degrees C for 2 hours, then the cells were injected on the right parietal lobe of the brain (3-4 rats in each group). The cells were washed in PBS buffer pH 7.4 prior to the injection, which is a very delicate procedure (Lund University, Rausing Laboratory; the study was approved in advance by the appropriate ethics committee in Sweden).

The rats were then kept in cages under 24 hour observation by trained personnel. When rats developed classical symptoms of brain tumor with clinically deteriorating symptoms, including neurological symptoms, the rats were euthanized and the number of days of survival until disease was noted. The brains were removed for future studies. The aim of this study was primarily to identify whether the antibodies present after injection of the treated glioma cells into the rat brain will be retained. However, there appeared to be signs of some extension of survival in treated rats versus rats who received 5000 non-treated rat glioma cells.

Because the antibodies are raised in rabbits, i.e. a different species, it is possible that the xenogeneic rabbit immunoglobulins eventually can induce a limited immune reaction and thereby be capable of inducing an innate immune reaction due to the xenogeneic reaction in the rat. Thus, the small extension in survival in antibody treated rats versus non treated may induce complement reaction, because the preincubated cells were coated with anti C1 IA and anti CRP, which then would allow the complement system to be activated. Some of the coated cells injected into the rat may thus be capable of inducing the rats' complement system. Therefore, the pre-incubation of the glial cells injected into the rats may suggest that it is possible to de-block the cells' coat of C1 inhibitor (C1 IA) and de-block the CRP blocking on the C3b via the otherwise natural block observed by CRP through its binding to Factor H and following C3b block.

Thus, there is an indication of an extended survival in the animals that received glial cells that were pre-treated with anti C1 IA and CRP, as shown by a slightly significant extension of life when compared to rats receiving un-treated glial cells, with a p value = 0.3.

### References

Mancini, G, Vaerman, JP et al. (1964). Protides of the biological fluids (XI Colloquium). Peters H. (ed), Amsterdam, Elsevier Publishing Co., p370-373 and Mancini, G, Carbonara, AO et al. (1965). Immunochemical quantitation of antigens by single radial immunodiffusion. Immunochem. 2, 235-254.
Osther, K., Hojgaard, K., and Dybkj r E., Acta neurol. Scand, 1974 50, 681, Osther, K., the Lancet, Mar. 2, 1974, p. 359
Osther, K., Linnemann, R., Acta path. microbiol. scand. 1973, 81, p. 365).
Pastan et al., Cell 47, 641 (1986) and Goldenberg, Calif. A Cancer Journal for Clinicians 44, 43 (1994)
Zweig, M. H., and Campbell, G., Clin. Chem. 39 (1993) 561-577

**Sequence listing**

| **SEQ ID NO** | |
|---|---|
| **SEQ ID NO: 1** | C1 inactivator, amino acid, homo sapiens |
| **SEQ ID NO: 2** | C-Reactive Protein, amino acid, homo sapiens |
| **SEQ ID NO: 3** | Complement Component C4, amino acid, homo sapiens |

## Claims

1. A pharmaceutical composition comprising as the active ingredient, one or more antibodies that bind to human C-reactive protein (anti-CRP) for use in the treatment of cancer selected from the group consisting of glioblastomas, oligodendroglioma and astrocytomas, inhibiting growth of cancer selected from the group consisting of glioblastomas, oligodendroglioma and astrocytomas and/or inhibiting proliferation of cancer selected from the group consisting of glioblastomas, oligodendroglioma and astrocytomas in an individual.

2. The pharmaceutical composition for use according to claim 1, wherein the one or more anti-CRP is IgG anti-CRP or IgM anti-CRP and wherein the one or more IgG anti-CRP is selected from the group consisting of IgG1 anti-CRP, IgG2 anti-CRP, IgG3 anti-CRP, IgG4 anti-CRP and mixtures thereof.

3. The pharmaceutical composition for use according to any one of the preceeding claims, wherein the compostion further comprises as the active ingredient, one or more antibodies that bind to human complement 1-inactivator (anti-C1 IA) and/or one or more antibodies that bind to human complement component 4 (anti-C4) and a pharmaceutically acceptable carrier, diluent and/or excipient.

4. The pharmaceutical composition for use according to any one of the preceeding claims, wherein:
- the one or more anti-C1 IA is IgG anti-C1 IA and/or IgM anti-C1 IA and wherein the one or more IgG anti-C1 IA is selected from the group consisting of IgG1 anti-C1 IA, IgG2 anti-C1 IA, IgG3 anti-C1 IA, IgG4 anti-C1 IA and mixtures thereof, and
- the one or more anti-C4 is IgG anti-C4 and/or IgM anti-C4 and wherein the one or more IgG anti-C4 is selected from the group consisting of IgG1 anti-C4, IgG2 anti-C4, IgG3 anti-C4, IgG4 anti-C4 and mixtures thereof.

5. The pharmaceutical composition for use according to any one of the preceeding claims, wherein the one or more antibodies is selected from the group consisting of a polyclonal antibody, a monoclonal antibody, a synthetic antibody, a recombinant antibody, a chimeric antibody, a heterochimeric antibody, a humanized antibody and an oligoclonal antibody.

6. The pharmaceutical composition for use according to claim 1, wherein the human CRP comprise or consist of the amino acid sequence set forth in SEQ ID NO: 2

7. The pharmaceutical composition for use according to to claim 3, wherein the human C1 IA comprise or consist of the amino acid sequence set forth in SEQ ID NO: 1

8. The pharmaceutical composition for use according to claim 3, wherein the human C4 comprise or consist of the amino acid sequence set forth in SEQ ID NO: 3

## Patentansprüche

1. Pharmazeutische Zusammensetzung, umfassend, als den Wirkstoff, einen oder mehrere Antikörper, die an humanes C-reaktives Protein (anti-CRP) binden, zur Verwendung bei der Behandlung von Krebs ausgewählt aus der Gruppe bestehend aus Glioblastomen, Oligodendrogliom und Astrozytomen, beim Hemmen des Wachstums von Krebs ausgewählt aus der Gruppe bestehend aus Glioblastomen, Oligodendrogliom und Astrozytomen, und/oder beim Hemmen der Proliferation von Krebs ausgewählt aus der Gruppe bestehend aus Glioblastomen, Oligodendrogliom und Astrozytomen bei einem Individuum.

2. Pharmazeutische Zusammensetzung zur Verwendung nach Anspruch 1, wobei das eine oder die mehreren anti-CRP IgG anti-CRP oder IgM anti-CRP sind, und wobei das eine oder die mehreren IgG anti-CRP ausgewählt sind aus der Gruppe bestehend aus IgG1 anti-CRP, IgG2 anti-CRP, IgG3 anti-CRP, IgG4 anti-CRP und Gemischen davon.

3. Pharmazeutische Zusammensetzung zur Verwendung nach einem der vorhergehenden Ansprüche, wobei die Zusammensetzung ferner, als den Wirkstoff, einen oder mehrere Antikörper, die an humanen Komplement-1-Inaktivator (anti-C1 IA) binden, und/oder einen oder mehrere Antikörper, die an humane Komplement-Komponente 4 (anti-C4) binden, und einen pharmazeutisch annehmbaren Träger, Verdünner und/oder Hilfsstoff, umfasst.

4. Pharmazeutische Zusammensetzung zur Verwendung nach einem der vorhergehenden Ansprüche, wobei:
- die eine oder die mehreren anti-C1 IA IgG anti-C1 IA und/oder IgM anti-C1 IA sind und wobei die eine oder die mehreren IgG anti-C1 IA ausgewählt sind aus der Gruppe bestehend aus IgG1 anti-C1 IA, IgG2 anti-C1 IA, IgG3 anti-C1 IA, IgG4 anti-C1 IA und Gemischen davon, und
- die eine oder die mehreren anti-C4 IgG anti-C4 und/oder IgM anti-C4 sind und wobei die eine oder die mehreren IgG anti-C4 ausgewählt sind aus der Gruppe bestehend aus IgG1 anti-C4, IgG2 anti-C4, IgG3 anti-C4, IgG4 anti-C4 und Gemischen davon.

5. Pharmazeutische Zusammensetzung zur Verwendung nach einem der vorhergehenden Ansprüche, wobei der eine oder die mehreren Antikörper ausgewählt sind aus der Gruppe bestehend aus einem polyklonalen Antikörper, einem monoklonalen Antikörper, einem synthetischen Antikörper, einem rekombinanten Antikörper, einem chimären Antikörper, einem heterochimären Antikörper, einem humanisierten Antikörper und einem oligoklonalen Antikörper.

6. Pharmazeutische Zusammensetzung zur Verwendung nach Anspruch 1, wobei das humane CRP die Aminosäuresequenz, die in SEQ ID NO: 2 dargelegt ist, umfasst oder daraus besteht.

7. Pharmazeutische Zusammensetzung zur Verwendung nach Anspruch 3, wobei der humane C1 IA die Aminosäuresequenz, die in SEQ ID NO: 1 dargelegt ist, umfasst oder daraus besteht.

8. Pharmazeutische Zusammensetzung zur Verwendung nach Anspruch 3, wobei das humane C4 die Aminosäuresequenz, die in SEQ ID NO: 3 dargelegt ist, umfasst oder daraus besteht.

## Revendications

1. Composition pharmaceutique ayant pour ingrédient actif un ou plusieurs anticorps se liant à la protéine C réactive (anti-CRP) humaine pour l'utilisation dans le traitement du cancer sélectionné parmi le groupe constitué des glioblastomes, des oligodendrogliomes et des astrocytomes, pour inhiber la croissance d'un cancer sélectionné parmi le groupe constitué des glioblastomes, des oligodendrogliomes et des astrocytomes et/ou pour inhiber la prolifération d'un cancer sélectionné parmi le groupe constitué des glioblastomes, des oligodendrogliomes et des astrocytomes chez le patient.

2. Composition pharmaceutique pour l'utilisation conformément à la revendication 1, dans laquelle les un ou plusieurs anti-CRP sont l'anti-CRP IgG ou l'anti-CRP IgM, et dans laquelle les un ou plusieurs anti-CRP IgG sont sélectionnés parmi le groupe constitué des anti-CRP IgG1, des anti-CRP IgG2, des anti-CRP IgG3, des anti-CRP IgG4 et des mélanges de ceux-ci.

3. Composition pharmaceutique pour l'utilisation selon l'une quelconque des revendications précédentes, dans laquelle la composition comprend en outre pour ingrédient actif, un ou plusieurs anticorps se liant au complément humain 1-inactivateur (anti-C1 IA) et/ou un ou plusieurs anticorps se liant au composant 4 du complément humain (anti-C4) et un support, diluant et/ou excipient pharmaceutiquement acceptable.

4. Composition pharmaceutique pour l'utilisation selon l'une quelconque des revendications précédentes, dans laquelle :
- les un ou plusieurs anti-C1 IA sont l'anti-C1 IA IgG et/ou l'anti-C1 IA IgM, et dans laquelle les un ou plusieurs anti-C1 IA IgG sont sélectionnés parmi le groupe constitué des anti-C1 IA IgG1, des anti-Cl IA IgG2, des anti-C1 IA IgG3, des anti-C1 IA IgG4 et des mélanges de ceux-ci,
- les un ou plusieurs anti-C4 sont l'anti-C4 IgG et/ou l'anti-C4 IgM, et dans laquelle les un ou plusieurs anti-C4 IgG sont sélectionnés parmi le groupe constitué des anti-C4 IgG1, des anti-C4 IgG2, des anti-C4 IgG3, des anti-C4 IgG4 et des mélanges de ceux-ci.

5. Composition pharmaceutique pour l'utilisation selon l'une quelconque des revendications précédentes, dans laquelle les un ou plusieurs anticorps sont sélectionnés parmi le groupe constitué d'un anticorps polyclonal, d'un anticorps monoclonal, d'un anticorps synthétique, d'un anticorps recombinant, d'un anticorps chimérique, d'un anticorps hétérochimérique, d'un anticorps humanisé et d'un anticorps oligoclonal.

6. Composition pharmaceutique pour l'utilisation selon la revendication 1, dans laquelle la CRP humaine comprend ou est constituée de la séquence d'acides aminés présentée dans SEQ ID NO : 2.

7. Composition pharmaceutique pour l'utilisation selon la revendication 3, dans laquelle le C1 IA humain comprend ou est constitué de la séquence d'aminoacides présentée dans SEQ ID NO : 1.

8. Composition pharmaceutique pour l'utilisation selon la revendication 3, dans laquelle le C4 humain comprend ou est constitué de la séquence d'acides aminés présentée dans SEQ ID NO : 3.
